# EUROPEAN PATENT APPLICATION

(11) **EP 4 678 634 A1**
(43) Date of publication of application: **14.01.2026**
(21) Application number: 24766507.8
(22) Date of filing: 07.03.2024
(51) Int. Cl.: C07D 231/56, C07D 209/04, C07D 215/00, C07C 13/48, A61K 31/4184, A61K 31/403, A61K 31/438, A61P 35/00, A61P 13/08, A61P 17/00

(54) **ANDROGEN RECEPTOR MODULATORS AND USES THEREOF**

(30) Priority: 08.03.2023 CN 202310224306
(71) Applicant: Haihe Biopharma Co., Ltd., Shanghai 201203 (CN)
(72) Inventor: TU, Wangyang, Shanghai 201203 (CN); CHEN, Xiaoxu, Shanghai 201203 (CN); YU, Bing, Shanghai 201203 (CN); ZHANG, Yixiang, Shanghai 201203 (CN); LI, Leping, Shanghai 201203 (CN)
(74) Representative: Sagittarius IP
(86) International application number: PCT/CN2024/080533
(87) International publication number: WO 2024/183793

(57) **Abstract**

The present invention relates to androgen receptor modulators and uses thereof, specifically to compounds having formula (I)-(VI), pharmaceutical compositions comprising said compounds, and uses of said compounds for preventing or treating disease, especially for regulating androgen receptor activity and treating diseases including prostate cancer.

## Description

The present application claims priority for Chinese patent application with application number 202310224306.4, filed on March 8, 2023, and named "ANDROGEN RECEPTOR MODULATORS AND USES THEREOF", the entire disclosures of which are incorporated herein by reference in their entirety.

### TECHNICAL FIELD

The present invention relates to compounds used as androgen receptor modulators or pharmaceutically acceptable salts thereof. The present invention also relates to pharmaceutical compositions comprising said compounds and to the use of said compounds for preventing or treating diseases, said diseases being in particular those mediated by the androgen receptor, such as cancer.

### BACKGROUND

Prostate cancer is the most common tumor and one of the most lethal tumors in men. Currently, the main treatments for prostate cancer include surgery, radiotherapy, chemotherapy, castration, androgen-targeted therapy, PARP inhibitors and PD-1 immune checkpoint inhibitor antibodies. Because the growths of most prostate cancer cells are androgen-dependent, castration and androgen-targeted therapy are common clinical treatments. Androgen-targeted therapy includes the inhibition of androgen synthesis and the inhibition of androgen receptor, among which Bicalutamide, Abiraterone and Enzalutamide are commonly used clinical drugs, which can effectively prolong the survival of prostate cancer patients. However, most prostate cancer patients eventually develop resistance to castration and androgen-targeted drugs, and the known mechanisms for developing resistance include 1) reactivation of the androgen receptor, 2) activation of the glucocorticoid receptor pathway, and 3) neuroendocrine-type prostate cancers (Nat Rev Cancer. 2015 Dec; 15(12):701-11); of these, androgen receptor reactivation accounts for more than 60% of drug-resistant patients.

Androgen receptor (AR) is a transcription factor activated by androgen. In prostate cancer, after activation via its ligand-binding domain, AR will enter the cell nucleus from the cell membrane and forms a binary complex to activate the transcription and expression of downstream genes. The androgen receptor is divided into three main structural domains: the N-terminal is a loosely structured region (NTD), whose main role is to bind other transcription factors to form a complex; in the middle is the DNA-binding region (DBD), which helps the androgen receptor to bind to the DNA of the downstream genes; and the C-terminal is the ligand-binding region (LBD), through which the androgen binds. Existing AR-targeted therapies mainly act on androgen synthesis and on the ligand-binding domain of the androgen receptor to antagonize the activation of the receptor by androgens. Mutations leading to resistance to AR-targeted therapies mainly occur in the LBD-binding region, including a) point mutations at amino acid sites, such as F877L, T878A, etc., which diminish the antagonistic effect of AR-targeted therapy or even produce an agonistic effect; and b) androgen receptor splice variations, such as AR-Vs, etc., which make the androgen receptor no longer express the complete ligand-binding domain, but only the N-terminal and DNA-binding domains. The absence of the LBD-binding region or mutations in the LBD-binding region renders existing androgen receptor antagonists ineffective in inhibiting the functions of androgen receptor, and thus no longer effective in treating these drug-resistant prostate patients.

The above resistance mechanisms arising from AR LBD domain mutations and deletions have necessitated the efforts to develop the next generation of AR-targeted drugs with different mechanisms of action to provide cancer patients with potential new therapies. AR-LBD binding PROTAC molecules can degrade the androgen receptor, thereby completely blocking the androgen receptor pathway. Arvinas' ARV-110 has shown preliminary efficacy in the clinic in prostate cancer patients harboring AR LBD mutations such as T878A, but ARV-110 does not bind to mutations such as AR-Vs which lacks LBD-binding domain. Therefore, it could not benefit prostate cancer patients with AR-Vs mutations. N-terminal targeting AR inhibitor EPI-7386 developed by ESSA is also in early clinical trials and has been shown to be well tolerated in clinical trials, but its efficacy remains to be tested.

Therefore, there are still strong unmet medical needs to develop AR-targeted agents with novel mechanisms of action as the potential effective treatment of prostate cancer. Moreover, despite the multiple therapeutic options available to cancer patients, there is still a need for effective and safe therapeutic agents and their preferred use in combination therapies.

### SUMMARY OF THE INVENTION

The compounds of the present disclosure are androgen receptor modulators that can be used to treat a variety of diseases and conditions such as those disclosed herein, and in particular for modulating androgen receptors containing mutations or deletions in the structural domains of the LBD; said diseases and conditions include, but are not limited to, prostate cancer, other prostate disorders, breast cancer, acne, hirsutism, hidradenitis suppurativa, androgenetic alopecia, cryptorchidism, androgen insensitivity, Kennedy's disease, etc.

Specifically, the present disclosure provides androgen receptor modulators as shown in formula (I): 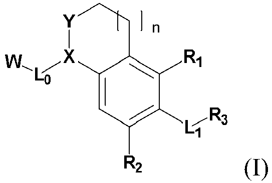 wherein, each of the variables R₁, R₂, R₃, n, X, Y, L₀, L₁ and ring W are as defined herein, including stereoisomers, geometrical isomers, tautomers, solvates hydrates, or pharmaceutically acceptable salts thereof, which may be used for preventing or treating androgen receptor-mediated diseases or disorders, particularly cancer.

The present disclosure also provides a method for preparing the compound of the present disclosure, an intermediate for preparing the compound of the present disclosure, and a method for preparing the intermediate.

The present disclosure also provides a composition comprising at least one of the compound of the present disclosure or the pharmaceutically acceptable salt thereof.

The present disclosure also provides a pharmaceutical composition comprising a therapeutically effective amount of the compound or the pharmaceutically acceptable salt thereof of the present disclosure, as well as one or more pharmaceutically acceptable carriers, diluents or excipients.

In one embodiment, the present disclosure provides a pharmaceutical composition comprising a therapeutically effective amount of the compound of the present disclosure or the pharmaceutically acceptable salt thereof.

In another embodiment, the present disclosure provides a combination, especially a pharmaceutical combination, comprising a therapeutically effective amount of the compound of the present disclosure or the pharmaceutically acceptable salt thereof, as well as one or more other therapeutic agents.

The compound of the present disclosure may be used alone, in combination with other compounds of the present disclosure, or in combination with one or more, preferably one or two other substances simultaneously or sequentially.

The compounds of the present disclosure are androgen receptor modulators that can modulate the androgen receptor in vivo and can thus be used to treat or prevent androgen receptor-mediated diseases or disorders, particularly cancer. Thus, the present disclosure may provide inhibition of the transcriptional activation function of the androgen receptor. Examples include uses of the compounds of the present disclosure for enhancing androgen-mediated responses in a subject, and methods of using the compounds of the present disclosure for modulating immune responses in a subject.

The compound of the present disclosure can be used for therapy.

The compound of the present disclosure can be used to prepare medicaments or drugs, which are used to treat or prevent androgen receptor-mediated diseases or obstacles, especially cancer.

The present disclosure also involves a method for inhibiting androgen receptor activity in individuals, wherein, the method includes administering a therapeutically effective amount of androgen receptor inhibitors such as the compound of the present disclosure or the pharmaceutically acceptable salt thereof to an individual in need.

In one embodiment of the present disclosure, the present disclosure provides a method for treating or preventing androgen receptor-mediated diseases or disorders, including administering an effective amount of a first therapeutic agent and an optional second therapeutic agent to patients in need, wherein the first therapeutic agent is the compound of the present disclosure or the pharmaceutically acceptable salt thereof, and the second therapeutic agent is one or more other therapeutic agents.

In another embodiment of the present disclosure, the present disclosure involves a method for treating or preventing androgen receptor-mediated diseases or disorders, such as cancer or infectious diseases or conditions, which includes administering a therapeutically effective amount of the compound of the present disclosure or the pharmaceutically acceptable salt thereof to an individual.

A preferred method of the present disclosure is the treatment of diseases or disorders mediated by androgen receptor containing mutations or deletions in the structural domain of LBD, said diseases or disorders include, but are not limited to, prostate cancer, other prostate disorders (e.g., prostatic hyperplasia, prostatitis, etc.), breast cancer, acne, hirsutism, hidradenitis suppurativa, androgenetic alopecia, cryptorchidism, androgen insensitivity, and Kennedy's disease.

In some embodiments of the present invention, said prostate cancer includes, but is not limited to, metastatic castration-resistant prostate cancer, primary/focal prostate cancer, locally progressive prostate cancer, recurrent prostate cancer, non-metastatic castration-resistant prostate cancer, metastatic castration-resistant prostate cancer, metastatic prostate cancer, hormone-sensitive prostate cancer.

In some embodiments of the present invention, said breast cancer includes, but is not limited to, Luminal A-type (ER+/PR+, HER-2-), Luminal B-type (ER+/PR+, HER-2+), HER-2+-type (ER-/PR-/HER-2+) and Basal-like (ER-/PR-/HER-2-) breast cancer.

Additionally, the present disclosure provides combination products or kits, the combination products or kits comprising the compounds of the present disclosure, as defined above, or pharmaceutically usable salts thereof, or pharmaceutical compositions thereof, and one or more other active agents, or pharmaceutical compositions comprising said active agents, which are intended for use concurrently, separately, or sequentially, in therapies for treating or preventing androgen receptor-mediated diseases or disorders.

### IMPLEMENTATIONS

On one hand, the present disclosure provides a compound of formula (I), or a stereoisomer, a geometrical isomer, a tautomer, a solvate, a hydrate, or a pharmaceutically acceptable salt thereof, 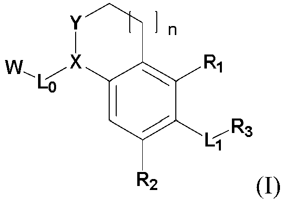
wherein, n is 0 or 1; preferably, n is 1;
X is N or CH;
Y is NR₄ or CHR₄;
L₀ is a bond, O, S, -NR₅-, -C(O)-NR₅-, -NR₅-C(O)-, -NR₅-(CH₂)ₘ- or -(CH₂)ₘ-NR₅-; m is 1 or 2; preferably, L₀ is a bond, O, S, -NR₅-, -NH-C(O)-, -C(O)-NH-, -NR₅-(CH₂)₂- or -NR₅-CH₂-; more preferably, L₀ is a bond, O, S, -NR₅-, -NH-C(O)-, -C(O)-NH-;
L₁ is a bond, O, S or -NR₅-, preferably, L₁ is a bond, O or S;
R₁, R₂ are each independently selected from H, C₁-C₆ alkyl, halogen, halo C₁-C₆ alkyl, CN, C₁-C₆ alkoxy, -C₁-C₆ alkyl-NR₆R₇ and -C(O)-NR₆R₇; and preferably, R₁, R₂ are each independently selected from H, halogen, CF₃, CN, C₁-C₆ alkyl, C₁-C₆ alkoxy, -CH₂-NH₂ and -C(O)NH-CH₃;
wherein R₄, R₅, R₆, R₇ are each independently selected from H, C₁-C₆ alkyl and acyl; preferably, R₄, R₅, R₆, R₇ are each independently H or C₁-C₆ alkyl;
R₃ is hydroxy, C₁-C₆ alkyl, C₃-C₈ cycloalkyl, C₆-C₁₂ aryl, 5- to 12-membered heteroaryl, or 4- to 12-membered heterocycloalkyl, and said alkyl, cycloalkyl, aryl, heteroaryl and heterocycloalkyl are each optionally substituted with one or more substituents independently selected from halogen and hydroxyl; preferably R₃ is C₁-C₆ alkyl, C₃- C₇ cycloalkyl, C₆-C₁₀ aryl, 5- to 10-membered heteroaryl or 5- to 7-membered heterocycloalkyl, each of which is optionally substituted with one or more substituents selected from halogen and hydroxyl; more preferably, R₃ is C₁-C₆ alkyl, C₃-C₇ cycloalkyl, C₆-C₁₀ aryl, 5- to 10-membered heteroaryl or 5-to 7-membered heterocycloalkyl, each of which is optionally substituted with one or more substituents selected from F, Cl and OH;
W is C₅-C₈ cycloalkyl, C₆-C₁₂ aryl, 5- to 12-membered heteroaryl or 4- to 12-membered -heterocycloalkyl, each of which is optionally substituted with one or more substituents independently selected from halogen, CN, oxo, -NR₇R₈, -OR₈, -C(O)-NHR₈, R₉, -OC(O)-NHR₉, -NHC(O)-R₁₀, -SO₂R₉, -SO₂NHR₈, -NR₇SO₂R₉, -NR₇SO₂NR₈R₁₀, substituted or unsubstituted C₃-C₈ cycloalkyl, 4- to 8-membered heterocycloalkyl, C₆-C₁₂ aryl, and 5- to 10-membered heteroaryl; wherein said C₃-C₈ cycloalkyl, 4- to 8-membered heterocycloalkyl, C₆-C₁₂ aryl, and 5- to 10-membered heteroaryl are each optionally further substituted with one or more substituents selected from halogen, oxo, NH₂, hydroxyl, C₁-C₄ alkyl and C₁-C₄ alkoxy;
wherein R₈ is H or is C₁-C₆ alkyl optionally substituted with one or more substituents selected from halogen and hydroxyl; preferably, R₈ is H or C₁-C₆ alkyl optionally substituted with F or OH;
R₉ is C₁-C₆ alkyl optionally substituted with one or more substituents selected from halogen, hydroxyl, and NH₂;
R₁₀ is C₁-C₆ alkyl or C₁-C₆ alkoxy;
preferably, said heterocycloalkyl comprises 1-2 heteroatoms selected from N, NR_{c}, O and S(O)ₚ, and R_{c} is each independently selected from hydrogen and C₁-C₄ alkyl, and p is 1 or 2.

In one preferred embodiment, the present invention provides the compound of formula (I), or the stereoisomer, the geometrical isomer, the tautomer, the solvate, the hydrate, or the pharmaceutically acceptable salt thereof, wherein W is C₅-C₈ cycloalkyl, C₆-C₁₂ aryl, 5- to 12-membered heteroaryl or 4- to 12-membered heterocycloalkyl, each of which is optionally substituted with one or more substituents independently selected from halogen, CN, oxo, NR₇R₈, OR₈, R₉, -C(O)-NHR₈, -OC(O)-NHR₉, -SO₂R₉, -SO₂NHR₈, -NR₇SO₂R₉, -NR₇SO₂NHR₁₀, -NHC(O)-R₁₀, and C₃-C₈ cycloalkyl, 4- to 8-membered heterocycloalkyl, C₆-C₁₀ aryl, 5- to 10-membered heteroaryl, wherein said C₃-C₈ cycloalkyl, 4- to 8-membered heterocycloalkyl, C₆-C₁₂ aryl and 5- to 10-membered heteroaryl are each optionally further substituted with one or more substituents independently selected from halogen, oxo, NH₂, hydroxyl, C₁-C₄ alkyl, and C₁-C₄ alkoxy;
more preferably, each of which is optionally further substituted with one or more substituents independently selected from -OH, F, Cl, CN, oxo, -NH₂, C₁-C₆ alkyl, C₁-C₆ alkoxy, haloC₁-C₆ alkyl, haloC₁-C₆ alkoxy, -(C₁-C₆ alkyl)OH, -(C₁-C₆ alkoxy)OH, -NH(C₁-C₃ alkyl), -NH(C₁-C₄ alkyl)OH, -NHC(O)(C₁-C₃ alkyl), -NHC(O)(C₁-C₄ alkoxy), -OC(O)NH(C₁-C₃ alkyl), -C(O)NH(C₁-C₃ alkyl), -O(C₁-C₄ alkyl)OH, -C(O)NH₂, -SO₂NH₂, -SO₂(C₁-C₄ alkyl), -SO₂(C₁-C₃ alkyl)NH₂, -SO₂NH(C₁-C₄ alkyl), -NHSO₂(C₁-C₄ alkyl), -N(CH₃)SO₂(C₁-C₃ alkyl), -NHSO₂(C₁-C₃ alkyl)NH₂, -NHSO₂CF₃, and C₃-C₇ cycloalkyl, 5- to 8-membered heterocycloalkyl, C₆-C₁₀ aryl and 5- to 10-membered heteroaryl, wherein said C₃-C₇ cycloalkyl, 5- to 8-membered heterocycloalkyl, C₆-C₁₀ aryl and 5- to 10-membered heteroaryl are each optionally substituted with one or more substituents independently selected from halogen, OH, NH₂, C₁-C₄ alkyl, and C₁-C₄ alkoxy.

In one preferred embodiment, the present invention provides the compound of formula (I), or the stereoisomer, the geometrical isomer, the tautomer, the solvate, the hydrate, or the pharmaceutically acceptable salt thereof, wherein W is C₅-C₈ cycloalkyl, C₆-C₁₂ aryl, 5- to 12-membered heteroaryl or 4- to 12-membered heterocycloalkyl, each of which is optionally substituted with one or more substituents independently selected from the group consisting of: -OH, F, Cl, CN, oxo, -NH₂, -NHCH₃, -NH(CH₂)₂OH, C₁-C₆ alkyl, C₁-C₆ alkoxy, haloC₁-C₆ alkyl, haloC₁-C₆ alkoxy, -NHC(O)CH₃, -NHC(O)OCH₃, -OC(O)NH-CH₃, -C(O)NH-CH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentyl, cyclohexyl, pyridinyl, phenyl, morpholinyl, 1,3-oxazinylalkyl, tetrahydrofuranyl, hexahydropyrimidinyl, piperazinyl, pyrrole, imidazole, pyrazole, 4-methylpiperazin-1-yl, piperidinyl and 4-hydroxy-piperidin-1-yl.

In one preferred embodiment, the present invention provides the compound of formula (I), or the stereoisomer, the geometrical isomer, the tautomer, the solvate, the hydrate, or the pharmaceutically acceptable salt thereof, wherein W is cyclopentane, cyclohexane, phenyl, pyrrole, pyrazole, imidazole, thiazole, oxazole, isoxazole, oxadiazole, pyridine, pyrimidine, pyrazine, pyridazine, tetrahydrofuran, pyrrolidine, pyrazolidine, imidazolidine, tetrahydropyran, piperidine, piperazine, hexahydropyrimidine, morpholine, octahydropyrrolo[3,2-b]pyrrole, indole, benzopyran, benzimidazole, benzoxazole, benzotriazole, 4-azaindole, 5-azaindole, 6-azaindole, 7-azaindole, quinoline, isoquiline, quinazoline, cinnoline, quinoxaline, naphthyl, indenyl, 1*H*-pyrazolo[4,3-b]pyridine, 1*H*-pyrazolo[4,3-*c*]pyridine, 1*H*-pyrazol[3,4-*c*]pyridine, 1*H*-pyrazolo[3,4-*b*]pyridine, 2*H*-pyrazolo[3,4-b]pyridine, 2,3-dihydro-oxazolo[4,5-*b*]pyridine, isoindoline, indoline, dihydrobenzofuran, 2,3-dihydro-1*H*-benzo[d]imidazole, 2,3-dihydro-benzo[*d*]oxazole, 2,3-dihydrobenzo[*d*]thiazole, 2,3-dihydro-1*H*-indazole, 1,2 dihydroquinoline, tetrahydroquinoline, tetrahydroisoquinoline, tetrahydronaphthalene, dihydroindene, chromane, isochromane, dihydrobenzofuran, dihydroisobenzofuran, 1,2-dihydro-1,8-naphthyridine, oxazolo[4,5-*b*]pyridine, pyridin-2(1*H*)-one, 2-indolinone, 2-benzoxazolone, quinolin-2(1*H*)-one, 1,4-dihydro-3(2*H*)-isoquinolinone, 1,3-dihydrobenzimidazol-2-one, 2,3-dihydrochromen-4-one, 5,8-dihydro-6*H*[1,6]naphthyridin-7-one, 7,8-dihydro-6*H*-[1,6]naphthyridin-5-one, 1,8-naphthyridin-2(1*H*)-one, 3a,4,5,6,7,7a-hexahydro-1*H*-pyrazolo[4,3-c]pyridine, 3a,4,5,6,7,7a-hexahydro-1*H*-pyrazolo[3,4-c]pyridine, 2*H*-chromene, oxazolo[5,4-c]pyridin-2(1*H*)-one or oxazolo[4,5-*b*]pyridin-2(3*H*)-one, each of which is optionally substituted with one or more substituents independently selected from the group consisting of: halogen, CN, oxo, -NR₇R₈, hydroxy, C₁-C₆ alkyl, C₁-C₆ alkoxy, haloC₁-C₆ alkyl, haloC₁-C₆ alkoxy, hydroxy-C₁-C₆ alkyl, hydroxy-C₁-C₆ alkoxy, hydroxy-C₁-C₆ alkyl-NR₈-, -C(O)-NHR₈, -OC(O)-NHR₉, -NHC(O)-R₁₀, -SO₂R₉, -SO₂NHR₈, -NR₇SO₂R₉, - NR₇SO₂NHR₈, C₃-C₈ cycloalkyl, 4- to 8-membered heterocycloalkyl, C₆-C₁₂ aryl and 5- to 10-membered heteroaryl, wherein said C₃-C₈ cycloalkyl, 4- to 8-membered heterocycloalkyl, C₆-C₁₂ aryl and 5- to 10-membered heteroaryl are each optionally further substituted with one or more substituents selected from halogen, oxo, NH₂, hydroxy, C₁-C₄ alkyl and C₁-C₄ alkoxy;
wherein, R₈ is H, or C₁-C₆ alkyl optionally substituted with one or more substituents of halogen or hydroxyl;
R₉ is C₁-C₆ alkyl optionally substituted with one or more substituents of halogen or NH₂;
R₁₀ is C₁-C₆ alkyl or C₁-C₆ alkoxy.

In one preferred embodiment, the present invention provides the compound of formula (I), wherein W is cyclopentane, cyclohexane, phenyl, pyrrole, pyrazole, imidazole, thiazole, oxazole, isoxazole, oxadiazole, pyridine, pyrimidine, pyrazine, pyridazine, tetrahydrofuran, pyrrolidine, pyrazolidine, imidazolidine, tetrahydropyran, piperidine, piperazine, hexahydropyrimidine, morpholine, octahydropyrrolo[3,2-*b*]pyrrole, indole, benzopyran, benzimidazole, benzoxazole, benzotriazole, 4-azaindole, 5-azaindole, 6-azaindole, 7-azaindole, quinoline, isoquinoline, quinazoline, cinnoline, quinoxaline, naphthyl, indenyl, imidazolopyridine, 1*H*-pyrazolo[4,3-b]pyridine, 1*H*-pyrazolo[4,3-c]pyridine, 1*H*-pyrazolo[3,4-c]pyridine, 1*H*-pyrazolo[3,4-*b*]pyridine, 2*H*-pyrazolo[3,4-b]pyridine, 2,3-dihydrooxazolo[4,5-b]pyridine, isoindoline, indoline, dihydrobenzofuran, 2,3-dihydro-1*H*-benzo[d]imidazole, 2,3-dihydrobenzo[*d*]oxazole, 2,3-dihydrobenzo[*d*]thiazole, 2,3-dihydro-1*H*-indazole, 1,2-dihydroquinoline, tetrahydroquinoline, tetrahydroisoquinoline, tetrahydronaphthalene, dihydroindene, chroman, isochroman, dihydrobenzofuran, dihydroisobenzofuran, 1,2-dihydro-1,8-naphthyridine, oxazolo[4,5-*b*]pyridine, pyridin-2(1*H*)-one, 2-indolone, 2-benzoxazolone, quinolin-2(1*H*)-one, 1,4-dihydro-3(2*H*)-isoquinolone, 1,3-dihydrobenzimidazol-2-one, 2,3-dihydrochromen-4-one, 5,8-dihydro-6*H*-[1 ,6]naphthyridin-7-one, 7,8-dihydro-6*H*-[1,6]naphthyridin-5-one, 1,8-naphthyridin-2(1*H*)-one, 3a,4,5,6,7,7a-hexahydro-1*H*-pyrazolo[4,3-c]pyridine, 3a,4,5,6,7,7a-hexahydro-1*H*-pyrazolo[3,4-c]pyridine, 2*H*-chromene, oxazolo[5,4-*c*]pyridin-2(1*H*)-one or oxazolo[4,5-*b*]pyridin-2(3H)-one, each of which is optionally substituted with one or more substituents independently selected from the group consisting of: -OH, F, Cl, CN, oxo, -NH₂, -NHCH₃, -NH(CH₂)₂OH, C₁-C₆ alkyl, C₁-C₆ alkoxy, haloC₁-C₆ alkyl, haloC₁-C₆ alkoxy, -NHC(O)CH₃, -NHC(O)OCH₃, -OC(O)NH-CH₃, -C(O)NH-CH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentane, cyclohexane, pyridinyl, phenyl, morpholinyl, 1,3-oxazinylalkyl, tetrahydrofuranyl, hexahydropyrimidinyl, piperazinyl, pyrrole, imidazole, pyrazole, 4-methyl-piperazin-1-yl, piperidinyl and 4-hydroxy-piperidin-1-yl.

In one preferred embodiment, the present invention provides the compound of formula (I), or the stereoisomer, the geometrical isomer, the tautomer, the solvate, the hydrate, or the pharmaceutically acceptable salt thereof, wherein W is selected from: 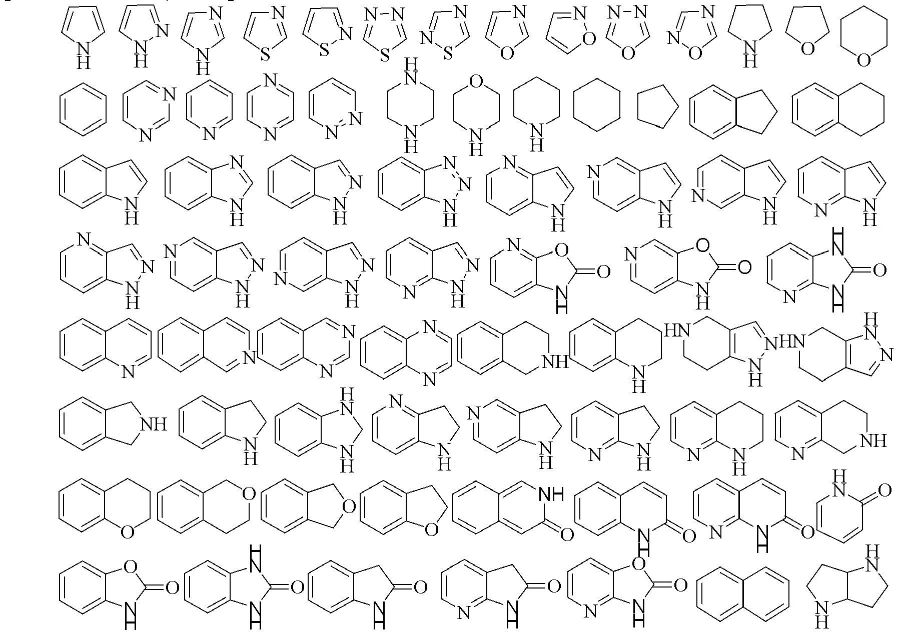 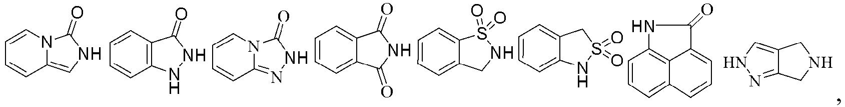 wherein, each of the above groups is optionally substituted with one or more substituents independently selected from -OH, F, Cl, CN, oxo, -NH₂, -NHCH₃, -NH(CH₂)₂OH, C₁-C₆ alkyl, C₁-C₆ alkoxy, haloC₁-C₆ alkyl, haloC₁-C₆ alkoxy, -NHC(O)CH₃, -NHC(O)OCH₃, -OC(O)NH-CH₃, -C(O)NH-CH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentane, cyclohexane, pyridyl, phenyl, morpholinyl, 1,3-oxazinylalkyl, tetrahydrofuranyl, hexahydropyrimidinyl, pyrrole, imidazole, pyrazole, piperazinyl, 4-methyl-piperazin-1-yl, piperidinyl, and 4-hydroxy-piperidin-1-yl.

It should be understood that, the W group of the present invention is connected to the rest of the compound of formula (I) at possible positions (e.g. at positions 1, 2, 3, 4, 5, 6, or 7), and in particular may be connected via the connecting position of the W group shown in the example compounds.

In one more preferred embodiment, the present invention provides the compound of formula (I), or the stereoisomer, the geometrical isomer, the tautomer, the solvate, the hydrate, or the pharmaceutically acceptable salt thereof, wherein:
n is 0 or 1;
X is N or CH;
Y is NR₄ or CHR₄, wherein R₄ is selected from H and C₁-C₆ alkyl;
L₀ is a bond, O, S, -NR₅-, -C(O)-NH-, -NH-C(O)-, or - NR₅-(CH₂)ₘ-; m is 1 or 2;
L₁ is a bond, O, S or -NR₅-;
wherein R₅ is H, acetyl or C₁-C₆ alkyl;
R₁, R₂ are each independently selected from H, C₁-C₆ alkyl, halogen, CN, -C₁-C₆ alkyl-NR₆R₇ and -C(O)-NR₆R₇, wherein R₆, R₇ are each independently selected from H and C₁-C₆ alkyl;
R₃ is C₁-C₆ alkyl or haloC₁-C₆ alkyl; or R₃ is C₃-C₈ cycloalkyl, C₆-C₁₂ aryl, 5- to 12-membered heteroaryl or 5- or 7-membered heterocyclicyl, each of which is optionally substituted with one or more substituents selected from halogen and hydroxyl, e.g. cyclopropyl, cyclopentyl, cyclohexyl, phenyl, pyridyl, hexahydropyrimidyl, piperazinyl, 4-methyl-piperazin-1-yl, piperidinyl, 1,3-oxazinylalkyl, morpholinyl, and 4-hydroxy-piperidin-1-yl, each of which is optionally substituted with one or more substituents selected from halogen and hydroxyl.

In one preferred embodiment, the ring W can optionally be independently substituted one or more times, e.g., once or twice, by substituents selected from halogen, CN, OH, oxo, NH₂, C₁-C₄ alkyl, C₁-C₄ alkoxy, C₃-C₆ cycloalkyl, C₁-C₄ haloalkyl, C₁-C₄ haloalkoxy, -NH(C₁-C₃ alkyl), -NH(C₁-C₄ alkyl)OH, -NHC(O)(C₁-C₃ alkyl), -NHC(O)(C₁-C₃ alkoxy), -OC(O)NH(C₁-C₃ alkyl), -C(O)NH(C₁-C₃ alkyl), -O(C₁-C₄ alkyl)OH, -C(O)NH₂, -SO₂NH₂, -SO₂(C₁-C₄ alkyl), -SO₂(C₁-C₃ alkyl)NH₂-, -NHSO₂(C₁-C₄ alkyl), -N(CH₃)SO₂(C₁-C₃ alkyl), -NHSO₂(C₁-C₃ alkyl)NH₂, -NHSO₂CF₃, 5- to 7-membered heterocycloalkyl, C₆-C₁₀ aryl, and 5- to 7-membered heteroaryl;
further preferably, the ring W can optionally be independently substituted one or more times, e.g., once or twice, by substituents selected from F, Cl, CN, OH, (=O), NH₂, C₁-C₃ alkyl, C₁-C₃ alkoxy, C₃-C₆ cycloalkyl, C₁-C₃ haloalkyl, C₁-C₃ haloalkoxy, -NHCH₃, -NH(CH₂)₂OH, -NHC(O)CH₃, -NHC(O)OCH₃, -OC(O)NHCH₃, -C(O)NHCH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, 5- to 7-membered heterocycloalkyl, C₆-C₈ aryl, and 5- to 8-membered heteroaryl;
said heterocycloalkyl, aryl and heteroaryl are preferably: cyclopentane, cyclohexane, phenyl, pyrrole, pyrazole, imidazole, thiazole, oxazole, isoxazole, oxadiazole, pyridine, pyrimidine, pyrazine, pyridazine, tetrahydrofuran, pyrrolidine, pyrazolidine, imidazolidine, tetrahydropyrimidine, piperidine, piperazine, hexahydropyrimidine, morpholine, octahydropyrrolo[3,2-b]pyrrole, indole, benzopyran, benzimidazole, benzoxazole, benzotriazole, 4-azabindole, 5-azabindole, 6-azabindole, 7-azabindole, quinoline, isoquiline, quinazoline, cinnoline, quinoxaline, naphthyl, indenyl, 1*H*-pyrazolo[4,3-b]pyridine, 1*H*-pyrazolo[4,3-c]pyridine, 1*H*-pyrazolo[3,4-c]pyridine, 1*H*-pyrazolo[3,4-*b*]pyridine, 2*H*-pyrazolo[3,4-b]pyridine, 2,3-dihydrooxazolo[4,5-*b*]pyridine, isoindoline, indoline, dihydrobenzofuran, 2,3-dihydro-1*H*-benzo[d]imidazole, 2,3-dihydrobenzo[*d*]oxazole, 2,3-dihydrobenzo[d]thiazole, 2,3-dihydro-1*H*-indazole, 1,2 dihydroquinoline, tetrahydroquinoline, tetrahydroisoquinoline, tetrahydronaphthalene, dihydroindene, chroman, isochroman, dihydrobenzofuran, dihydroisobenzofuran, 1,2-dihydro-1,8-naphthyridine, oxazolo[4,5-*b*]pyridine, 3a,4,5,6,7,7a-hexahydro-1*H*-pyrazolo[4,3-c]pyridine, 3a,4,5,6,7,7a-hexahydro-1*H*-pyrazolo[3,4-c]pyridine or 2*H*-chromene;
more preferably are cyclopentane, cyclohexane, benzene ring, pyridine ring, piperazine, pyrazine, pyrimidine, oxazolidine, oxazinane, isoxazinane, morpholine, tetrahydrofuran, pyrrolidine, tetrahydropyran, piperidine, hexahydropyrimidine, pyrrole, imidazole, pyrazole, 4-methyl-piperazin-1-yl or 4-hydroxy-piperidin-1-yl.

In one preferred embodiment, the ring W is optionally substituted or unsubstituted cyclopentane, cyclohexane, tetrahydrofuran ring, tetrahydropyran ring, pyrrolidine, pyrazolidine, imidazolidine, oxazolidine, piperidine ring, morpholine, piperazine ring, piperidine ring, hexahydropyrimidine, oxazinane, octahydropyrrolo[3,2-b]pyrrole, pyrrolidine ring, pyrazole ring, imidazole ring, pyrazine ring, pyridazine ring, pyrimidine ring, pyridine ring, benzene ring, indole ring, benzimidazole ring, indazole ring, benzotriazole ring, benzoxazole, 3a,4,5,6,7,7a-hexahydro-1*H*-pyrazolo[4,3-*c*]pyridine, 3a,4,5,6,7,7a-hexahydro-1*H*-pyrazolo[3,4-c]pyridine, isoindoline, indoline, 2,3-dihydro-1*H*-benzo[d]imidazole, 2,3-dihydro-benzo[d]oxazole, 2,3-dihydro-1*H*-indazole, oxazolo[4,5-*b*]pyridine, 1*H*-pyrazolo[4,3-b]pyridine, 1*H*-pyrazolo[4,3-c]pyridine, 1*H*-pyrazolo[3,4-*c*]pyridine, 1H-pyrazolo[3,4-*b*]pyridine, 2*H*-pyrazolo[3,4-b]pyridine, 2,3-dihydro-oxazolo[4,5-*b*]pyridine, tetrahydroquinoline, tetrahydroisoquinoline, dihydrobenzopyran, 1,2-dihydro-1,8-naphthyridine, azaindole, tetrahydronaphthalene or dihydroindene;
further preferably, the ring W is cyclopentane, cyclohexane, tetrahydrofuran ring, tetrahydropyran ring, pyrrolidine, pyrazolidine, imidazolidine, oxazolidine, piperidine ring, morpholine, piperazine ring, piperidine ring, hexahydropyrimidine, oxazinane, octahydropyrrolo[3,2-*b*]pyrrole, pyrrole ring, pyrazole ring, imidazole ring, pyrazine ring, pyridazine ring, pyrimidine ring, pyridine ring, benzene ring, indole ring, benzimidazole ring, indazole ring, benzotriazole ring, benzoxazole, 3a,4,5,6,7,7a-hexahydro-1*H*-pyrazolo[4,3-c]pyridine, 3a,4,5,6,7,7a-hexahydro-1*H*-pyrazolo[3,4-c]pyridine, isoindoline, indoline, 2,3-dihydro-1*H*-benzo[d]imidazole, 2,3-dihydrobenzo[*d*]oxazole, 2,3-dihydro-1*H*-indazole, oxazolo[4,5-b]pyridine, 1*H*-pyrazolo[4,3-b]pyridine, 1*H*-pyrazolo[4,3-c]pyridine, 1*H*-pyrazolo[3,4-c]pyridine, 1*H*-pyrazolo[3,4-*b*]pyridine, 2*H*-pyrazolo[3,4-b]pyridine, 2,3-dihydrooxazolo[4,5-*b*]pyridine, tetrahydroquinoline, tetrahydroisoquinoline, dihydrobenzopyran, 1,2-dihydro-1,8-naphthyridine, azaindole, tetrahydronaphthalene, or dihydroindene, each of which is substituted once or twice by substituents selected from F, Cl, CN, OH, (=O), NH₂, C₁-C₃ alkyl, C₁-C₃ alkoxy, C₃-C₆ cycloalkyl, C₁-C₃ haloalkyl, -NHCH₃, -NH(CH₂)₂OH, NHC(O)CH₃, -NHC(O)OCH₃, -OC(O)NHCH₃, -C(O)NHCH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentane, cyclohexane, benzene ring, pyridine ring, piperidine, tetrahydropyran, piperazine, pyrazine, pyrimidine, hexahydropyrimidine, oxazinane, isoxazinane, tetrahydrofuran, pyrrolidine, oxazolidine, morpholine, pyrrole, imidazole, pyrazole, 4-methyl-piperazin-1-yl and 4-hydroxy-piperidin-1-yl.

In one embodiment, X is N.

In one embodiment, X is CH.

In one embodiment, Y is NR₄ or CHR₄, and R₄ is selected from H, C₁-C₃ alkyl and acetyl.

In one preferred embodiment, Y is NR₄, preferably, R₄ is selected from H, C₁-C₃ alkyl, and acetyl.

In another preferred embodiment, Y is CHR₄, preferably, R₄ is selected from H and C₁-C₃ alkyl; more preferably, R₄ is H.

In one preferred embodiment, R₁, R₂ are each independently selected from H, C₁-C₆ alkyl, halogen, haloC₁-C₄ alkyl, CN, C₁-C₄ alkoxy, -CH₂-NH₂, and -C(O)NH-CH₃; preferably, R₁, R₂ are each independently selected from H, halogen, CN, CF₃, C₁-C₄ alkyl and C₁-C₄ alkoxy; more preferably, R₁, R₂ are each independently selected from H, C₁-C₆ alkyl, halogen and CN;

In a preferred embodiment, R₁, R₂ are each independently selected from H, halogen, CN, -CH₂-NH₂, and -C(O)NH-CH₃; more preferably, R₁, R₂ are each independently selected from H, halogen, and CN.

In one preferred embodiment, the present invention provides the compound of formula (I), or the stereoisomer, the geometrical isomer, the tautomer, the solvate, the hydrate, or the pharmaceutically acceptable salt thereof.
where: n is 0 or 1;
X is N or CH;
Y is NR₄ or CHR₄;
L₀ is a bond, O, -NR₅-, -C(O)-NR₅- or -NR₅-C(O)-;
L₁ is a bond or O;
R₁, R₂ are each independently selected from H, C₁-C₆ alkyl, halogen, CN, -C₁-C₆ alkyl-NR₆R₇, and -C(O)-NR₆R₇; wherein R₄, R₅, R₆, R₇ are each independently selected from H and C₁-C₆ alkyl;
R₃ is C₁-C₆ alkyl or haloC₁-C₆ alkyl; or R₃ is 5- or 6-membered heterocycloalkyl optionally substituted with one or more substituents selected from halogen and hydroxyl, such as piperazinyl, 4-methyl-piperazin-1-yl, piperidinyl, or 4-hydroxy-piperidin-1-yl.

In one specific embodiment, the present disclosure provides a compound of formula (II) as follows: 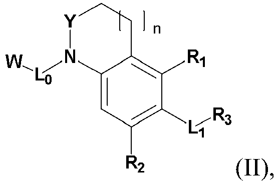 other variables R₁, R₂, R₃, n, Y, L₀, L₁, ring W are as described in formula (I).

In one preferred embodiment, Y is CHR₄, and R₄ is selected from H and C₁-C₃ alkyl in the compound of formula (II).

In one preferred embodiment, L₀ in the compound of formula (II) is a bond.

In one preferred embodiment, Y is CHR₄ and L₀ is a bond in the compound of formula (II).

In one specific embodiment, the present disclosure provides a compound of formula (III) as follows: 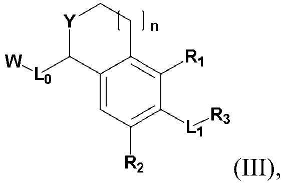 the other variables R₁, R₂, R₃, n, Y, L₀, L₁, ring W are as described in formula (I).

In one preferred embodiment, L₀ in the compound of formula (III) is a bond, O, S, -NH- or -NH(C=O)-.

In one specific embodiment, the present disclosure provides compounds of formula (IV), (V), (VI) as follows: 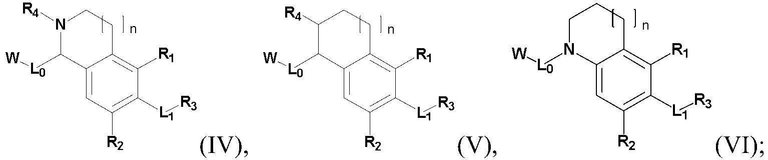 wherein the variables R₁, R₂, R₃, R₄, L₀, L₁, ring W are as described in formula (I).

In one preferred embodiment, R₁, R₂ in the compounds of formulae (IV), (V), (VI) are each independently selected from H, C₁-C₆ alkyl, halogen, CN, -haloC₁-C₆ alkyl and C₁-C₆ alkoxy.

In one preferred embodiment, R₁, R₂ in the compounds of formulae (IV), (V), (VI) are each independently selected from H, C₁-C₃ alkyl, halogen and CN.

In one embodiment of the present disclosure, the compound of formula (I) is selected from the following exemplified compounds:

| | | | |
|---|---|---|---|
| HANT-100 | 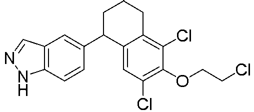 | HANT-101 | 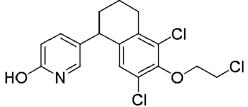 |
| HANT-102 | 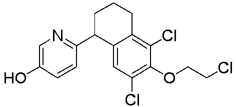 | HANT-103 | 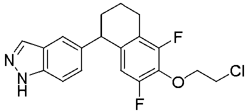 |
| HANT-105 | 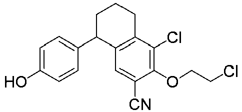 | HANT-106 | 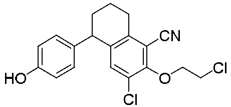 |
| HANT-107 | 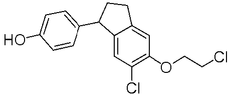 | HANT-108 | 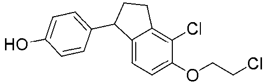 |
| HANT-109 | 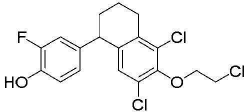 | HANT-110 | 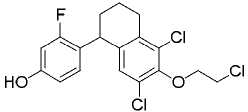 |
| HANT-112 | 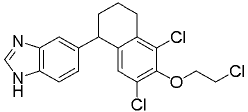 | HANT-113 | 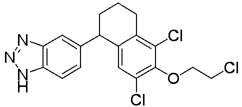 |
| HANT-114 | 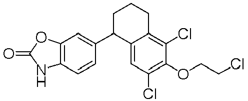 | HANT-115 | 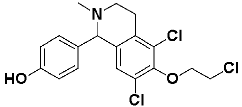 |
| HANT-117 | 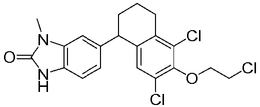 | HANT-118 | 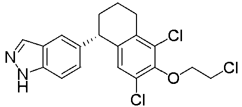 |
| HANT-121 | 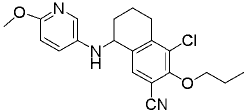 | HANT-122 | 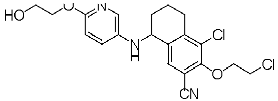 |
| HANT-123 | 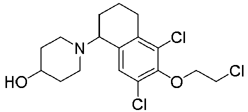 | HANT-124 | 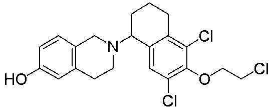 |
| HANT-125 | 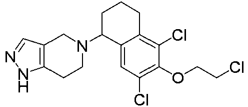 | HANT-126 | 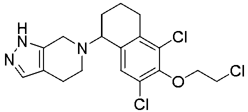 |
| HANT-127 | 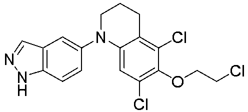 | HANT-132 | 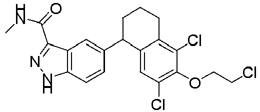 |
| HANT-131 | 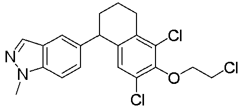 | HANT-134 | 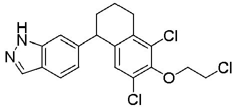 |
| HANT-133 | 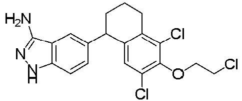 | HANT-137 | 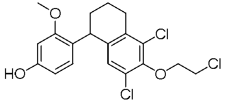 |
| HANT-135 | 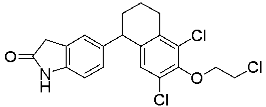 | HANT-144 A | 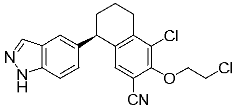 |
| HANT-144 B | 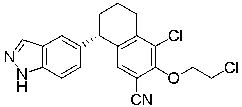 | HANT-146 | 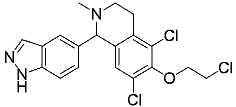 |
| HANT-146 A | 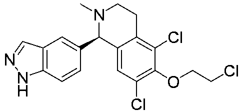 | HANT-146 B | 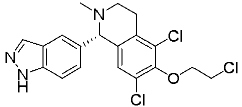 |
| HANT-148 | 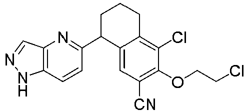 | HANT-149 | 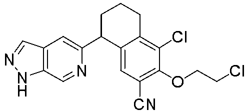 |
| HANT-150 A | 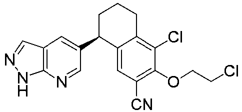 | HANT-150 B | 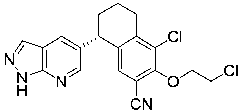 |
| HANT-164 | 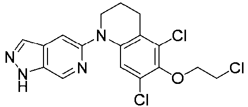 | HANT-165 | 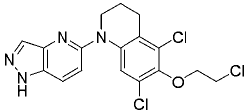 |
| HANT-166 | 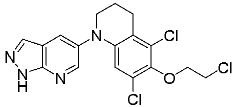 | HANT-184 | 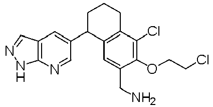 |
| HANT-170 | 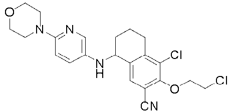 | HANT-171 | 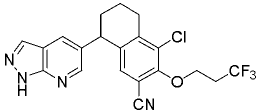 |
| HANT-173 | 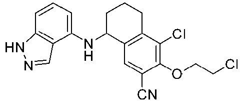 | HANT-175 | 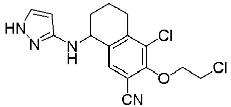 |
| HANT-183 | 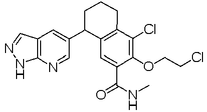 | HANT-186 | 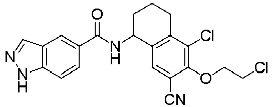 |
| HANT-187 | 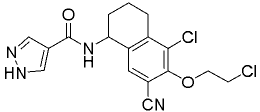 | HANT-188 | 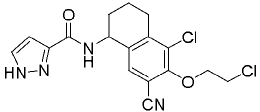 |
| HANT-191 A | 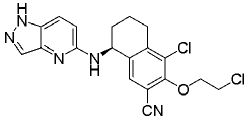 | HANT-191 B | 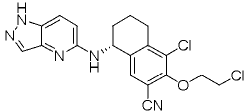 |
| HANT-191 | 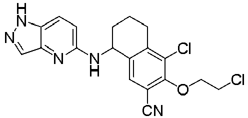 | HANT-192 | 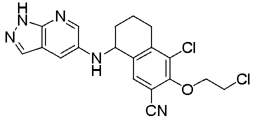 |
| HANT-192 A | 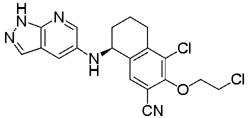 | HANT-192 B | 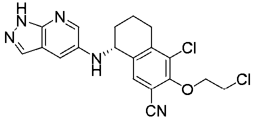 |
| HANT-193 | 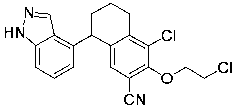 | HANT-194 | 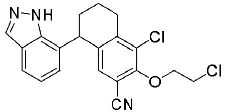 |
| HANT-196 | 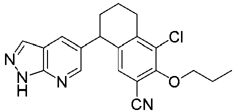 | HANT-200 | 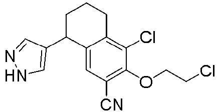 |
| HANT-203 | 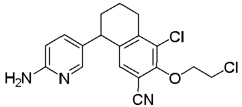 | HANT-202 | 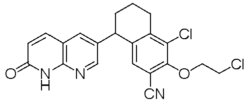 |
| HANT-204 A | 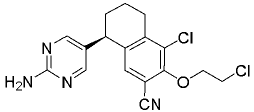 | HANT-204 | 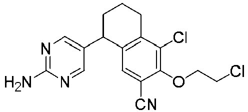 |
| HANT-205 | 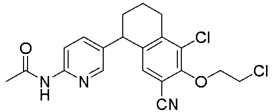 | HANT-204 B | 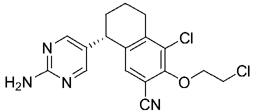 |
| HANT-207 | 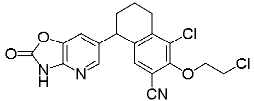 | HANT-206 | 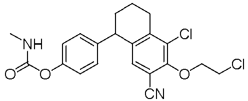 |
| HANT-209 | 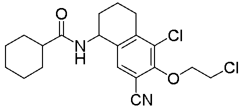 | HANT-208 | 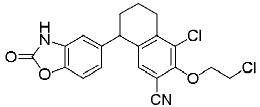 |
| HANT-211 | 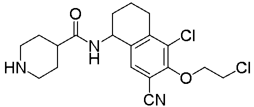 | HANT-210 | 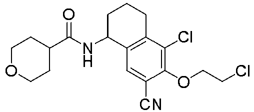 |
| HANT-212 | 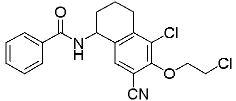 | HANT-213 | 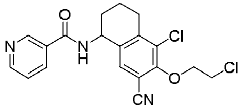 |
| HANT-214 | 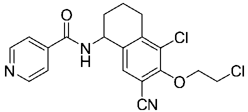 | HANT-215 | 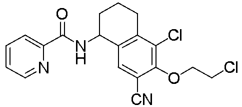 |
| HANT-216 | 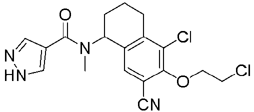 | HANT-218 | 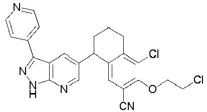 |
| HANT-220 | 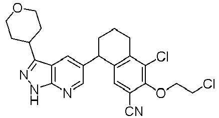 | HANT-224 | 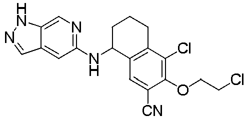 |
| HANT-225 | 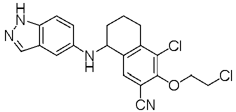 | HANT-226 | 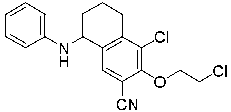 |
| HANT-227 | 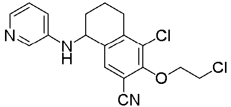 | HANT-228 | 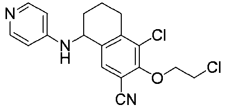 |
| HANT-229 | 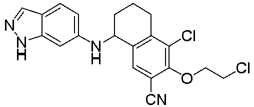 | HANT-232 | 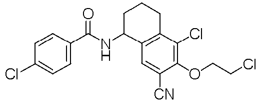 |
| HANT-233 | 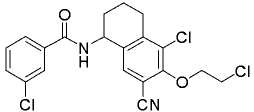 | HANT-234 | 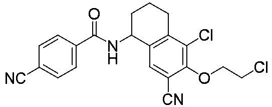 |
| HANT-235 | 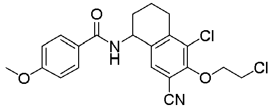 | HANT-239 | 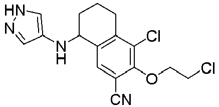 |
| HANT-240 | 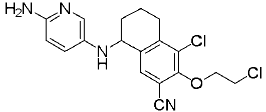 | HANT-241 | 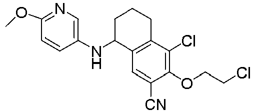 |
| HANT-246 | 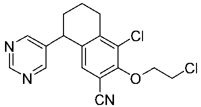 | HANT-247 | 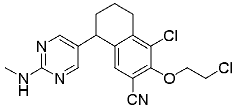 |
| HANT-249 | 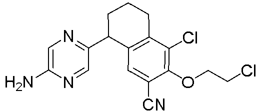 | HANT-248 | 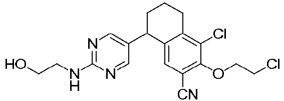 |
| HANT-251 | 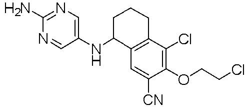 | HANT-250 | 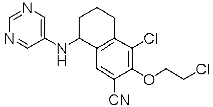 |
| HANT-252 | 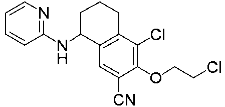 | HANT-254 | 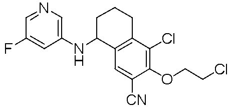 |
| HANT-255 | 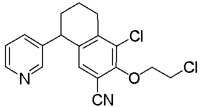 | HANT-256 | 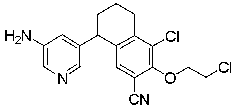 |
| HANT-257 | 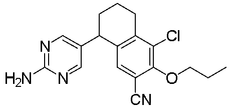 | HANT-261 | 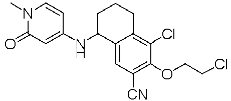 |
| HANT-262 | 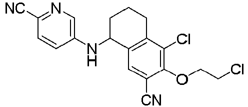 | HANT-263 | 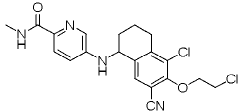 |
| HANT-264 | 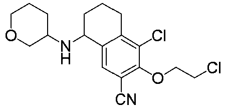 | HANT-265 | 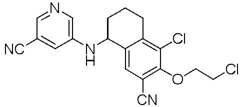 |
| HANT-266 | 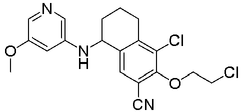 | HANT-267 | 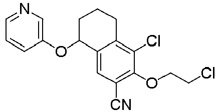 |
| HANT-268 | 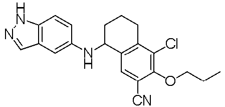 | HANT-269 | 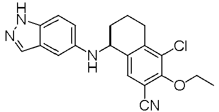 |
| HANT-334 | 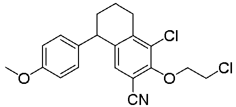 | HANT-335 | 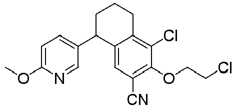 |
| HANT-336 A | 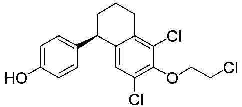 | HANT-336 B | 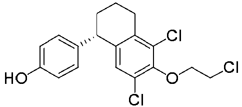 |
| HANT-337 | 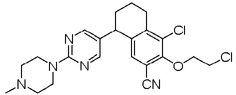 | HANT-338 | 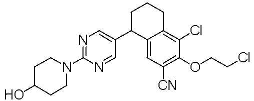 |
| HANT-339 | 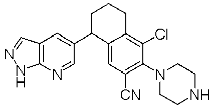 | HANT-340 | 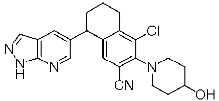 |
| HANT-948 | 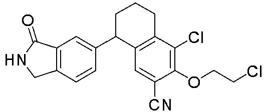 | HANT-949 | 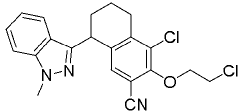 |
| HANT-950 | 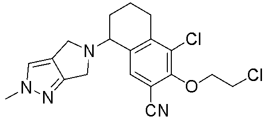 | HANT-977 | 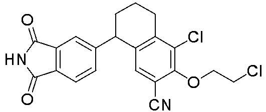 |
| HANT-978 | 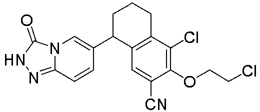 | HANT-979 | 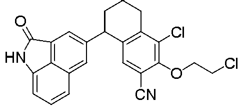 |
| HANT-982 | 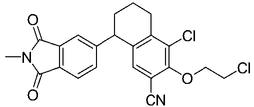 | HANT-983 | 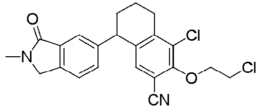 |

On the other hand, the present disclosure provides a method for preparing the compound of the present disclosure.

In one embodiment, the present disclosure also provides an intermediate for preparing the compound of the present disclosure and a preparation method thereof.

On the other hand, the present disclosure provides a composition comprising at least one compound or the pharmaceutically acceptable salt thereof of the present disclosure.

In one embodiment, the present disclosure provides a pharmaceutical composition comprising at least one compound or the pharmaceutically acceptable salt thereof of the present disclosure, as well as at least one pharmaceutically acceptable carrier, diluent or excipient.

In another aspect, the present disclosure provides a pharmaceutical composition comprising a therapeutically effective amount of at least one compound of the present disclosure, or the stereoisomer, the geometric isomer, the tautomer, the solvate, the hydrate or the pharmaceutically acceptable salt thereof, and pharmaceutically acceptable excipient.

In another aspect, the present disclosure provides pharmaceutical combination products comprising at least one compound of the present disclosure or the pharmaceutically usable salt thereof and one or more other active agents.

In another aspect, the present disclosure provides the use of the compound of formula (I), or the stereoisomer, the geometrical isomer, the tautomer, the solvate, the hydrate, or the pharmaceutically acceptable salt thereof, in the preparation of drugs for use in the prevention, treatment, or alleviation of disorders or diseases caused by abnormal androgen activity in patients.

In one embodiment, said aberrant androgenic activity is caused by an androgen receptor containing a mutation or deletion in the structural domain of the LBD.

On the other hand, the present disclosure provides methods of preventing, treating, or mitigating a disorder or disease mediated by one or more androgenic abnormal activities, the method comprising administering to an individual in need of such treatment an effective amount of the compound of formula I, or the stereoisomer, the geometrical isomer, the tautomer, the solvate, the hydrate, or the pharmaceutically acceptable salt thereof, or the pharmaceutical composition comprising them.

In some embodiments of the present disclosure, said disorder or disease includes, but is not limited to tumour, such as prostate cancer, breast cancer, other prostate disorders, breast cancer, acne, hirsutism, hidradenitis suppurativa, androgenetic alopecia, cryptorchidism, androgen insensitivity, Kennedy's disease, etc.

In some embodiments of the present disclosure, said prostate cancer includes, but is not limited to, metastatic desmoplasia-resistant prostate cancer, primary/focal prostate cancer, locally progressive prostate cancer, recurrent prostate cancer, non-metastatic castration-resistant prostate cancer, metastatic castration-resistant prostate cancer, metastatic prostate cancer, and hormone-sensitive prostate cancer.

Said breast cancer includes, but is not limited to, Luminal A (ER+/PR+, HER-2-), Luminal B (ER+/PR+, HER-2+), HER-2+ (ER-/PR-/HER-2+) and Basal-like (ER-/PR-/HER-2-) breast cancers.

In specific embodiments, using the Envision combined with GraphPad Prism (6.0) and/or GraphPad Prism (6.0) two-way ANOVA with Dunnett and Tukey test analysis disclosed herein, the compounds of the present disclosure have an IC₅₀ ≤ 2 µM, preferably IC₅₀ ≤ 1.5 µM, more preferably IC₅₀ ≤ 1 µM, even more preferably IC₅₀ ≤ 0.5 µM, and most preferably IC₅₀ ≤ 0.2 µM.

It should be understood that within the scope of the present disclosure, the technical features defined in each of the technical solutions the present disclosure and the specific technical features described in the following (as examples) can be combined with each other to form new or preferred technical solutions that are not described one by one in the specification. It is also understood that each individual element of the embodiment is its own independent embodiment.

### Terminology

In the present disclosure, unless otherwise explicitly stated, the terms used in the present disclosure have the meanings defined below. Terms not explicitly defined in the present disclosure have general meanings that are generally understood by those skilled in the art.

When a group has a wavy line " 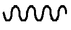 ", the wavy line represents the connection position between the group and the rest of the molecule.

As used herein, "- - - -" represents single or double bonds. Those skilled in the art can determine - - - - whether to represent a single bond or a double bond based on the valence of the relevant ring atoms and the connected groups, which falls within the scope of their abilities. Those skilled in the art can understand that the relevant rings can be saturated, partially saturated or aromatic.

As used herein, "heteroatoms" refer to nitrogen (N), oxygen (O), or sulfur (S) atoms, particularly nitrogen or oxygen atoms, each of which can be substituted or unsubstituted, including their oxidized forms. Examples of heteroatoms include but are not limited to -O-, -N=, -NR-, -S-, -S(O)- and -S(O)₂-, wherein R is hydrogen, C₁-C₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Any heteroatom with an unsatisfied valence bond is considered to have a hydrogen atom sufficient to satisfy the valence bond, unless otherwise indicated.

As used herein, "halogen" or "halo" refers to fluorine, chlorine, bromine and iodine. The preferred halogen as a substituent is fluorine and chlorine.

As used herein, "alkyl" refers to a monovalent hydrocarbon group of completely saturated straight or branched chains. Alkyl preferably contains 1-20 carbon atoms, more preferably 1-16 carbon atoms, 1-10 carbon atoms, 1-6 carbon atoms or 1-4 carbon atoms. "C₁-C₆ alkyl" represents an alkyl with 1-6 carbon atoms, and "C₁-C₃ alkyl" represents an alkyl with 1-3 carbon atoms. Representative examples of alkyl include but are not limited to methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, etc.

As used herein, "alkoxy" refers to an alkyl-O group, wherein alkyl as defined herein. "C₁-C₆ alkoxy" represents an alkoxy with 1-6 carbon atom. Representative examples of alkoxy include but are not limited to methoxy, ethoxy, propanoxy, 2-propanoxy, butoxy, tert butoxy, pentoxy, hexoxy, cyclopropyloxy, cyclohexyloxy, etc. Alkoxy preferably contains 1-6 or 1-4 carbon atoms.

As used herein, "haloC₁-C₆ alkyl" refers to C₁-C₆ alkyl as defined herein where one or more hydrogen atoms are substituted by one or more halogens, for example, fluoromethyl, difluoromethyl, trifluoromethyl, trichloromethyl, difluoroethyl, trifluoroethyl, chloromethyl, dichloromethyl, trichloromethyl, chloroethyl, dichloroethyl, trichloroethyl etc.

As used herein, "haloC₁-C₆ alkoxy" refers to C₁-C₆ alkoxy as defined above with one or more hydrogen atoms substituted by one or more halogens, for example, fluoromethoxy, difluoromethoxy, trifluoromethoxy, difluoroethoxy, trifluoroethoxy, chloromethoxy, dichloromethoxy, trichloromethoxy, chloroethoxy, dichloroethoxy, trichloroethoxy, etc.

As used herein, "cycloalkyl" refers to saturated or partially saturated non-aromatic carbon rings, including monocyclo, bicyclo, or tricyclo, with 3-12 ring carbon atoms, preferably 3-10 ring carbon atoms, for example, 3-8, 3-7 or 4-7 ring carbon atoms. Exemplary monocyclic cycloalkyls include but are not limited to cyclopropyl, cyclobutyl, cyclopentyl, cyclopentenyl, cyclohexyl and cyclohexene. Exemplary bicyclic cycloalkyls include bornyl, tetrahydronaphthyl, decahydronaphthyl, bicyclo[2.1.1]hexyl, bicyclo[2.2.1]heptyl, bicyclo[2.2.1]heptyl, 6,6-dimethyldicyclo[3.1.1]heptyl, 2,6,6-trimethyldicyclo[3.1.1]heptyl, bicyclo[2.2.2]octyl, etc. Exemplary tricyclic cycloalkyls include diamond alkyl, etc.

As used herein, "aryl" refers to aromatic hydrocarbon ring system group having 6 to 18 carbon atoms in the ring portion and at least one aromatic ring. Aryl preferably refers to C₆-C₁₂ aryl or C₆-C₁₀ aryl. Non-limiting examples of aryl include phenyl, biphenyl, naphthyl or anthryl.

As used herein, "arylalkyl" is an alkyl group as described above substituted by an aryl group as described above. Preferably, arylalkyl is C₆-C₁₂ aryl-C₁-C₆ alkyl. Non-limiting examples of arylalkyl include benzyl, naphthylmethyl, and the like.

As used herein, "heteroaryl" refers to a 5-14 membered, preferably 5-10 membered, more preferably 5-7 membered, or 5-6 membered aromatic ring system containing 1-8, preferably 1-4, further preferably 1-3, more preferably 1 or 2 heteroatoms selected from N, O and S, including a single ring, a double ring, or a fused multi ring, with the remaining ring atoms being carbon atoms. The preferred heteroaryl is 5-10 membered heteroaryl, more preferably 5-7 membered heteroaryl or 5-6 membered heteroaryl, each containing 1, 2 or 3 heteroatoms selected from N, O and S. Examples of heteroaryl include but are not limited to pyrrolyl, furanyl, thienyl, pyrazolyl, imidazolyl, thiazolyl, isothiazolyl, indolyl, benzotriazolyl, benzoimidazolyl, benzothiazolyl, benzooxazolyl, quinolinyl, isoquinolinyl, cinnolinyl, quinazolinyl, quinoxalinyl, tetrahydroquinolinyl, oxazolopyridinyl, imidazolopyridinyl, 7-azaindolyl, 6-azaindolyl, 5-azaindolyl, 4-azaindolyl, azaindazolyl.

As used herein, "heterocycle" refers to completely saturated or unsaturated, aromatic or non-aromatic cyclic groups, for example, a ring system consisting of 4- to 7-membered single rings, 7- to 12-membered double rings, or 10- to 15-membered triple rings, preferably 4- to 12-membered single rings or double rings, which contain at least one heteroatom on a ring containing at least one carbon atom. A ring containing heteroatoms of the heterocycle can contain 1-3, 1-4, 1-5 or 1-6, preferably 1, 2 or 3 heteroatoms selected from nitrogen, oxygen, and sulfur atoms, wherein nitrogen and sulfur heteroatoms can also be optionally oxidized, For example, the sulfur heteroatoms may form -S(O)- or -S(O)2- structures.

Exemplary monocyclic heterocycles include fully saturated, partially saturated or aromatic heterocycles, for example, pyrrolidine, pyrrole, pyrazole, oxocyclobutane, oxocyclooxetane, oxocyclohexane, pyrazoline, imidazole, imidazoline, imidazolidine, triazole, thiazole, thiadiazole, thiazolidine, isothiazole, isothiazolidine, furan, tetrahydrofuran, thiophene, piperidine, piperazine, 2-oxopiperazine, 2-oxopiperidine, 2-oxypyrrolidine, 4-piperidone, pyridine, pyrazine, pyrimidine, pyridazine, tetrahydropyran, morpholine, thiomorpholine, sulfanomorpholino, sulfonomorpholino, 1,3-dioxolane and tetrahydro-1,1-dioxothiophene, 1,1,4-trioxo-1,2,5-thiadiazolidin-2-, etc.

Exemplary bicyclic heterocycles include fully saturated, partially saturated or aromatic heterocycles, for example, indole, dihydroindole, Indazole, benzothiazole, benzoxazole, benzimidazole, benzopyrazole, benzotriazole, quinoline, isoquinoline, tetrahydroisoquinoline, pyridine oxazole, pyridine imidazole, pyridine-pyridine, pyridine-pyrazole, etc.

"Heterocycloalkyl" means a group formed by the loss of one or more hydrogens atoms from a heterocycle as defined above. Heterocycloalkyl group may be attached to other parts of the molecule at either the heteroatom or the carbon atom. Preferably, the heterocycloalkyl group is a 4-12-membered heterocycloalkyl group containing 1-4 heteroatoms selected from nitrogen, oxygen and sulfur, such as a 5- to -7-membered heterocycloalkyl group.

As used herein, "acyl" refers to group R'-C(O)-, wherein R' is C₁-C₆ alkyl, or C₆-C₁₂ aryl-C₁-C₆ alkyl as defined above. Representative examples of acyl groups include, but are not limited to, formyl, acetyl, propionyl, butyryl, pentanoyl, hexanoyl, benzoyl, and the like. As used herein, "oxo" refers to the group (=O).

The term "optionally" used herein refers to the events described subsequently that may or may not occur, and this description includes both the situations in which the event occurred and the situations in which it did not occur. For example, "optionally substituted alkyl" includes "unsubstituted alkyl" and "substituted alkyl" as defined herein. "Optionally substituted with halogens" includes situations where "substituted with halogens" and situations where "not substituted with halogens", such as being substituted with 0-3 halogens. Those skilled in the art should understand that for any functional group containing one or more substituents, the group does not include any spatially impractical, chemically incorrect, synthetic infeasible, and/or inherently unstable substitution patterns.

The term "substituted" used herein refers to one or more hydrogen atoms on a given atom or group being replaced by one or more substituents selected from a given substituent group, provided that they do not exceed the normal valence of the given atom. When the substituent is an oxo (i.e. =O), the two hydrogen atoms on a single atom are replaced by oxygen. There is no oxygen substituents present on the aromatic portion. When a ring system (such as a carbon ring or heterocycle) is replaced by a carbonyl group or double bond, it is intended that the carbonyl group or double bond is part of the ring (i.e., within the ring). Only when the combination of substituents and/or variables leads to chemically correct and stable compounds, such combinations are allowed. A chemically correct and stable compound means that it is sufficiently stable to be separated from the reaction mixture and its chemical structure can be determined, and subsequently formulated into a preparation with at least practical utility. For example, in the absence of a clear list of substituents, the terms "substituted" used herein refer to one or more hydrogen atoms on a given atom or group being independently replaced by one or more substituents, such as 1, 2, 3 or 4 substituents. When an atom or group is replaced by multiple substituents, the substituents can be the same or different. Alkyl, alkenyl, alkoxy, cycloalkyl, heteroaryl, heterocycle, heterocycloalkyl, carbonyl, sulfonyl, sulfinyl, and other functional groups as used herein can be substituted with substituents, and the substituents include but not limited to OH, Boc, halogen, cyanide, substituted or unsubstituted alkyl, substituted or unsubstituted alkoxy, substituted or unsubstituted cycloalkyl, substituted or unsubstituted heterocycloalkyl; NRR', C(O)R, SO₂R, C(O)NRR' or C(O)OR, and R and R' are each independently selected from H and substituted or unsubstituted alkyl.

As used herein, "pharmaceutically acceptable salts" includes acid addition salts and base addition salts. Pharmaceutically acceptable salt includes those obtained by reacting an active compound acting as a base with an inorganic or organic acid to form a salt, such as hydrochloride, hydrobromide, sulphate, nitrate, phosphate, methanesulfonate, oxalate, maleate, succinate, citrate, formate, benzoate, fumarate, tartrate, salicylate, mandelate, carbonate, etc. It is known to those skilled in the art that acid addition salts are prepared by reaction of the described compounds with suitable inorganic or organic acids by any of a number of known methods.

As used herein, "pharmaceutically acceptable excipients" include any and all solvents, dispersants, coatings, surfactants, antioxidants, preservatives (such as antibacterial agents, antifungal agents), isotopes, absorption retardants, salts, preservatives, drugs, drug stabilizers, adhesives, excipients, disintegrants, lubricants, sweeteners, correctors, dyes, similar substances and their combinations, which is well-known to those skilled in the art.

Compounds of the present disclosure or pharmaceutically usable salts thereof contain one or more asymmetric centers, thus producing enantiomers, diastereomers, and other stereoisomers that can be defined as (R)- or (S)- in absolute stereochemistry, or as (D)- or (L)-for amino acids. It is intended herein to include all such possible isomers as well as their racemic and optically pure forms, whether or not they are specifically described herein. Optically active (+)- and (-)-, (R)- and (S)-, or (D)- and (L)- isomers may be prepared using chiral synthesis or chiral reagents, or split by conventional techniques such as chromatography and hierarchical crystallization. Conventional techniques for the preparation/separation of individual enantiomers include chiral synthesis from suitable optically pure precursors or splitting of racemates (or racemates of salts or derivatives) using, for example, chiral high-pressure liquid chromatography (HPLC).

"Stereoisomers" are compounds comprising the same atoms bonded by the same bonds but having different three-dimensional structures that are not interchangeable. The present disclosure contemplates a variety of stereoisomers and mixtures thereof, and includes "enantiomers", which refers to two such stereoisomers whose molecules are non-overlapping mirror images of each other.

"tautomer" refers to the transfer of a proton from one atom of a molecule to another atom of the same molecule. The present disclosure encompasses any of the reciprocal isomers of any of the described compounds.

As used herein, "solvate" means solvent addition forms containing stoichiometric or non-stoichiometric solvents. If the solvent is water, the solvent compound formed is a hydrate, and when the solvent is ethanol, the solvent compound formed is an ethanol compound. Hydrates are formed by combining one or more molecules of water with one molecule of said substance, wherein the water retains its molecular state of H₂O, and such combinations can result in the formation of one or more hydrates, such as hemihydrate, monohydrate and dihydrate.

As used herein, the "therapeutically effective amount" of the compound of the present disclosure refers to the amount of the compound of the present disclosure that can cause individual biological or medical reactions, improve symptoms, slow or delay disease progression, or prevent disease.

As used herein, "individual" refers to animals. Preferably, animals are mammals. Individuals also refer to primates (for example, humans), cows, sheep, goats, horses, dogs, cats, rabbits, rats, mice, fish, birds, etc. In a preferred embodiment, the individual is a human.

As used herein, "inhibition" refers to the alleviation or inhibition of a specific patient, symptom or disease, or a significant decrease in biological activity or process baseline activity.

As used herein, in one embodiment, the term "treatment" or any disease or condition in an embodiment refers to the improvement of diseases or conditions (i.e., preventing or slowing down the development of the disease or at least one clinical symptom). In another embodiment, "treatment" refers to improving at least one physical parameter that may not be perceived by the patient. In another embodiment, "treatment" refers to the regulation of diseases or conditions on the body (such as stable and perceptible symptoms) or physiology (such as stable body parameters) or both.

### Beneficial effect

The compounds of the present disclosure are inhibitors of the N-terminus of the androgen receptor, are highly selective for the AR receptor, and show good proliferation inhibition in cells expressing AR splice variants (AR-V) lacking LBD of the androgen receptor, in particular cells expressing AR splice variants (AR-vs) which are resistant to second-generation AR antagonists.

### General Synthesis Procedures

In an embodiment, the compound of the present disclosure can be synthesized through the following general synthesis scheme, where each variable is defined herein, and the specific reaction conditions are the same as in the examples.

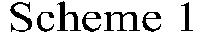

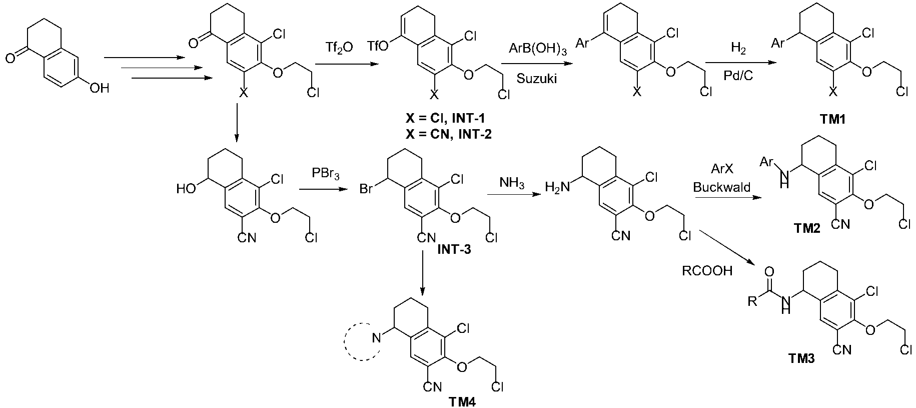

From 6-hydroxy-1-tetrahydronaphthalenone, 5,7-disubstituted alkoxynaphthalenones could be obtained by halogenation, coupling and alkylation, respectively, which would react with Tf₂O to afford the important intermediate INT-1/2. Suzuki coupling between INT-1/2 and different boric acids or boron esters followed by reductive hydrogenation would obtain the first type of target product TM1. Alternatively, starting from alkoxynaphthones, the carbonyl group could be reduced to an alcohol and reacted with PBr₃ to afford the important intermediate INT-3; treating INT-3 with ammonia would convert Br to an amino group, and the afforded benzylamines could undergo Buckwald coupling or amide condensation reaction to yield TM2 or TM3. Meanwhile, INT-3 could be directly substituted with heterocycloalkylamino groups to yield TM4.

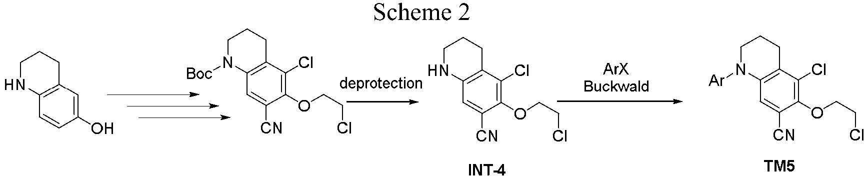

Alternatively, starting from 6-hydroxy-1,2,3,4-tetrahydroquinoline, through Boc protection, halogenation, alkylation followed by acid deprotection, would lead to the 5,7-position bis-substituted tetrahydroquinoline intermediate INT-4; INT-4 undergoes Buckwald coupling with various haloaromatic rings to obtain TM5.

### DETAILED DESCRIPTION OF EMBODIMENTS

In the present application, when the chemical name and structural formula are inconsistent, the one shown in the structural formula shall prevail, unless it can be inferred from the context that the chemical name rather than the structural formula is correct.

The present disclosure will be further described in combination with specific examples. It should be understood that these examples are only used to illustrate the present disclosure and not to limit the scope of the present disclosure. The experimental methods without specific conditions in the following embodiments are usually under conventional conditions, or the conditions recommended by the manufacturer. Unless otherwise stated, percentages and parts are weight percentages, weight parts.

The experimental materials and reagents used in the following examples can be obtained from commercial channels unless otherwise stated.

In each embodiment, experimental instrument descriptions (e.g., ¹H NMR was recorded by a Varian Mercury-300 or Varian Mercury-400 NMR instrument, ¹³C NMR was recorded by a Varian Mercury-400 or Varian Mercury-500 or Varian Mercury-600 NMR instrument, chemical shifts were expressed as δ (ppm); mass spectra were recorded by Finnigan/MAT-95 (EI) with Finnigan LCQ/DECA and Micromass Ultra Q-TOF (ESI) type mass spectrometers; silica gel for reversed-phase HPLC separations was 200-300 mesh. The method of SFC purification is (Column: Chiralpak IG 250mm*4.6mm 5um, mobile phase: Hex-EtOH, 30°C).

Among them, the abbreviations of the reagents are listed below:
DCM: Dichloromethane; DCE: 1,2-dichloroethane; THF: Tetrahydrofuran; MeCN: acetonitrile; DMF: N,N-dimethylformamide; Tol: Toluene; TFA: Trifluoroacetic acid; PE: Petroleum ether; EA: Ethyl acetate; TEA: triethylamine; DIEA: N,N-diisopropylethylamine; HEPES: 4-(2-Hydroxyethyl)-1-piperazineethanesulfonic acid; Tf₂O: Trifluoromethanesulfonic anhydride; DBAD: Dibenzyl azodicarboxylate; Trt-Cl: Trityl chloride; TosCl: Tosyl chloride; Triphenylchloromethane; ACN: acetonitrile; (Bpin)₂: Bis(pinacolato)diboron; AcOK: Potassium acetate; AcOH: Acetic acid; Pybop: Benzotriazol-1-yl-oxytripyrrolidinophosphonium hexafluorophosphate; DHP: 3,4-Dihydro-2*H*-pyran; BINAP: 1,1'-binaphthyl-2,2'-bis(diphenylphosphine); PTSA: p-Toluenesulfonic acid; NMP: 1-Methyl-2-pyrrolidinone; EGTA: Ethylenebis(oxyethylenenitrilo)tetraacetic acid; DTT: DL-Dithiothreitol; EDTA: Ethylenediaminetetraacetic acid; DIPEA: N,N-Diisopropylethylamine; Raney Ni: Rennie's Nickel; Boc₂O: Di-tert-butyl dicarbonate; NBS: N-Bromosuccinimide; NCS: N-Chlorosuccinimide; NIS: N-Iodosuccinimide; Pd(dppf)Cl₂: 1,1'-bis(diphenylphosphino)ferrocene palladium dichloride; Pd₂(dba)₃: Tri(dibenzalacetone)dipalladium; Pd(OAC)₂: Fumaronitrile: Fumaric acid nitrile; P(*n*Bu)₃: tri-n-butyl; LDA: Lithium diisopropylamide; HATU: 2-(7-azobenzotriazole)-N,N,N',N'-tetramethylurea hexafluorophosphate; HBTU: O-Benzotriazole-tetramethyluronium hexafluorophosphate; Xphos: 2-(Dicyclohexylphosphino)-2',4',6'-tri-i-propyl-1,1'-biphenyl; Xantphos: 4,5-Bis(diphenylphosphino)-9,9-dimethylxanthene; tBuBrettphos-Pd-G3: [(2-Di-tert-butylphosphino-3,6-dimethoxy-2',4',6'-triisopropyl-1,1'-biphenyl)-2-(2'-amino-1,1'-b iphenyl)]palladium(II) methanesulfonate; TLC: Thin-layer chromatography; RT: Retention time; LCMS: Liquid chromatography-mass spectrometry.

### Synthesis of Key Intermediates

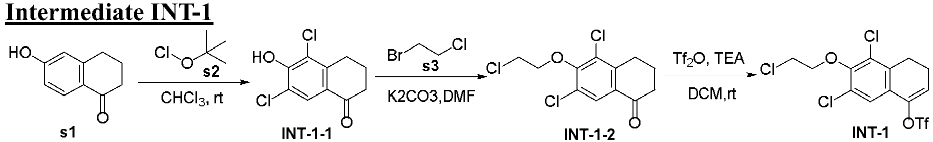

### Step 1: Synthesis of 5,7-dichloro-6-hydroxy-3,4-dihydronaphthalen-1(2H)-one

Compound **S1** (10 g, 61.7 mmol) was added to trichloromethane (650 mL), then **S2** (16.2 mL, 142 mmol) was added at 0 °C and stirred overnight at room temperature. The crude reaction was concentrated to obtain **INT-1-1** as a yellow solid (9.5 g, Yield: 66.9%); LCMS (ESI): *m*/*z* = 231.0 [M+H] ⁺.

### Step 2: Synthesis of 5,7-dichloro-6-(2-chloroethoxy)-3,4-dihydronaphthalen-1(2H)-one

Compounds **INT-1-1** (5 g, 21.7 mmol) and **S3** (13.6 g, 95.5 mmol) were dissolved in DMF (80 mL), then potassium carbonate (3.3 g, 23.8 mmol) was added in and the reaction was kept at 35 °C for 26 h. The reaction solution was filtered and concentrated under reduced pressure to obtain **INT-1-2** (4.65 g, yield: 72.6%) as a yellow solid; LCMS (ESI): *m*/*z* = 293.0[M+H]⁺.

### Step 3: Synthesis of 5,7-dichloro-6-(2-chloroethoxy)-3,4-dihydronaphthalen-1-yl trifluoromethanesulfonic acid

Compounds **INT-1-2** (4.65 g, 15.9 mmol), TEA (5.12 g, 50.7 mmol) were dissolved in a three-necked flask containing DCM (100 mL), then Tf₂O (22.4 g, 79.5 mmol) was added at 0 °C under nitrogen protection and the reaction was stirred at room temperature overnight. The reaction solution was extracted with DCM, and the organic phase was concentrated under reduced pressure, and the residue was purified by flash chromatography to obtain the yellow oil **INT-1** (6.2 g, yield: 95.3%). LCMS (ESI): *m*/*z* =424.9[M+H]⁺; ¹H NMR (400 MHz, CDCl₃) δ 7.30 (s, 1H), 6.08 (t, *J =* 4.8 Hz, 1H), 4.28 (t, *J =* 6.3 Hz, 2H), 3.88 (t, *J =* 6.2 Hz, 2H), 2.98 (t, *J* = 8.3 Hz, 2H), 2.56 (td, *J =* 8.4, 4.8 Hz, 2H).

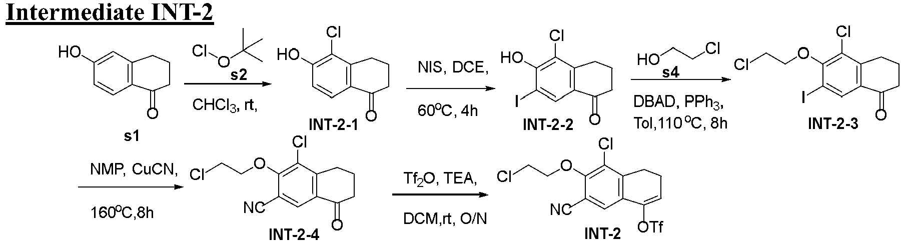

### Step 1: Synthesis of 5-chloro-6-hydroxy-3,4-dihydronaphthalen-1 (2H)-one

Compound **S1** (20 g, 125 mmol) was added to trichloromethane (300 mL), followed by addition of **S2** (12 g, 110 mmol) at 0 °C and stirred overnight at room temperature. The crude reaction was concentrated to obtain **INT-2-1** as a solid (18 g, Yield: 75%). LCSM (ESI): *m*/*z* = 197.0 [M+H]⁺.

### Step 2: Synthesis of 5-chloro-6-hydroxy-7-iodo-3,4-dihydronaphthalen-1(2H)-one

Compounds **INT-2-1** (18 g, 91.8 mmol) and NIS (26 g, 115 mmol) were dissolved in DCE (300 mL) and reacted at 60 °C for 4 h. The reaction was cooled to RT, filtrated, and concentrated under reduced pressure to afford **INT-2-2** as a brown solid (20 g, Yield: 77%). LCMS (ESI): *m*/*z* = 322.9[M+H]⁺.

### Step 3: Synthesis of 5-chloro-6-(2-chloroethoxy)-7-iodo-3,4-dihydronaphthalen-1(2H)-one

Compounds **INT-2-2** (20 g, 62 mmol), **S4** (15 g, 186 mmol), DBAD (14 g, 61 mmol), PPh₃ (16 g, 61 mmol) were dissolved in Tol (300 mL) and reacted under nitrogen protection at 110 °C for 8 h. The reaction was cooled to RT, concentrated under reduced pressure, and the residue was purified by flash chromatography to obtain white solid **INT-2-3** (15 g, yield: 85%). LCMS (ESI): m/z =384.9[M+H]⁺.

### Step 4: Synthesis of 4-chloro-3-(2-chloroethoxy)-8-oxo-5,6,7,8-tetrahydronaphthalene-2-carbonitrile

The compound **INT-2-3** (5 g, 13 mmol), CuCN (2.4 g, 26 mmol) and NMP (80 mL) reacted in a sealed tube under nitrogen protection at 120 °C for 8 h. The reaction was cooled to RT and extracted with EA. The organic phase was concentrated under reduced pressure, and the residue was purified by flash chromatography to obtain the white solid **INT-2-4** (3.2 g, Yield: 86%). LCMS (ESI): *m*/*z* =284.0[M+H]⁺.

### Step 5: Synthesis of 5-chloro-6-(2-chloroethoxy)-7-cyano-3,4-dihydronaphthalen-1-yl trifluoromethanesulfonic acid

**INT-2-4** (3.2 g, 11.3 mmol), TEA (3.4 g, 33.9 mmol) were dissolved in DCM (100 mL) in a three-necked flask, and Tf₂O (16 g, 56.5 mmol) was added at 0 °C under nitrogen protection and the reaction was stirred at room temperature overnight. The reaction solution was extracted with DCM, and the organic phase was concentrated under reduced pressure. The residue was purified by flash chromatography to obtain **INT-2** (3.5 g, yield: 66%) as a yellow solid. LCMS (ESI): *m*/*z* = 416.0 [M+H]⁺; ¹H NMR (400 MHz, CDCl₃) δ 7.46 (s, 1H), 6.15 (t, *J =* 4.8 Hz, 1H), 4.48 (t*, J =* 6.0 Hz, 2H), 3.90 (t, *J =* 6.0 Hz, 2H), 3.08 (t, *J =* 8.3 Hz, 2H), 2.66-2.56 (m, 2H).

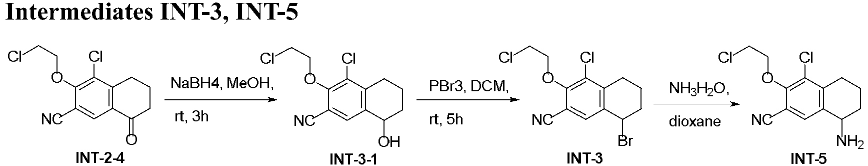

### Step 1: Synthesis of 4-chloro-3-(2-chloroethoxy)-8-hydroxy-5,6,7,8-tetrahydronaphthalene-2-carbonitrile

Compound **INT-2-4** (800 mg, 2.8 mmol) was added into methanol (10 mL), then NaBH4 (430 mg, 11.3 mmol) was added at 0 °C and the reaction was stirred at room temperature for 3 h. The reaction solution was concentrated and the residue was extracted with EA. The organic phase was dried and concentrated to obtain **INT-3-1** (700 mg, yield: 87%) as a solid. LCMS (ESI): *m*/*z* =286.0[M+H]⁺.

### Step 2: Synthesis of 8-bromo-4-chloro-3-(2-chloroethoxy)-5,6,7,8-tetrahydronaphthalene-2-carbonitrile

Compound **INT-3-1** (700 mg, 2.4 mmol) was added to DCM (10 mL), followed by PBr₃ (1.3 g, 4.8 mmol) at 0 °C. The mixture was stirred at room temperature for 5 h, then extracted with DCM. The organic phase was dried and concentrated, and the residue was purified by flash chromatography to obtain **INT-3** as a solid (500 mg, Yield: 59%). LCMS (ESI): *m*/*z* =347.9[M+H]⁺; ¹H NMR (400 MHz, CDCl₃) δ 7.55 (s, 1H), 5.45 (t, *J =* 3.7 Hz, 1H), 4.42 (td, *J* = 6.1, 1.7 Hz, 2H), 3.88 (t, *J =* 6.1 Hz, 2H), 3.18-3.05 (m, 1H), 2.82-2.65 (m, 1H), 3.18-3.05 (m, 1H), 3.18-3.05 (m, 1H), 2.18-3.05 (m, 1H), 2.18-3.05 (m, 1H).2.82-2.65 (m, 1H), 2.43-2.32 (m, 1H), 2.29-2.18 (m, 1H), 2.12-1.96 (m, 2H).

### Step 3: Synthesis of 8-amino-4-chloro-3-(2-chloroethoxy)-5,6,7,8-tetrahydronaphthalene-2-carbonitrile

Compound **INT-3** (200 mg, 0.56 mmol) was dissolved in dioxane (510 mL) in a sealed tube, then ammonia (5 mL) was added in. The sealed tube was stirred at 40 °C for 12 h. The crude reaction mixture was filtered, concentrated, and purified by flash chromatography (DCM: MeOH= 10:1) to obtain **INT-5** (110 mg, Yield: 69%). LCMS (ESI): *m*/*z* =285.1 [M+H]⁺; ¹H NMR (400 MHz, DMSO-d₆) δ 7.92 (s, 1H), 4.37 (t, *J =* 5.2 Hz, 2H), 3.96 (t, *J =* 4.8 Hz, 2H), 3.80 (dd, *J* = 7.2,4.8 Hz, 1H), 2.73 (t, *J* = 8.0 Hz, 2H), 2.22 (brs, 2H), 1.97-1.82 (m, 2H), 1.74-1.67 (m, 1H), 1.56-1.48 (m, 1H).

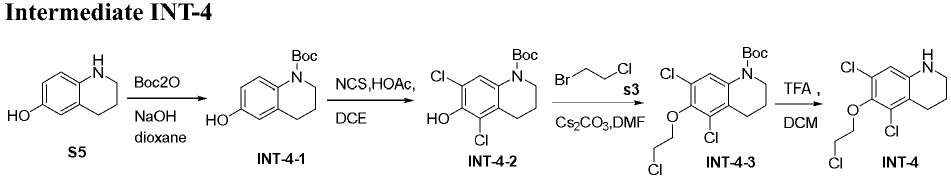

### Step 1: Synthesis of tert-butyl 6-hydroxy-3,4-dihydroquinoline-1(2H)-carboxylate

Compound **S5** (2 g, 13.4 mmol) was added to dioxane (40 mL), followed by Boc anhydride (4.5 g, 20.7 mmol) and aq. NaOH (40 mL, 1M). The mixture was reacted at room temperature for 5 h then concentrated. The residue was washed with iced NH₄Cl, extracted with EA, and dried with anhydrous Na₂SO₄. The organic phase was concentrated under reduced pressure, and the residue was purified by flash chromatography to obtain crude product as a white solid **INT-4-1** (2.3 g, yield: 69.6%). LCMS (ESI): *m*/*z* =250.1[M+H]⁺.

### Step 2: Synthesis of tert-butyl 5,7-dichloro-6-hydroxy-3,4-dihydroquinoline-1(2 H)-carboxylate

Compound **INT-4-1** (4.8 g, 19.2 mmol) was added to DCE (120 mL), followed by NCS (5.9 g, 44.2 mmol) and acetic acid (24 mL). The mixture was reacted at room temperature for 8 h then concentrated. The residue was washed with iced sodium bicarbonate, extracted with DCM and dried with anhydrous Na₂SO₄. The organic phase was concentrated under reduced pressure, and the residue was purified by flash chromatography to obtain crude product **INT-4-2** as a white solid (3.6 g, yield: 59%). LCMS (ESI): *m*/*z*=318.1[M+H]⁺.

### Step 3: Synthesis of tert-butyl 5,7-dichloro-6-(2-chloroethoxy)-3,4-dihydroquinoline-1(2H)-carboxylate

Compound **INT-4-2** (3.6 g, 11.3 mmol), **S3** (3.25 g, 22.7 mmol) was dissolved in DMF (30 mL), and then cesium carbonate (4.4 g, 13.5 mmol) was added in. The reaction was stirred at 35 °C for 16 h. The crude reaction mixture was washed with aq. NH₄Cl and extracted with EA and dried with anhydrous Na₂SO₄. The organic phase was concentrated under reduced pressure, and the residue was purified by flash chromatography to obtain compound **INT-4-3** as colourless oil (1.35 g, yield: 31.3%). LCMS (ESI): *m*/*z* = 380.1 [M+H]⁺.

### Step 4: Synthesis of 5,7-dichloro-6-(2-chloroethoxy)-1,2,3,4-tetrahydroquinoline

Compound **INT-4-3** (1.35 g, 3.5 mmol) was dissolved in DCM (20 mL), followed by addition of TFA (8 mL) The reaction was stirred at room temperature for 2 h. The reaction solution was adjusted to pH~7 with aq. sodium bicarbonate under an ice bath, then extracted with DCM. The organic phase was concentrated under reduced pressure to obtain a white solid, **INT-4** (890 mg, Yield: 89.5%). LCMS (ESI): *m*/*z* = 280.0 [M+H]⁺; ¹H NMR (400 MHz, CDCl₃) δ 6.41 (s, 1H), 4.16 (t, *J =* 6.4 Hz, 2H), 3.84 (t, *J = 6.4* Hz, 2H), 3.25-3.21 (m, 2H), 2.73 (t, *J =* 6.6 Hz, 2H), 1.96-1.91 (m, 2H).

### Example 1

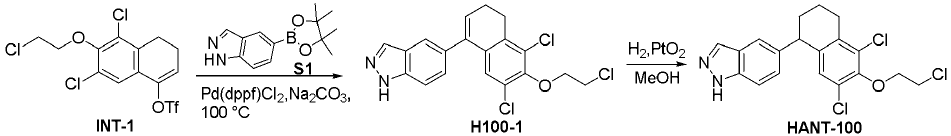

### Step 1: 5-(5,7-dichloro-6-(2-chloroethoxy)-3,4-dihydronaphthalen-1-yl)-1H-indazole

Compound **INT-1** (500 mg, 1.18 mmol), compound **S1** (430 mg, 1.76 mmol), Pd(dppf)Cl2 (86 mg, 0.18 mmol) and sodium carbonate (312 mg, 2.95 mmol) were sequentially dissolved in dioxane/water (10/2 ml) in a dry flask under N₂ protection, then stirred at 100 °C for 6 h. The crude reaction was extracted with EA, washed with saturated aq. NH₄Cl. The organic phase was dried with Na₂SO₄, filtered, concentrated, and purified by flash chromatography (DCM/MeOH=25:1) to obtain **H100-1** (250 mg, yield: 63%) as a white solid. LCMS (ESI): *m*/*z* =393.1 [M+H]⁺.

### Step 2: 5-(5,7-dichloro-6-(2-chloroethoxy)-1,2,3,4-tetrahydronaphthalen-1-yl)-1H-indazole

Compound **H100-1** (250 mg, 0.6 mmol), PtO₂ (50 mg, 20 wt%), MeOH (10 mL) were added sequentially into a dry flask and reacted under H₂ at room temperature for 5 h. The crude reaction mixture was filtered, concentrated and purified by prep-HPLC to obtain **HANT-100** (40 mg, Yield: 16%). LCMS (ESI): m/z= 395.2[M+H]⁺. ¹H NMR (400 MHz, CDCl₃): δ 8.02 (s, 1H), 7.44 (d, *J =* 8.4 Hz, 1H), 7.39 (s, 1H), 7.12 (d, *J =* 8.4 Hz, 1H), 6.82 (s, 1H), 4.26 (t, *J* = 6.4 Hz, 2H), 4.15 (t, *J =* 6.8 Hz, 1H), 3.88 (t, *J =* 6.4 Hz, 2H), 2.88-2.84 (m, 2H), 2.16-2.11 (m, 1H), 1.96-1.70 (m, 3H).

### Example 2

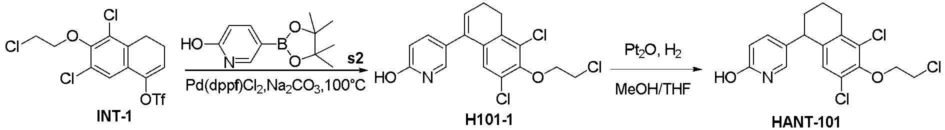

Synthesis was performed using methods described in Example 1 to afford the compound HANT-101 (4.4 mg, white solid, yield: 14.6%). LCMS (ESI): *m*/*z* =372.0 [M+H]⁺; ¹H NMR (400 MHz, CD₃OD) δ 7.40-7.35 (m, 1H), 7.10 (d, *J =* 2.6 Hz, 1H), 6.93(s, 1H), 6.53 (d, *J = 9.4* Hz, 1H), 4.23 (t, *J =* 5.8 Hz, 2H), 3.96 (s, 1H), 3.88 (t, *J =* 5.8 Hz, 2H), 2.83 (s, 2H), 2.03 (s, 1H), 1.80 (s, 3H).

### Example 3

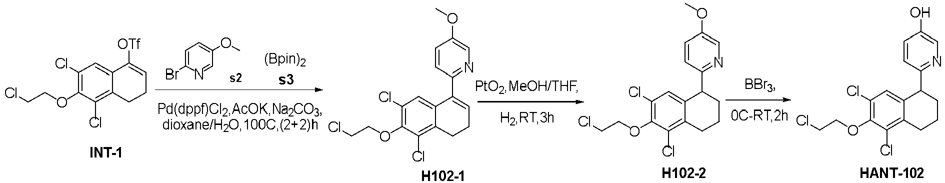

### Step 1: 2-(5,7-dichloro-6-(2-chloroethoxy)-3,4-dihydronaphthalen-1-yl)-5-methoxypyridine

Compounds **S2** (1.0 g, 5.3 mmol), **S3** (1.76 g, 6.9 mmol), potassium acetate (1.04 g, 10.6 mmol) and Pd(dppf)Cl2 (0.39 g, 0.53 mmol) were sequentially dissolved in dioxane (30 mL) in a dry flask under N₂, and stirred at 100 °C for 2 h. **INT-1** (300 mg, 5.3 mmol), sodium carbonate (150 mg, 10.6 mmol) and water (2 mL) were added to the reaction solution and stirred under N₂ at 100 °C for 2 h. Then the reaction was cooled to RT, extracted with saturated aq. NH₄Cl and EA, and the organic phase was washed with saturated brine, dried with sodium sulfate and filtered and concentrated under reduced pressure. The residue was purified by flash chromatography to obtain grey oily compound **H102-1** (90 mg, yield: 33.2%). LCMS (ESI): m/z =384[M+H]⁺.

### Step 2: 2-(5,7-Dichloro-6-(2-chloroethoxy)-1,2,3,4-tetrahydronaphthalen-1-yl)-5-methoxypyridine

Compound **H102-1** (90 mg, 0.23 mmol) was dissolved in MeOH/THF (6 mL/3 mL), followed by PtO₂ (26.7 mg, 0.12 mmol), and the reaction was stirred under H₂ at room temperature for 3 h. The reaction solution was filtered and concentrated under reduced pressure, and then purified by flash chromatography to obtain the yellow oil compound **H102-2** (86 mg, yield: 95.0%). LCMS (ESI): *m*/*z* =386[M+H]⁺.

### Step 3: 6-(5,7-dichloro-6-(2-chloroethoxy)-1,2,3,4-tetrahydronaphthalen-1-yl)pyridin-3-ol

Compound **H102-2** (30 mg, 0.078 mmol) was added to BBr₃ (0.9 mL, 0.78 mmol) at 0 °C in a sealed flask, then the reaction was allowed to warm up to room temperature and kept for 2 h. The reaction solution was extracted with DCM, and the organic phase was washed with saturated brine, dried over Na₂SO₄ and concentrated under reduced pressure. The residue was purified by neutral reversed-phase prep-HPLC to obtain white solid **HANT-102** (5.9 mg, yield: 20.4%). LCMS (ESI): *m*/*z* =372[M+H]⁺; ¹H NMR (400 MHz, CDCl₃) δ 8.18 (d, *J =* 2.0 Hz, 1H), 7.15 (dd, *J =* 8.4, 2.5 Hz, 1H), 6.89 (d, *J =* 8.5 Hz, 1H), 6.77 (s, 1H), 4.21 (t, *J =* 6.3 Hz, 3H), 3.85 (t, *J =* 6.4 Hz, 2H), 2.83 (t, *J =* 6.3 Hz, 2H), 2.12-2.06 (m, 1H), 2.00-1.96 (m, 1H), 1.89 (dd, *J=* 7.9, 5.8 Hz, 1H), 1.80-1.76 (m, 1H).

### Example 4

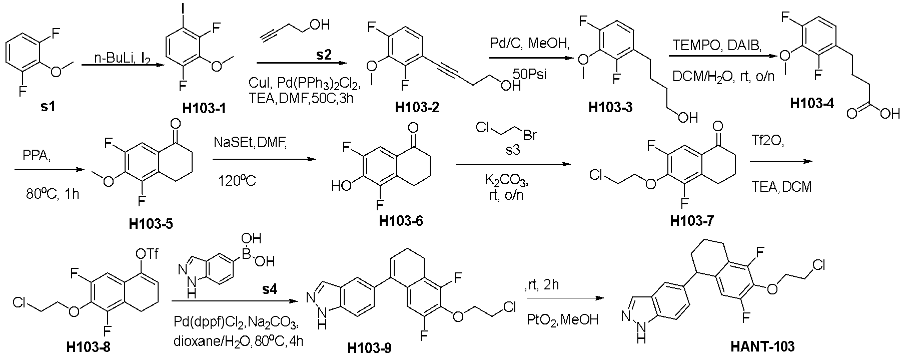

### Step 1: Synthesis of 1,3-difluoro-4-iodo-2-methoxybenzene

Compound **S1** (5.0 g, 34.7 mmol) was dissolved in THF (100 mL) in a three-necked flask. n-BuLi (15.5 mL) was added at -78 °C and stirred for 1 h. then I₂ (9.3 g, 36.6 mmol) was added in and stirred at -78 °C for 1 h. The reaction solution was extracted with DCM; the organic phase was dried and concentrated, and the residue was purified by flash chromatography to obtain **H103-1** (8.4 g, yield: 89%) as a solid. LCMS (ESI): *m*/*z* =270.9[M+H]⁺.

### Step 2: Synthesis of 4-(2,4-difluoro-3-methoxyphenyl)but-3-yn-1-ol

Compounds **H103-1** (8.4 g, 31.1 mmol), **S2** (4.4 g, 62 mmol), Pd(PPh₃)₂Cl₂ (112 mg, 0.15 mmol), CuI (1.2 g, 6.2 mmol), and TEA (31.5 g, 311 mmol) were dissolved in DMF (100 mL) and stirred for 3 h at 50 °C. The reaction solution was extracted with EA; the organic phase was dried and concentrated, and the residue was purified by flash chromatography to obtain **H103-2** as solid (4.1 mg, yield: 62%). LCMS (ESI): m/z =213.1[M+H]⁺.

### Step 3: Synthesis of 4-(2,4-difluoro-3-methoxyphenyl)butanol

Compound **H103-2** (4.5 g, 21.2 mmol) and Pd/C (900 mg) dissolved in methanol (10 mL) and stirred at room temperature overnight. The reaction solution was filtered and concentrated to obtain compound **H103-3** (4.1 g, yield: 85%). LCMS (ESI): *m*/*z* =217.1[M+H]⁺.

### Step 4: Synthesis of 4-(2,4-difluoro-3-methoxyphenyl)butanoic acid

Compound **H103-3** (2.0 g, 8.7 mmol), TEMPO (400 mg, 2.6 mmol), DAIB (7.0 g, 21.7 mmol) were dissolved in DCM/water (20 mL/4 mL) and stirred at room temperature overnight.The reaction solution was concentrated and the residue was purified by flash chromatography to obtain solid **H103-4** (1.4 g, yield: 75%). LCMS (ESI): *m*/*z* =229.1[M+H]+.

### Step 5: Synthesis of 5,7-difluoro-6-methoxy-3,4-dihydronaphthalen-1(2H)-one

Compound **H103-4** (1.4 g, 6.1 mmol) was added to PPA (20 mL) and stirred at 80 °C for 1 h. The reaction solution was extracted with EA, the organic phase was dried and concentrated, and the residue was purified by flash chromatography to obtain **H103-5** as solid (900 mg, yield: 69.7%). LCMS (ESI): *m*/*z*=213.1[M+H]⁺.

### Step 6: Synthesis of 5,7-difluoro-6-hydroxy-3,4-dihydronaphthalen-1(2H)-one

Compound **H103-5** (850 mg, 4.0 mmol) and NaSEt (1.2 g, 20.0 mmol) were dissolved in DMF (20 mL) and stirred at 120 °C for 3 h. The reaction solution was extracted with EA, and the organic phase was dried and concentrated. The residue was purified by flash chromatography to afford **H103-6** as a solid (476 mg, Yield: 60%). LCMS (ESI): *m*/*z* = 199.0 [M+H]⁺.

### Step 7: Synthesis of 6-(2-chloroethoxy)-5,7-difluoro-3,4-dihydronaphthalen-1(2H)-one

Compound **H103-6** (476 mg, 2.4 mmol), **S3** (1.03 mg, 7.2 mmol), potassium carbonate (1.0 mg, 7.2 mmol) were dissolved in DMF (10 mL) and stirred at room temperature overnight. The reaction solution was extracted with EA; the organic phase was dried and concentrated, and the residue was purified by flash chromatography to obtain **H103-7** as solid (300 mg, yield: 49%). LCMS (ESI): *m*/*z*=261.0[M+H]⁺.

### Step 8: Synthesis of 6-(2-chloroethoxy)-5,7-difluoro-3,4-dihydronaphthalen-1-yl trifluoromethanesulfonic acid

Compound **H103-7** (300 mg, 1.15 mmol), TEA (350 mg, 3.5 mmol), were dissolved in DCM (10 mL) in a three-neck flask, followed by addition of Tf₂O (1.63 g, 5.8 mmol) at 0 °C, and the reaction was stirred at room temperature overnight. The reaction solution was extracted with DCM; the organic phase was dried and concentrated, and the residue was purified by flash chromatography to obtain **H103-8** as solid (350 mg, yield: 77%). LCMS (ESI): *m*/*z* =393.0[M+H]⁺.

### Step 9: Synthesis of 5-(6-(2-chloroethoxy)-5,7-difluoro-3,4-dihydronaphthalen-1-yl)-1Hindazole

Compound **H103-8** (300 mg, 0.77 mmol), **S4** (186 mg, 1.15 mmol), Pd(dppf)Cl2 (112 mg, 0.15 mmol), Na₂CO₃ (163 mg, 1.53 mmol) was dissolved in dioxane/water (5 mL/1 mL) and stirred at 80 °C for 4 h. The reaction solution was extracted with EA; the organic phase was dried and concentrated, and the residue was purified by flash chromatography to obtain **H103-9** as solid (150 mg, yield: 54%).LCMS (ESI): *m*/*z* =361.1[M+H]+.

### Step 10: Synthesis of 5-(6-(2-chloroethoxy)-5,7-difluoro-1,2,3,4-tetrahydronaphthalen-1-yl)-1H indazole

Compound **H103-9** (150 mg, 0.42 mmol), PₜO₂ (50 mg) were dissolved in to MeOH/THF (3 mL/1 mL) and reacted under H₂ for 2 h. The reaction solution was filtrated, concentrated under reduced pressure, and the residue was purified by prep-HPLC to obtain **HANT-103** (61.3 mg, yield: 61%) as a white solid. LCMS (ESI): *m*/*z* = 363.1 [M+H]⁺; ¹H NMR (300 MHz, DMSO-d6) δ 12.89 (s, 1H), 8.01 (s, 1H), 7.56 - 7.40 (m, 2H), 7.13 (d, *J=* 9.3 Hz, 1H), 6.45 (d, *J=* 11.5 Hz, 1H), 4.36 (t, *J =* 5.2Hz, 2H), 4.20 (t, *J =* 6.5 Hz, 1H), 3.90 (t, *J =* 5.2 Hz, 2H), 2.84 - 2.73 (m, 2H), 2.17 - 2.04 (m, 1H), 1.98 - 1.82 (m, 2H), 1.83 - 1.66 (m, 1H).

### Example 5

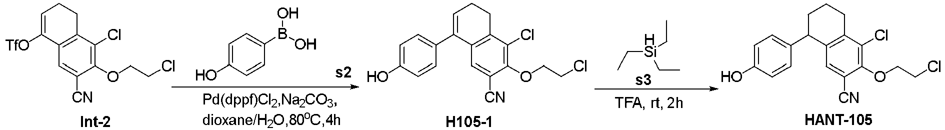

### Step 1: Synthesis of 4-chloro-3-(2-chloroethoxy)-8-(4-hydroxyphenyl)-5,6-dihydronaphthalene-2-carbonitrile

Compound **INT-2** (170 mg, 0.41 mmol), **S2** (85 mg, 0.61 mmol), Pd(dppf)Cl2 (60 mg, 0.08 mmol), Na₂CO₃ (87 mg, 0.82 mmol) was dissolved in dioxane/water (3 mL/0.5 mL) and stirred at 80 °C for 4 h. The reaction was cooled to room temperature and extracted with EA. The organic phase was dried and concentrated, and the residue was purified by flash chromatography to obtain the yellow oily compound **H105-1** (130 mg, yield: 88%). LCMS (ESI): *m*/*z* =360.0[M+H]⁺.

### Step 2: Synthesis of 4-chloro-3-(2-chloroethoxy)-8-(4-hydroxyphenyl)-5,6,7,8-tetrahydronaphthalene-2-carbonitrile

Compound **H105-1** (100 mg, 0.28 mmol) and **S3** (65 mg, 0.56 mmol) was dissolved in TFA (5 mL) and reacted under N₂ at room temperature for 2 h. The reaction solution was adjusted to pH=8 with aqueous sodium bicarbonate, then extracted with EA. The organic phase was concentrated under reduced pressure, and the residue was purified by flash chromatography to obtain a white solid **HANT-105** (58.7 mg, yield: 58%). LCMS (ESI): *m*/*z* =362.1[M+H]⁺; ¹H NMR (400 MHz, DMSO-d₆) δ 9.30 (s, 1H), 7.11 (s, 1H), 6.86 (d, *J =* 8.2 Hz, 2H), 6.70 (d, *J =* 8.4 Hz, 2H), 4.39 (t, *J* = 5.1 Hz)., 2H), 4.05 (t, *J* = 5.8 Hz, 1H), 3.97 (t, *J* = 5.2 Hz, 2H), 2.89-2.80 (m, 2H), 2.03-1.89 (m, 1H), 1.84-1.66 (m, 3H).

### Example 6

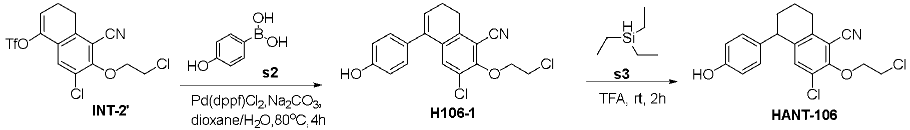

### Step 1: Synthesis of 3-chloro-2-(2-chloroethoxy)-5-(4-hydroxyphenyl)-7,8-dihydronaphthalene-1-carbonitrile

Compound **INT-2'** (100 mg, 0.24 mmol, synthesised with similar procedures as compound **INT-2), S2** (50 mg, 0.36 mmol), Pd(dppf)Cl2 (36 mg, 0.05 mmol), and Na₂CO₃ (52 mg, 0.5 mmol) were dissolved in dioxane/water (3 mL/0.5 mL) mixture and stirred at 80 °C for 4 h. The reaction was cooled to room temperature, then extracted with EA. The organic phase was dried and concentrated, and the residue was purified by flash chromatography to obtain the yellow oily compound **H106-1** (80 mg, yield: 93%). LCMS (ESI): *m*/*z* =360.0[M+H]⁺ .

### Step 2: Synthesis of 3-chloro-2-(2-chloroethoxy)-5-(4-hydroxyphenyl)-5,6,7,8-tetrahydronaphthalene-1-carbonitrile

Compounds **H106-1** (80 mg, 0.23 mmol) and S3 (53 mg, 0.45 mmol) were dissolved in TFA (3 mL) and stirred under N₂ at room temperature for 2 h. The reaction solution was adjusted to pH=8 with aqueous sodium bicarbonate, then extracted with EA. The organic phase was concentrated under reduced pressure, and the residue was purified by prep-HPLC to obtain a white solid **HANT-106** (49.8 mg, yield: 82%). LCMS (ESI): *m*/*z* =362.1[M+H]⁺; ¹H NMR (400 MHz, DMSO-d₆) δ 9.30 (s, 1H), 7.10 (s, 1H), 6.88 (d, *J =* 8.5 Hz, 2H), 6.70 (d, *J* = 8.4 Hz, 2H), 4.43-4.35 (m, 2H), 4.06-3.98 (m, 1H), 3.99-3.91 (m, 2H), 2.98-2.82 (m, 2H), 2.04-1.93 (m, 1H), 1.87-1.62 (m, 3H).

### Example 7

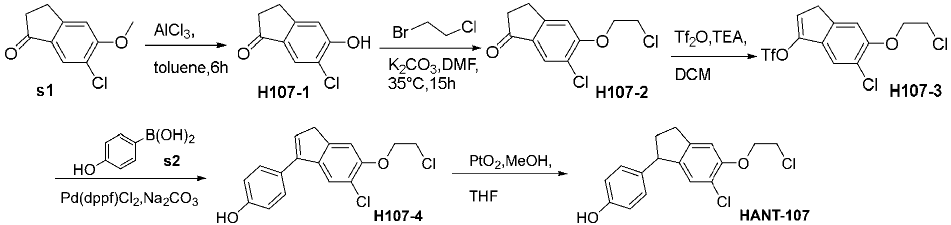

### Step 1: Synthesis of 6-chloro-5-hydroxy-2,3-dihydro-1H-inden-1-one

Compound **S1** (800 mg, 4.08 mmol) was dissolved in toluene (10 mL), and then AlCl₃ (1.63 g, 12.2 mmol) was added in. The reaction was kept at 75 °C under N₂ for 6 h, then cooled to room temperature and extracted with aq. NH₄Cl and EA, and the organic phase was dried with anhydrous Na₂SO₄, filtered, and concentrated under reduced pressure. The residue was purified by flash chromatography to obtain compound **H107-1** (530 mg, yield: 70.9%). LCMS (ESI): *m*/*z* =183.0 [M+H]⁺.

### Step 2: Synthesis of 6-chloro-5-(2-chloroethoxy)-2,3-dihydro-1H-inden-1-one

Compound **H107-1** (500 mg, 2.7 mmol) was dissolved in DMF (15 mL), followed by addition of 1-bromo-2-chloroethane (985 mg, 6.89 mmol) and potassium carbonate (454 mg, 3.29 mmol). The reaction was kept at 35 °C for 15 h. The compound was extracted with aq. NH₄Cl and EA, and the organic phase was dried with anhydrous Na₂SO₄, filtered, and then concentrated under reduced pressure to obtain the compound **H107-2** (490 mg, yellow solid, yield: 72.8%). LCMS (ESI): *m*/*z* =245.0 [M+H]⁺.

### Step 3: Synthesis of 5-chloro-6-(2-chloroethoxy)-1H-inden-3-yl trifluoromethanesulfonate

Compound **H107-2** (400 mg, 1.63 mmol) and Et₃N (480 mg, 4.75 mmol) were dissolved in DCM (10 mL), then Tf₂O (1.2 mL) was added at 0 °C. The reaction was kept at room temperature under N₂ for 3 h. The reaction solution was then extracted with aq. NH₄Cl and DCM, and the organic phase was dried with anhydrous Na₂SO₄, filtered, and then concentrated under reduced pressure. The residue was purified by flash chromatography to obtain compound **H107-3** (390 mg, yellow oil, yield: 63.4%). LCMS (ESI): *m*/*z* =376.9 [M+H]⁺.

### Step 4: Synthesis of 4-(5-chloro-6-(2-chloroethoxy)-1H-inden-3-yl)phenol

Compound **H107-3** (300 mg, 0.80 mmol) and compound **S2** (165 mg, 1.20 mmol) were dissolved in a mixed solution of dioxane and water (5.5 mL, 10:1), then Pd(dppf)Cl2 (88 mg, 012 mmol) and sodium carbonate (168 mg, 1.56 mmol) were added and the reaction was heated to 105 °C for 6 h. The reaction solution was cooled to room temperature and extracted with aq. NH₄Cl and EA. The organic phase was dried with anhydrous Na₂SO₄ and filtered. The residue was concentrated under reduced pressure and purified by flash chromatography to obtain compound **H107-4** (66 mg, yellow solid, yield: 25.7%). LCMS (ESI): *m*/*z* =321.0 [M+H]⁺.

### Step 5: Synthesis of 4-(6-chloro-5-(2-chloroethoxy)-2,3-dihydro-1H-inden-1-yl)phenol

Compound **H107-4** (66 mg, 0.20 mmol) and PtO₂ (13 mg, 0.2 eq) were dissolved in a mixed solution of methanol and THF (1.5 mL, 2:1) and hydrogenated with H₂ at room temperature for 3 h. The reaction was extracted with aq. NH₄Cl and DCM, and the organic phase was dried with anhydrous Na₂SO₄, filtered, and concentrated under reduced pressure. The residue was purified by prep-HPLC to obtain compound **HANT-107** (4.1 mg, white solid, yield: 6.6%). LCMS (ESI): *m*/*z* = 323.0 [M+H]⁺; ¹H NMR (400 MHz, CD₃OD) δ 7.00 (s, 1H), 6.97 (d, *J= 8.4* Hz, 2H), 6.80 (s, 1H), 6.73 (d, *J =* 8.4 Hz, 2H), 4.28 (t, *J =* 5.6 Hz, 2H), 4.18 (t, *J =* 8.2 Hz, 1H), 3.87 (t, *J =* 5.6 Hz, 2H), 2.96 (d, *J =* 5.4 Hz, 1H), 2.88 (dt, *J =* 16.2, 8.4 Hz, 1H), 2.53 (d, *J* =8.4 Hz, 1H), 1.99 (dd, *J=* 12.6, 9.0 Hz, 1H).

### Example 8

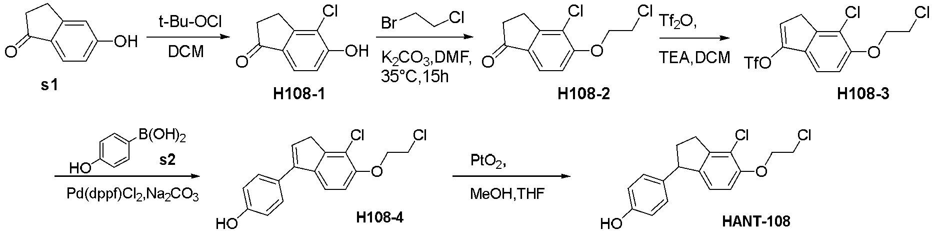

### Step 1: Synthesis of 4-chloro-5-hydroxy-2,3-dihydro-1H-inden-1-one

Compound S1 (2.0 g, 13.5 mmol) was dissolved in chloroform (100 mL), and tert-butyl hypochlorite (2.2 g, 20.2 mmol) was added at 0 °C. The reaction was kept at room temperature overnight. The reaction solution was extracted with aq. NH₄Cl and DCM, and the organic phase was dried with anhydrous Na₂SO₄, filtered and concentrated under reduced pressure. The residue was purified by flash chromatography to obtain compound **H108-1** (1.75 g, yellow solid, yield: 71%). LCMS (ESI): *m*/*z* =183.0 [M+H]⁺.

### Step 2: Synthesis of 4-chloro-5-(2-chloroethoxy)-2,3-dihydro-1H-inden-1-one

Compound **H108-1** (1.5 g, 8.2 mmol) was dissolved in 25 mL of DMF, then 1-bromo-2-chloroethane (2.95 g, 20.6 mmol) and potassium carbonate (1.37 g, 9.9 mmol) were added in, and the reaction was kept at 35 °C overnight. The reaction mixture was extracted with aq. NH₄Cl and EA, and the organic phase was dried with anhydrous Na₂SO₄, filtered and concentrated under reduced pressure to obtain compound **H108-2** (1.37 g, white solid, yield: 68.5%). LCMS (ESI): *m*/*z* =245.0 [M+H]⁺.

### Step 3: Synthesis of 7-chloro-6-(2-chloroethoxy)-1H-inden-3-yl trifluoromethanesulfonate

Compound **H108-2** (1.0 g, 4.08 mmol) and Et₃N (1.2 g, 11.8 mmol) were dissolved in DCM (15 mL), and Tf₂O (3 mL) was added at 0 °C. The reaction was kept under nitrogen for 3 h. The reaction solution was extracted with aq. NH₄Cl and DCM, and the organic phase was dried with anhydrous Na₂SO₄, filtered and concentrated under reduced pressure. The residue was purified by flash chromatography to obtain compound **H108-3** (1.1 g, yellow oil, yield: 71.8%). LCMS (ESI): *m*/*z* =376.9 [M+H]⁺.

### Step 4: Synthesis of 4-(7-chloro-6-(2-chloroethoxy)-1H-inden-3-yl)phenol

Compound **H108-3** (500 mg, 1.33 mmol) and compound **S2** (275 mg, 1.99 mmol) were dissolved in a mixed solution of dioxane and water (11 mL, 10 :1), then Pd(dppf)Cl2 (195 mg, 027 mmol) and sodium carbonate (280 mg, 2.64 mmol) were added in. The reaction was kept at 105 °C for 6 h under N₂ protection. The reaction solution was cooled to room temperature and extracted with aq. NH₄Cl and EA, the organic phase was dried with anhydrous Na₂SO₄, filtered and concentrated under reduced pressure. The residue was purified by flash chromatography to obtain compound **H108-4** (173 mg, yellow solid, yield: 40.5%). LCMS (ESI): m/z =321.0 [M+H]⁺.

### Step 5: Synthesis of 4-(4-chloro-5-(2-chloroethoxy)-2,3-dihydro-1H-inden-1-yl)phenol

Compound **H108-4** (173 mg, 0.54 mmol) and PtO₂ (34 mg, 0.2 eq) were dissolved in a mixed solution of methanol and THF (3 mL, 2 :1), and the reaction was hydrogenated with H₂ at room temperature for 3 h. The reaction was extracted with aq. NH₄Cl and DCM, and the organic phase was dried with anhydrous Na₂SO₄, filtered, and then concentrated under reduced pressure. The residue was purified by prep-HPLC to obtain compound **HANT-108** (9.3 mg, white solid, yield: 5.3%). LCMS (ESI): *m*/*z* = 323.1 [M+H]⁺; ¹H NMR (400 MHz, CDCl₃) δ 6.97 (d, *J =* 8.4 Hz, 2H), 6.84 (d, *J* = 8.2 Hz, 1H), 6.72 (t, *J =* 8.2 Hz, 1H), 6.75 (t, *J =* 8.2 Hz, 1H).6.72 (t, *J =* 7.8 Hz, 3H), 4.31-4.20 (m, 3H), 3.86 (t, *J=* 5.4 Hz, 2H), 3.17-3.02 (m, 1H), 2.91 (dd, *J =* 16.4, 8.2 Hz, 1H), 2.62-2.48 (m, 1H), 2.00 (dd, *J =* 12.6, 9.0Hz, 1H).

### Example 9

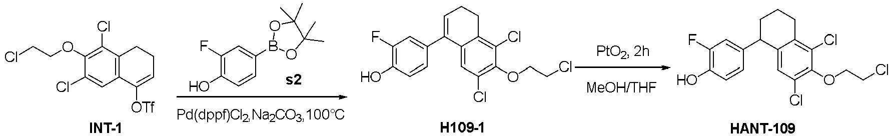

Synthesis was performed using methods described in **Example 1** to afford compound **HANT-109** (10.3 mg, white solid, yield: 25.6%). LCMS (ESI): *m*/*z* =389.0 [M+H]⁺; ¹H NMR (400 MHz, CD₃OD) δ 6.84 (dd, *J =* 14.4, 5.6 Hz, 2H), 6.76-6.63 (m2H), 4.23 (t, *J =* 5.8 Hz, 2H), 4.00 (t, *J =* 6.4 Hz, 1H), 3.88 (t, *J =* 5.8 Hz, 2H), 2.83 (t, *J =* 5.6 Hz, 2H), 2.06-2.02 (m, 1H), 1.95-1.69 (m, 3H).

### Example 10

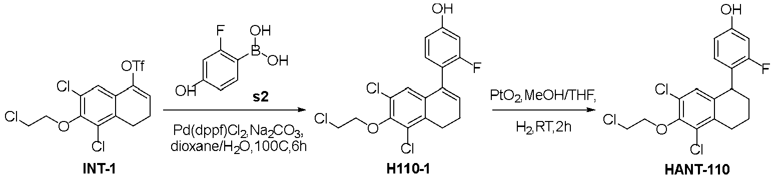

### Step 1: Synthesis of 4-(5,7-dichloro-6-(2-chloroethoxy)-3,4-dihydronaphthalen-1-yl)-3-fluorophenol

Compounds **INT-1** (50 mg, 0.12 mmol) and **S2** (28 mg, 0.18 mmol) were dissolved in a mixture of dioxane/water (1 mL /0.2 mL) in a sealed tube and sodium carbonate (25 mg, 0.24 mmol) and Pd(dppf)Cl2 (17.2 mg, 0.024 mmol) were added. The reaction was stirred at 100 °C for 6 h under N₂ protection. The reaction mixture was cooled, extracted with aq. NH₄Cl and EA, and the organic phase was washed with saturated brine, dried over Na₂SO₄ and concentrated by filtration under reduced pressure. The residue was purified by flash chromatography to obtain a colorless solid **H110-1** (33 mg, yield: 2.7%). LCMS (ESI): *m*/*z* =387 [M-H]⁺.

### Step 2: Synthesis of 4-(5,7-dichloro-6-(2-chloroethoxy)-1,2,3,4-tetrahydronaphthalen-1-yl)-3-fluorophenol

Compound **H110-1** (33 mg, 0.085 mmol) and PtO₂ (10 mg, 0.0425 mmol) were dissolved in MeOH (2 mL)/THF (1 mL), and the reaction was hydrogenated with H₂ at room temperature for 2 h. The reaction mixture was filtered and concentrated under reduced pressure, and purified by neutral prep-HPLC to obtain the grey solid **HANT-110** (11.8 mg, yield: 35.5%). LCMS (ESI): *m*/*z* =389 [M+H]⁺; ¹H NMR (400 MHz, CDCl₃) δ 6.82 (s, 1H), 6.67 (t, *J =* 8.5 Hz, 1H), 6.59 (dd, *J =* 11.3, 2.3 Hz, 1H), 6.52 (dd, *J =* 8.4, 2.3 Hz, 1H), 4.94 (s, 1H), 4.26 (dd, *J* = 14.0, 7.5 Hz, 3H), 3.87 (t, *J =* 6.4 Hz, 2H), 2.81 (t, *J =* 6.3 Hz, 2H), 2.03-1.99 (m, 1H), 1.92-1.75 (m, 3H).

### Example 11

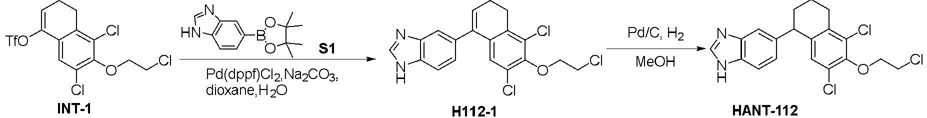

Synthesis was performed using methods described in **Example 1,** with the exception that Pd-C was used instead of PtO; in the hydrogenation reaction to obtain **HANT-112** (6 mg, white solid, yield: 19.9 %). LCMS (ESI): *m*/*z* = 395.0 [M+H]⁺. ¹H NMR (400 MHz, CDCl₃) δ 8.06 (s, 1H), 7.60 (d, *J =*7.6 Hz, 1H), 7.25 (s, 1H), 7.04 (d, *J =* 8.4 Hz, 1H), 6.83 (s,1H), 4.26 (t, *J =* 6.4 Hz, 2H), 4.17 (t, *J =* 6.4 Hz, 1H), 3.88 (t, *J =* 6.4 Hz, 2H), 2.84 (t, *J=* 5.6 Hz, 2H),1.91-1.79 (m, 1H), 1.79-1.77 (m, 2H), 1.25 (s, 1H).

### Example 12

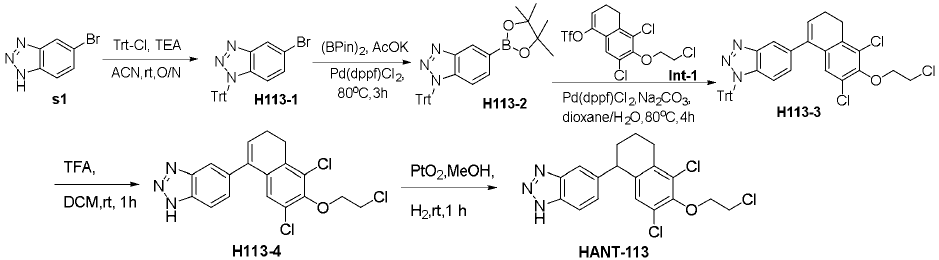

### Step 1: Synthesis of 5-bromo-1-triphenyl-1H-benzo[d][1,2,3]triazole

Compound **S1** (1.0 g, 5.05 mmol), Trt-Cl (2.1 g, 7.6 mmol), Et₃N (1.53 g, 15.2 mmol) were dissolved in ACN (20 mL) and stirred at room temperature overnight. The reaction solution was extracted with EA; the organic phase was dried and concentrated, and the residue was purified by flash chromatography to obtain **H113-1** as solid (2.0 g, yield: 90%). LCMS (ESI): *m*/*z* =440.1[M+H]⁺.

### Step 2: Synthesis of 5-(4,4,5,5-tetramethyl-1,3,2-dioxobenzofuran-2-yl)-1-trimethyl-1H benzo[d][1,2,3]triazole

Compound **H113-1** (2.0 g, 4.6 mmol), (Bpin)₂ (1.74 g, 6.8 mmol), Pd(dppf)Cl2 (333 mg, 0.46 mmol), and AcOK (1.34 g, 13.7 mmol) were dissolved in dioxane (30 mL) and stirred at 80 °C for 3 h. The reaction was cooled and the reaction solution was extracted with EA. The organic phase was dried and concentrated, and the residue was purified by flash chromatography to obtain **H113-2** as solid (800 mg, yield: 36%). LCMS (ESI): *m*/*z* =488.2[M+H]₊.

### Step 3: Synthesis of 5-(5,7-dichloro-6-(2-chloroethoxy)-3,4-dihydronaphthalen-1-yl)-1-triphenyl-1H benzo[d][1,2,3 ]triazole

Compounds **H113-2** (100 mg, 0.24 mmol), **INT-1** (176 mg, 0.36 mmol), Pd(dppf)Cl2 (35 mg, 0.05 mmol), and Na₂CO₃ (51 mg, 0.48 mmol) were dissolved in dioxane/water (3mL/0.5mL) and stirred at 80 °C under N₂ protection. The reaction was cooled and the reaction solution was extracted with EA. The organic phase was dried and concentrated, and the residue was purified by flash chromatography to obtain **H113-3** as solid (150 mg crude, yield: 98%). LCMS (ESI): *m*/*z* =636.1[M+H]⁺.

### Step 4: Synthesis of 5-(5,7-dichloro-6-(2-chloroethoxy)-3,4-dihydronaphthalen-1-yl)-1H-benzo[d][1,2,3]triazole

Compound **H113-3** (150 mg, 0.24 mmol) and TFA (2 mL) was dissolved in DCM (2 mL) and the reaction was kept at room temperature for 1 h. The reaction solution was directly subjected to prep-HPLC purification to obtain **H113-4** (35 mg, yield: 38%) as a white solid. LCMS (ESI): *m*/*z* = 394.0 [M+H]⁺.

### Step 5: Synthesis of 5-(5,7-dichloro-6-(2-chloroethoxy)-1,2,3,4-tetrahydronaphthalen-1-yl)-1H-benzo[d][1,2,3]triaz ole

Compound **H113-4** (35 mg, 0.09 mmol), PtO₂ (15 mg) was dissolved in MeOH/THF (3 mL/1 mL) and stirred at room temperature for 1 h. The reaction mixture was filtered, and the organic phase was dried and concentrated. The residue was purified by prep-HPLC to obtain **HANT-113** as a solid (5.4 mg, yield: 17%). LCMS (ESI): *m*/*z* = 396.0 [M+H]⁺; ¹H NMR (400 MHz, CD₃OD) δ 7.82 (d, *J =* 8.7 Hz, 1H), 7.50 (s, 1H), 7.24 (d, *J =* 8.6 Hz, 1H), 6.81 (d, *J =* 0.8 Hz, 1H), 4.33 (t, *J =* 6.7 Hz, 1H), 4.25 (t, *J =* 5.8 Hz, 2H), 3.89 (t, *J =* 5.8 Hz, 2H), 2.89 (t, *J* = 6.4 Hz, 2H), 2.23-2.11 (m, 1H), 1.98-1.89 (m, 2H), 1.85-1.79 (m, 1H).

### Example 13

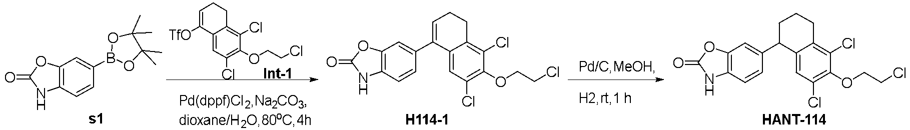

Synthesis was performed using methods described in **Example 1,** with the exception that Pd-C was used instead of PtO₂ in the hydrogenation reaction to **obtain HANT-114** (36.7 mg, yield: 28%) as a white solid. LCMS (ESI): *m*/*z* = 412.0 [M+H]⁺; ¹H NMR (400 MHz, DMSO-d₆) δ 11.58 (s, 1H), 7.05 (d, *J=* 1.6 Hz, 1H), 7.02 (d, *J =* 8.0 Hz, 1H), 6.85 (dd, *J =* 8.1, 1.6 Hz, 1H), 6.81 (d, *J =* 0.9 Hz, 1H), 4.21 (dd, *J =* 5.8, 4.5 Hz, 2H), 4.15 (t, *J =* 6.5 Hz, 1H), 3.99-3.92 (m2H), 2.79 (t, *J =* 6.4 Hz, 2H), 2.04-1.94 (m, 1H), 1.86-1.75 (m, 2H), 1.76-1.67 (m, 1H).

### Example 14

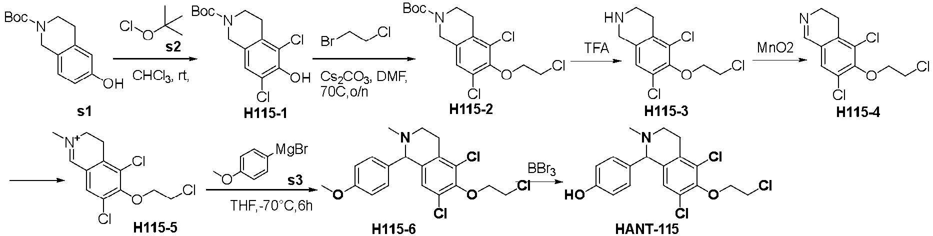

### Step 1: Synthesis of tert-butyl 5,7-dichloro-6-hydroxy-3,4-dihydroisoquinoline-2(1H)-carboxylate

Compound S1 (3 g, 12 mmol) was dissolved in trichloromethane (300 mL) the **S2** (3.3 g, 30 mmol) was added slowly to the mixture in an ice bath. The reaction was stirred at room temperature overnight, then concentrated under reduced pressure to obtain crude yellow solid **H115-1** (2 g, yield: 52.6%). LCMS (ESI): m/z =318.1 [M+H]⁺.

### Step 2: Synthesis of tert-butyl 5,7-dichloro-6-(2-chloroethoxy)-3,4-dihydroisoquinoline-2(1H)-carboxylate

Compound **H115-1** (2.1 g, 6.6 mmol) and 1-bromo-2-chloroethane (2.37 g, 16.5 mmol) were dissolved in DMF (30 mL), then cesium carbonate (4.3 g, 13.2 mmol) was added and reacted at 70 °C for 16 h. The compound was extracted with aq. NH₄Cl and EA and dried with anhydrous Na₂SO₄. The organic phase was concentrated under reduced pressure, and the residue was prepared by flash chromatography (PE/EA) (=20:1) to obtain **H115-2** (2 g, yield: 80%) as a white solid. LCMS (ESI): *m*/*z* =380.1[M+H]⁺.

### Step 3: Synthesis of 5,7-dichloro-6-(2-chloroethoxy)-1,2,3,4-tetrahydroisoquinoline

Compound **H115-2** (2 g, 5.2 mmol) was dissolved in a single-neck flask with DCM (20 mL) and TFA (15 mL), and stirred at room temperature overnight. The reaction was adjusted to pH~9 with sodium bicarbonate in an ice bath, extracted with DCM, and the organic phase was concentrated under reduced pressure to obtain **H115-3** (1.45 g, yield: 98.6%) as a white solid. LCMS (ESI): *m*/*z* =280.0 [M+H]⁺.

### Step 4: Synthesis of 5,7-dichloro-6-(2-chloroethoxy)-3,4-dihydroisoquinoline

Compound **H115-3** (1.17 g, 4.1 mmol) was dissolved in DCM (35 mL) followed by addition of MnO₂ (4.36 g, 50 mmol), and the mixture was stirred at room temperature overnight. The mixture was filtered and concentrated to obtain **H115-4** (1.0 g, yield: 86.2%) as a yellow solid. LCMS (ESI): *m*/*z* = 278.0 [M+H]⁺.

### Step 5: Synthesis of 5,7-dichloro-6-(2-chloroethoxy)-2-methyl-3,4-dihydroisoquinoline-2-iodo

Compound **H115-4** (250 mg, 0.9 mmol) was dissolved in acetone (12 mL), then iodomethane (1.3 g, 9 mmol) was added in at 0 °C and reacted for 6 h. The reaction solution was filtered and concentrated under reduced pressure to obtain a yellow solid **H115-5** (376 mg, yield: 100%). LCMS (ESI): *m*/*z* =292.0[M]⁺.

### Step 6: 5,7-Dichloro-6-(2-chloroethoxy)-1-(4-methoxyphenyl)-2-methyl-1,2,3,4-tetrahydroisoquinoline

Compound **H115-5** (150 mg, 0.51 mmol) was dissolved in THF (18 mL) in a three-neck flask, cooled down to -70 °C under N₂ and **S3** (3.2 mL, 3.08 mmol, 1 M in THF) was added in and reacted for 6 h. The reaction was extracted with iced aq. NH₄Cl and EA, and the organic phase was dried with anhydrous Na₂SO₄, filtered and concentrated under reduced pressure to obtain a yellow oil compound **H115-6** (60 mg, yield: 29.2%). LCMS (ESI): *m*/*z =400.0* [M+H]⁺.

### Step 7: 4-(5,7-dichloro-6-(2-chloroethoxy)-2-methyl-1,2,3,4-tetrahydroisoquinolin-1-yl)phenol

Compound **H115-6** (45 mg, 0.11 mmol) was dissolved in DCM (5 mL) in a three-neck flask and boron tribromide (0.34 mL, 0.34 mmol) was added in under N₂ protection at 0 °C and reacted for 16 h. The reaction solution was extracted with iced aq. NH₄Cl and DCM, and the organic phase was dried over anhydrous Na₂SO₄, filtered and concentrated under reduced pressure. The residue was subjected to prep-HPLC purification to obtain HANT-115 (4.4 mg, yield: 10.1%) as a white solid. LCMS (ESI): *m*/*z* = 386.0 [M+H]⁺; ¹H NMR (400 MHz, CD₃OD) δ 7.03 (d, *J =* 8.6 Hz, 2H), 6.78 (d, *J =* 8.6 Hz, 2H),6.61 (s, 1H), 4.23 (d, *J =* 5.4 Hz, 3H), 3.87 (t, *J=* 5.8 Hz, 2H), 3.20-3.11 (m, 1H), 3.04-2.89 (m, 2H), 2.71-2.53 (m, 1H), 2.21 (s, 3H).

### Example 15

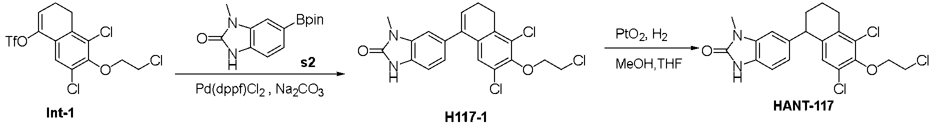

Synthesis was performed using methods described in **Example 1** to obtain **HANT-117** (15.1 mg, white solid, yield: 37.5%). LCMS (ESI): *m*/*z* =425.0 [M+H]⁺; ¹H NMR (400 MHz, CD₃OD) δ 7.00 (d, *J=* 8.0 Hz, 1H), 6.86 (d, *J =* 1.4 Hz, 1H), 6.81-6.75 (m, 2H), 4.23 (t, *J=* 5.8 Hz, 2H), 4.16-4.09 (m, 1H), 3.88 (t, *J =* 5.8 Hz, 2H), 3.34 (s, 3H), 2.87 (dd, *J =* 16.1, 6.2 Hz, 2H), 2.11 (s, 1H), 2.03-1.76 (m, 3H).

### Example 16

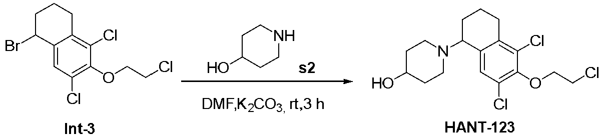

**INT-3** (100 mg, 0.28 mmol), **S2** (56 mg, 0.56 mmol) and potassium carbonate (77 mg, 0.56 mmol) were dissolved in DMF (3 mL) and reacted at room temperature for 3 h. The reaction solution was extracted with EA, and the organic phase was concentrated under reduced pressure. The residue was purified by prep-HPLC to obtain the white solid **HANT-123** (78.7 mg, yield: 74%). LCMS (ESI): *m*/*z* = 378.1 [M+H]⁺; ¹H NMR (400 MHz, DMSO-d₆) δ 7.69 (s, 1H), 4.52 (d, *J=* 4.2 Hz, 1H), 4.24-4.16 (m, 2H), 3.95 (dd, *J =* 6.1, 4.4 Hz, 2H), 3.78-3.67 (m, 2H).3.78-3.67 (m, 1H), 3.48-3.38 (m, 1H), 2.79-2.65 (m, 2H), 2.60-2.51 (m, 1H), 2.48-2.39 (m, 2H), 2.11 (t, *J* = 10.5 Hz, 1H), 2.04-1.96 (m, 1H), 1.92-1.85 (m, 1H)), 1.79-1.67 (m, 2H), 1.65-1.53 (m, 1H), 1.54-1.41 (m, 2H), 1.39-1.22 (m, 1H).

### Example 17

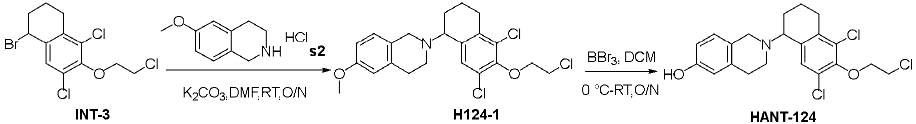

### Step 1: Synthesis of 2-(5,7-dichloro-6-(2-chloroethoxy)-1,2,3,4-tetrahydronaphthalen-1-yl)-6-methoxy-1,2,3,4tetrah ydroisoquinoline

Compounds **INT-3** (200 mg, 0.56 mmol) and S2 (279.3 mg, 1.4 mmol) were dissolved in DMF (12 mL), then potassium carbonate (154.2 mg, 1.12 mmol) was added in and the reaction was kept at room temperature overnight. The reaction mixture was extracted with saturated aq. NH₄Cl and EA, and the organic phase was washed with aq. NH₄Cl and saturated brine, dried over Na₂SO₄ and concentrated by filtration under reduced pressure. The residue was purified by flash chromatography to obtain the colorless oily compound **H124-1** (90 mg, yield: 36.6%). LCMS (ESI): *m*/*z* =440[M+H]⁺.

### Step 2: Synthesis of 2-(5,7-dichloro-6-(2-chloroethoxy)-1,2,3,4-tetrahydronaphthalen-1-yl)-1,2,3,4-tetrahydroisoqui nolin-6-ol

Compound **H124-1** (90 mg, 0.20 mmol) was dissolved in DCM (8 mL), then BBr₃ (2.0 mL, 2.0 mmol) was added in at 0 °C. The flask was sealed tightly and stirred overnight at room temperature. The reaction solution was extracted with saturated brine and DCM, and the organic phase was washed with saturated brine, dried over Na₂SO₄ and concentrated under reduced pressure. The residue was purified by neutral reversed-phase prep-HPLC to obtain **HANT-124** (41.0 mg, yield: 51.7%) as a yellow solid. LCMS (ESI): m/z =426[M+H]⁺; ¹H NMR (400 MHz, CDCl₃) δ 7.79 (s, 1H), 6.89 (d, *J =* 8.2 Hz, 1H), 6.65-6.60 (m, 2H), 4.63 (s, 1H), 4.25 (t, *J =* 6.3 Hz, 2H), 3.87 (t, *J =* 6.4 Hz, 3H), 3.81 (d, *J =* 14.2 Hz, 1H), 3.67 (d, *J =* 13.7 Hz, 1H),2.92-2.73 (m, 4H), 2.65-2.55 (m, 2H), 2.16-2.02 (m, 2H), 1.64 (dd, *J=* 15.2, 6.0 Hz, 2H).

### Example 18

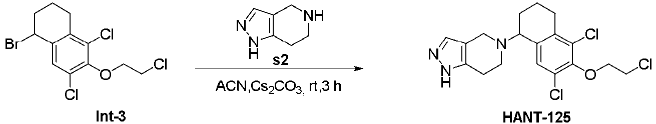

Compounds INT-3 (150 mg, 0.42 mmol), S2 (164 mg, 0.84 mmol) and cesium carbonate (273 mg, 0.84 mmol) were dissolved in acetonitrile (5 mL), and the reaction was stirred at room temperature for 3 h. The reaction solution was extracted with EA, and the organic phase was concentrated under reduced pressure. The residue was purified by prep-HPLC to obtain a white solid HANT-125 (99.5 mg, yield: 59.6%). LCMS (ESI): *m*/*z* = 400.1[M+H]⁺; ¹H NMR (400 MHz, DMSO-d₆) δ 12.34 (s, 1H), 7.69 (s, 1H), 7.30 (s, 1H), 4.22 (dd, *J =* 6.0, 4.4 Hz, 2H), 3.96 (dd, *J=* 5.9, 4.5 Hz, 3H), 3.60-3.49 (m, 2H), 2.84-2.53 (m, 6H), 2.10-1.91 (m, 2H), 1.64 (dt, *J=* 21.9, 11.7 Hz, 2H).

### Example 19

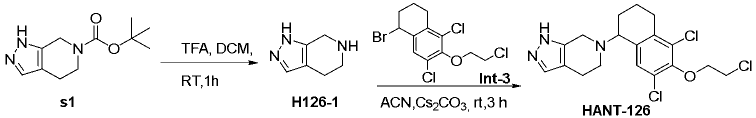

### Step 1: Synthesis of 3a,4,5,6,7,7a-hexahydro-1H-pyrazolo[3,4-c]pyridine

Compound **S1** (230 mg, 1.03 mmol) and TFA (1 mL) were dissolved in DCM (3 mL) and reacted at room temperature for 1 h. The reaction solution was concentrated under reduced pressure to obtain the yellow oily compound **H126-1** (110 mg, Yield: 91%). LCMS (ESI): *m*/*z* =126.1[M+H]⁺.

### Step 2: Synthesis of 6-(5,7-dichloro-6-(2-chloroethoxy)-1,2,3,4-tetrahydronaphthalen-1-yl)-3a,4,5,6,7,7a-hexahydro -1H-pyrazolo[3,4-c]pyridine

Compound **H126-1** (110 mg, 1.06 mmol), **INT-3** (370 mg, 1.06 mmol) and cesium carbonate (690 mg, 2.12 mmol) were dissolved in acetonitrile (5 mL), and the reaction was stirred at room temperature for 3 h. The reaction solution was extracted with EA, and the organic phase was concentrated under reduced pressure. The residue was purified by prep-HPLC to obtain a white solid **HANT-126** (73.6 mg, yield: 18%). LCMS (ESI): *m*/*z* =402.1[M+H]⁺; ¹H NMR (400 MHz, DMSO-d₆) δ 12.33 (s, 1H), 7.69 (s, 1H), 7.41 (s, 1H), 4.22 *(t, J =* 5.2 Hz, 2H), 3.96 (dd, J= 6.0, 4.5 Hz, 3H), 3.61 (s, 2H), 2.79 (d, *J* = 17.4 Hz, 1H), 2.70-2.52 (m, 5H), 2.01 (d, *J=* 13.2 Hz, 2H), 1.70-1.52 (m, 2H).

### Example 20

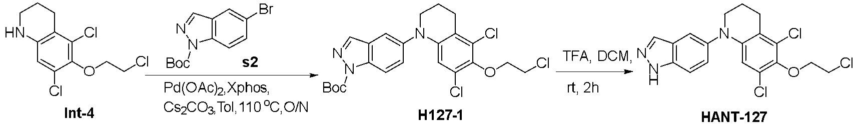

### Step 1: Synthesis of tert-butyl 5-(5,7-dichloro-6-(2-chloroethoxy)-3,4-dihydroquinolin-1(2H)-yl)-1H-indazole-1-carboxylate

Compound **INT-4** (150 mg, 0.54 mmol), **S2** (191 mg, 0.65 mmol), Pd(OAc)₂ (12 mg, 0.05 mmol), XPhos (51.3 mg, 0.1 mmol), and Cs₂CO₃ (350 mg, 1.1 mmol) were dissolved in Tol (5 mL) and stirred at 110 °C overnight. The reaction was cooled to room temperature and extracted with EA. The organic phase was dried and concentrated, and the residue was purified by flash chromatography to obtain **H127-1** as a solid (130 mg, yield: 49%). LCMS (ESI): *m*/*z* = 496.1 [M+H]⁺.

### Step 2: Synthesis of 5,7-dichloro-6-(2-chloroethoxy)-1-(1H-indazol-5-yl)-1,2,3,4-tetrahydroquinoline

Compound **H127-1** (130 mg, 0.26 mmol) and TFA (1 mL) were dissolved in DCM (3 mL) and stirred at room temperature for 2 h. The reaction solution was adjusted to pH~8 with aqueous sodium bicarbonate, extracted with EA, and the organic phase was concentrated under reduced pressure. The residue was purified by prep-HPLC to obtain a white solid **HANT-127** (17.1 mg, yield: 58%). LCMS (ESI): *m*/*z* = 396.0 [M+H]⁺; ¹H NMR (400 MHz, CD₃OD) δ 8.04 (d, *J= 0.9* Hz, 1H), 7.65-7.56 (m, 2H), 7.25 (dd, *J =* 8.9, 1.9 Hz, 1H), 6.24 (s, 1H), 4.13 (t, *J=* 5.8Hz, 2H), 3.83 (t, *J =* 5.8 Hz, 2H), 3.61-3.55 (m, 2H), 2.89 (t, *J =* 6.6 Hz, 2H), 2.14-2.05 (m, 2H).

### Example 21

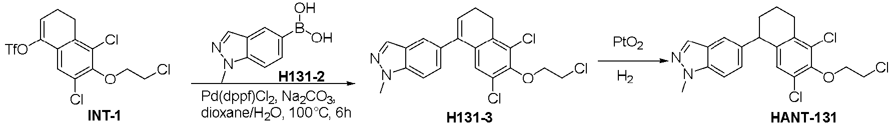

Synthesis was performed using methods described in **Example 10** to afford compound **HANT-131** (4.2 mg, white solid), yield: 16.7%. LCMS (ESI): *m*/*z* =409 [M+H]⁺; ¹H NMR (400 MHz, CD₃OD) δ 7.92 (d, *J =* 1.0 Hz, 1H), 7.49 (dt, *J =* 8.8, 1.0 Hz, 1H), 7.40 (dt, *J =* 1.6, 0.8 Hz, 1H), 7.17 (dd, *J =* 8.8, 1.6 Hz, 1H), 7.40 (dt, *J =* 1.6, 0.8 Hz, 1H).1H), 7.40 (dt, *J =* 1.6, 0.8 Hz, 1H), 7.17 (dd, *J=* 8.8, 1.6 Hz, 1H), 6.78 (d, *J=* 1.0 Hz, 1H), 4.22 (dt, *J=* 11.4, 6.4 Hz, 3H), 4.05 (s, 3H), 3.88 (t, *J =* 5.8 Hz, 2H), 2.96-2.78 (m, 2H), 2.23-2.11 (m, 1H), 2.05-1.80 (m, 3H).

### Example 22

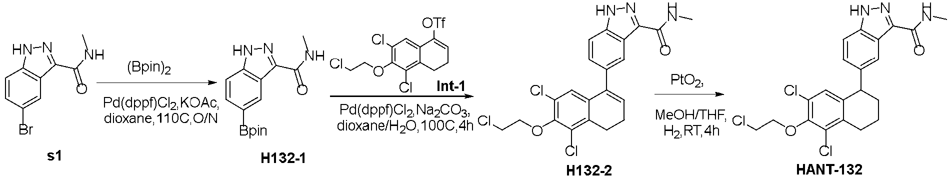

### Step 1: Synthesis of N-methyl-5-(4,4,5,5-tetramethyl-1,3,2-dioxobenzofuran-2-yl)-1H-indazole-3-carboxamide

Compound S1 (540 mg, 2.13 mmol), (Bpin)₂ (705 mg, 2.77 mmol), potassium acetate (418 mg, 4.26 mmol), and Pd(dppf)Cl2 (625 mg, 0.852 mmol) were sequentially added into dioxane (30 mL) in a dry flask and reacted overnight at 110 °C under N₂ protection. The reaction was cooled to room temperature, extracted with saturated aq. NH₄Cl and EA. The organic phase was washed with saturated brine, dried with Na₂SO₄, filtrated and concentrated under reduced pressure to obtain the crude black solid **H132-1** (730 mg, purity: 62%). LCMS (ESI): *m*/*z* =302[M+H]⁺.

### Step 2: Synthesis of 5-(5,7-dichloro-6-(2-chloroethoxy)-3,4-dihydronaphthalen-1-yl)-N-methyl-1H-indazole-3-carb oxamide

The crude **H132-1** (330 mg, 1.1 mmol) and **INT-1** (289 mg, 1.1 mmol) were dissolved in a mixture of dioxane/water (30 mL/6 mL), then sodium carbonate (144.4 mg, 2.2 mmol) and Pd(dppf)Cl2 (99.7 mg, 0.22 mmol) were added in, and the reaction was stirred for 4 h at 100 °C under N₂ protection. The reaction was cooled, extracted with saturated aq. NH₄Cl and EA. The organic phase was washed with saturated brine, dried with Na₂SO₄, filtered and concentrated under reduced pressure. The residue was purified by flash chromatography to obtain the yellow solid **H132-2** (100 mg, yield: 32.6%). LCMS (ESI): *m*/*z* =450[M+H]⁺.

### Step 3: Synthesis of 5-(5,7-dichloro-6-(2-chloroethoxy)-1,2,3,4-tetrahydronaphthalen-1-yl)-N-methyl-1H-indazole-3 -carboxamide

**H132-2** (112 mg, 0.25 mmol) was dissolved in MeOH (10 mL)/THF (5 mL), then PtO₂ (28.2 mg, 0.125 mmol) was added in and stirred under H₂ at room temperature for 4 h. The reaction mixture was filtered and concentrated under reduced pressure and purified by neutral reversed-phase prep-HPLC to obtain the white solid **HANT-132** (59.3 mg, yield: 57.8%). LCMS (ESI): *m*/*z* =452[M+H]⁺; ¹H NMR (400 MHz, CD₃OD) δ 7.91 (s, 1H), 7.52 (d, *J = 8.7* Hz, 1H), 7.17 (d*, J=* 8.7 Hz, 1H), 6.81 (s, 1H), 4.24 (t, *J =* 5.7 Hz, 3H), 3.89 (t*, J=* 5.7 Hz, 2H), 2.94 (s, 3H), 2.88 (d, *J = 3.5* Hz, 2H), 2.13 (dd, *J=* 13.8, 7.0 Hz, 1H), 1.93 (dd, *J* = 22.2, 9.6 Hz, 2H), 1.86-1.75 (m, 1H).

### Example 23

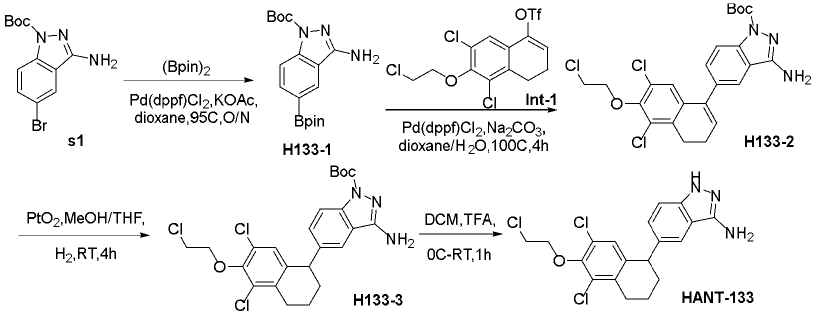

### Step 1: Synthesis of tert-butyl 3-amino-5-(4,4,5,5-tetramethy1-1,3,2-dioxobenzofuran-2-yl)-1H-indazole-1-carboxylate

Compound **S1** (300 mg, 0.96 mmol), (Bpin)₂ (366.3 mg, 1.44 mmol), potassium acetate (228.4 mg, 2.4 mmol), and Pd(dppf)Cl2 (70.4 mg, 0.096 mmol) were sequentially added to dioxane (30 mL) in a dry flask under N₂ protection at 95 °C. The reaction was cooled to room temperature, extracted with saturated aq. NH₄Cl and EA. The organic phase was washed with saturated brine, dried with Na₂SO₄, filtrated and concentrated under reduced pressure. The residue was purified by flash chromatography to obtain **H133-1** (317 mg, yield: 91.9%) as a yellow solid. LCMS (ESI): *m*/*z* =360[M+H]⁺.

### Step 2: Synthesis of tert-butyl 3-amino-5-(5,7-dichloro-6-(2-chloroethoxy)-3,4-dihydronaphthalen-1-yl)-1H-indazole-1-carbo xylate

Compound **H133-1** (317 mg, 0.88 mmol) and compound **INT-1** (375.3 mg, 0.88 mmol) were dissolved in a mixed solution of dioxane/water (30 mL/6 mL) and sodium carbonate (187.2 mg, 1.76 mmol) and Pd(dppf)Cl2 (129.3 mg, 0.176 mmol) were added into and the mixture was stirred at 100 °C for 4 h under N₂ protection. The reaction was cooled to room temperature, extracted with saturated aq. NH₄Cl and EA. The organic phase was washed with saturated brine, dried over Na₂SO₄, filtrated and concentrated under reduced pressure. The residue was purified by flash chromatography to obtain the brown oily compound **H133-2** (200 mg, yield: 44.6%). LCMS (ESI): *m*/*z* = 452 [M-56]⁺.

### Step 3: Synthesis of tert-butyl 3-amino-5-(5,7-dichloro-6-(2-chloroethoxy)-1,2,3,4-tetrahydronaphthalen-1-yl)-1H-indazole-1-carboxylate

Compound **H133-2** (200 mg, 0.39 mmol) was dissolved in MeOH (10 mL)/THF (5 mL), and then PtO₂ (44.8 mg, 0.195 mmol) was added in and stirred under H₂ at room temperature for 4 h. The reaction solution was filtered and concentrated under reduced pressure to obtain the crude yellow oily compound **H133-3** (160 mg, yield: 63.7%). LCMS (ESI): *m*/*z* =454[M-56]⁺.

### Step 4: Synthesis of 5-(5,7-dichloro-6-(2-chloroethoxy)-1,2,3,4-tetrahydronaphthalen-1-yl)-1H-indazol-3-amine

The crude compound **H133-3** (160 mg, 0.31 mmol) was dissolved in DCM (12 mL), then TFA (3 mL) was added at 0 °C and the reaction was stirred at room temperature for 1 h. The reaction mixture was filtered and concentrated under reduced pressure, and the residue was purified by neutral reversed-phase prep-HPLC to obtain **HANT-133** (32.0 mg, yield: 32.6%) as a white solid. LCMS (ESI): *m*/*z* =410[M+H]⁺; ¹H NMR (400 MHz, CD₃OD) δ 7.34 (s, 1H), 7.25 (d, *J =* 8.6 Hz, 1H), 7.09 (dd, *J =* 8.7, 1.6 Hz, 1H), 6.83 (s, 1H), 4.23 (t, *J =* 5.8 Hz, 2H), 4.17 (t, *J=* 6.7 Hz, 1H),3.88 (t, *J =* 5.8 Hz, 2H), 2.87 (dd, *J =* 9.6, 4.0 Hz, 2H), 2.15-2.07 (m, 1H), 1.99-1.77 (m, 3H).

### Example 24

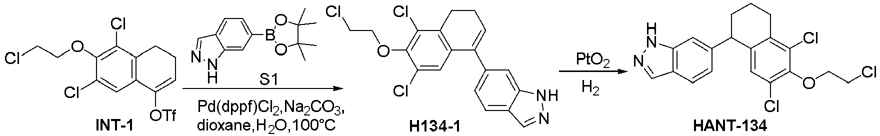

Synthesis was performed using methods described in **Example 1** to afford **HANT-134** (22.5 mg, yield: 13%). LCMS (ESI): *m*/*z* = 395.2 [M+H]⁺.¹H NMR (400 MHz, CD₃OD) δ 7.99 (s, 1H), 7.71-7.69 (m, 1H), 7.17 (s, 1H), 6.93-6.90 (m, 1H), 6.83 (s, 1H), 4.26-4.23 (m, 3H), 3.89 (t, *J =* 5.6 Hz, 2H), 2.89-2.86 (m, 2H), 2.16-2.11 (m, 1H), 1.99-1.83 (m, 2H), 1.82-1.79 (m, 1H).

### Example 25

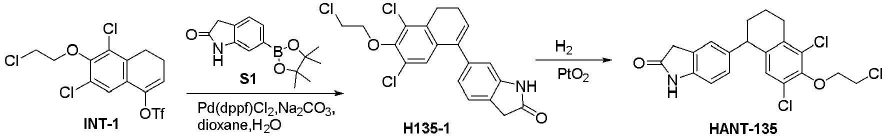

Synthesis was performed using methods described in **Example 1** to afford **HANT-135** (16.5 mg, yield: 11%). LCMS (ESI): *m*/*z* = 410.2 [M+H]⁺. ¹H NMR (400 MHz, CD₃OD) δ 6.96 (s, 1H), 6.93 (d, *J =* 8.0 Hz, 1H), 6.84-6.81 (m, 2H),4.23 (t, *J =* 5.6 Hz, 2H), 4.06-4.03 (m,1H), 3.88 (t, *J =* 6.0 Hz, 2H), 3.49 (s, 2H), 2.86-2.82 (m,2H), 2.09-2.04 (m, 1H), 1.96-1.85 (m, 1H), 1.84-1.75 (m, 2H).

### Example 26

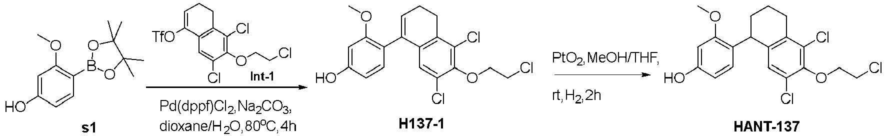

Synthesis was performed using methods described in **Example 1** to afford **HANT-137** (123.5 mg, yield: 41%) as a white solid. LCMS (ESI): *m*/*z* = 401.0 [M+H]⁺; ¹H NMR (400 MHz, DMSO-d₆) δ 9.33 (s, 1H), 6.76 (s, 1H), 6.49-6.37 (m, 2H),6.26 (dd, *J =* 8.2, 2.3 Hz, 1H), 4.29 (t, *J =* 6.1 Hz, 1H), 4.24-4.16 (m, 2H), 3.95 (t, *J =* 5.2 Hz, 2H), 3.74 (s, 3H), 2.79-2.68 (m, 2H), 1.85-1.64 (m, 4H).

### Example 27

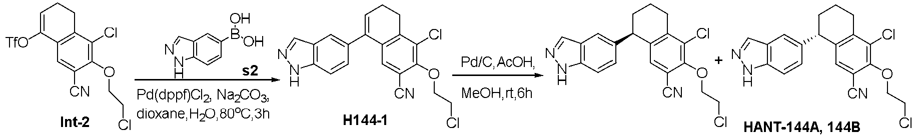

### Step 1: Synthesis of 4-chloro-3-(2-chloroethoxy)-8-(1H-indazol-5-yl)-5,6-dihydronaphthalene-2-carbonitrile

Compounds **INT-2** (200 mg, 0.48 mmol), **S2** (117 mg, 0.72 mmol), Pd(dppf)Cl2 (71 mg, 0.1 mmol) and Na₂CO₃ (102 mg, 1.0 mmol) were added to dioxane/water (5 mL/1 mL) and stirred under nitrogen protection at 80 °C for 3 h. The reaction was cooled to room temperature and extracted with EA. The organic phase was dried and concentrated, and the residue was purified by flash chromatography to obtain solid **H144-1** (220 mg, yield: 80%). LCMS (ESI): *m*/*z* =3 84.1 [M+H]⁺.

### Step 2: Synthesis of 4-chloro-3-(2-chloroethoxy)-8-(1H-indazol-5-yl)-5,6,7,8-tetrahydronaphthalene-2-carbonitrile

Compound **H144-1** (100 mg, 0.26 mmol), Pd/C (100 mg) and AcOH (0.2 mL) was dissolved in MeOH (5 mL) and reacted under H₂ at room temperature for 6 h. The reaction solution was filtrated, concentrated under reduced pressure, and the residue was purified by flash chromatography to obtain the crude white solid **HANT-144,** LCMS (ESI): *m*/*z* =386.1 [M+H]⁺, which is subjected to SFC Chiral Separation (mobile phase: Hex-EtOH-70-30-20MIN) to afford two enantiomers **HANT-144A** (17.1 mg, retention time: 6.528 min) and **HANT-144B** (15.7 mg, retention time: 9.085 min).

**HANT-144A:** ¹H NMR (400 MHz, DMSO-*d*₆) δ 13.02 (s, 1H), 7.99 (s, 1H), 7.49 (d, *J =* 8.6 Hz, 1H), 7.38 (d, *J =* 1.6 Hz, 1H), 7.18-7.06 (m, 2H), 4.44-4.37 (m, 2H), 4.29 (t, *J=* 6.2 Hz, 1H), 4.02-3.93 (m, 2H), 2.95-2.85 (m, 2H), 2.11-1.97 (m, 1H), 1.94-1.73 (m, 3H).

**HANT-144B:** ¹H NMR (400 MHz, DMSO-*d*₆) δ 13.02 (s, 1H), 7.99 (s, 1H), 7.49 (d, *J =* 8.6 Hz, 1H), 7.38 (s, 1H), 7.19-7.06 (m, 2H), 4.45-4.36 (m, 2H), 4.28 (t, *J* = 6.4 Hz, 1H), 4.02-3.91 (m, 2H), 2.95-2.86 (m, 2H), 2.10-1.99 (m, 1H), 1.93-1.70 (m, 3H).

### Example 28

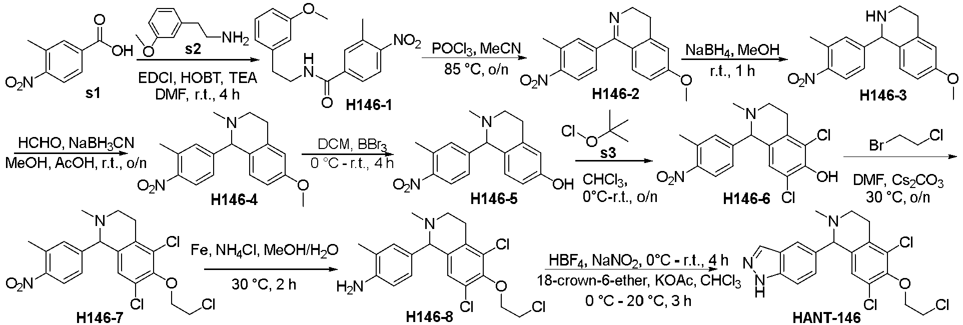

### Step 1: Synthesis of N-(3-methoxyphenethyl)-3-methyl-4-nitrobenzamide

In a dry flask, compound **S1** (5.2 g, 28.7 mmol), **S2** (4.34 g, 28.7 mmol), DMF (57 mL), EDCI (8.2 g, 43.1 mmol), HOBT (5.81 g, 43.1 mmol), and TEA (8.7 g, 86.1 mmol) were sequentially added in, and stirred at room temperature under N₂ for 4 h. The reaction was quenched with 400 mL water, and extracted by EA (100 mL*3). The organic phase was dried with anhydrous Na₂SO₄, filtered, concentrated under reduced pressure, and the residue was purified by flash chromatography (DCM: EA=1:1) to obtain **H146-1** (7.35 g, yield: 82%) as a yellow solid. LCMS (ESI): *m*/*z* =315.1 [M+H]⁺.

### Step 2: Synthesis of 6-methoxy-1-(3-methyl-4-nitrophenyl)-3,4-dihydroisoquinoline

To a dry flask was added **H146-1** (7.35 g, 2.34 mmol), acetonitrile (100 mL), POCl₃ (7.16 g, 46.8 mmol) and TEA (8.7 g, 86.1 mmol) sequentially, and stirred overnight at 85 °C under N₂. The reaction was cooled, concentrated under reduced pressure and quenched with water. The reaction mixture was extracted by EA, and the organic phase was dried with anhydrous Na₂SO₄, filtered, concentrated under reduced pressure. The residue was purified by flash chromatography (DCM: EA=1:1) to obtain **H146-2** (4.4 g, yield: 63%) as a yellow solid. LCMS (ESI): *m*/*z* =297.1 [M+H]⁺.

### Step 3: Synthesis of 6-methoxy-1-(3-methyl-4-nitrophenyl)-1,2,3,4-tetrahydroisoquinoline

Compound **H146-2** (4.4 g, 14.8 mmol), methanol (60 mL), and sodium borohydride (1.69 g, 44.6 mmol) were added sequentially into a dry flask and stirred under N₂ at room temperature for 1 h. The reaction was quenched with water, concentrated under reduced pressure and extracted by EA. The organic phase was dried with anhydrous Na₂SO₄ and filtered, concentrated to obtain a yellow solid **H146-3** (4.4 g, yield: 99%). LCMS (ESI): *m*/*z* =299.1 [M+H]⁺.

### Step 4: Synthesis of 6-methoxy-2-methyl-1-(3-methyl-4-nitrophenyl)-1,2,3,4-tetrahydroisoquinoline

Compound **H146-3** (4.4 g, 14.8 mmol), methanol (100 mL), aqueous formaldehyde (4.75 g, 147.7 mmol), and acetic acid (1 drop) were sequentially added to a dry flask and stirred for 30 min followed by addition of sodium cyanoborohydride (1.86 g, 29.5 mmol). The reaction was stirred overnight at room temperature under N₂, then quenched with water. The reaction mixture was concentrated under reduced pressure, extracted by EA, and the organic phase was dried over Na₂SO₄, filtered and concentrated to obtain **H146-4** (4.4 g, yield: 95%) as a yellow solid. LCMS (ESI): *m*/*z* =313.1 [M+H]⁺.

### Step 5: Synthesis of 2-methyl-1-(3-methyl-4-nitrophenyl)-1,2,3,4-tetrahydroisoquinolin-6-ol

Compound **H146-4** (4.4 g, 14.1 mmol), DCM (20 mL), and boron tribromide (40 mL, 17% in DCM) were added sequentially into a dry flask at 0 °C, and stirred at room temperature under N₂ for 4 h. The reaction was quenched with methanol, concentrated under reduced pressure, and adjusted pH to alkaline with aq. sodium bicarbonate then extracted by EA. The organic phase was dried with anhydrous Na₂SO₄, filtered and concentrated to obtain **H146-5** as a brown solid (4.0 g, yield: 95%). LCMS (ESI): *m*/*z* =299.1 [M+H]⁺.

### Step 6: Synthesis of 5,7-dichloro-2-methyl-1-(3-methyl-4-nitrophenyl)-1,2,3,4-tetrahydroisoquinolin-6-ol

Compound **H146-5** (4.0 g, 13.4 mmol), trichloromethane (60 mL), and **S3** (2.9 g, 26.84 mmol) were added sequentially to a dry flask at 0 °C and stirred overnight at room temperature under N₂. The reaction was diluted with water, extracted by DCM, and the organic phase was dried with anhydrous Na₂SO₄, filtered and concentrated. The residue was purified by flash chromatography (DCM:EA=30:1) to obtain **H146-6** (520 mg, yield: 10%) as a yellow solid. LCMS (ESI): *m*/*z* =367.1 [M+H]⁺.

### Step 7: Synthesis of 5,7-dichloro-2-methyl-1-(3-methyl-4-nitrophenyl)-1,2,3,4-tetrahydroisoquinolin-6-ol

Compound **H146-6** (520 mg, 1.41 mmol), DMF (15 mL), 1-bromo-2-chloroethane (304 mg, 2.12 mmol), and cesium carbonate (914 mg, 2.84 mmol) were sequentially added to a dry flask and stirred overnight at 30°C under N₂. The reaction was diluted with water, extracted by EA. The organic phase was dried with Na₂SO₄, filtered and concentrated to obtain **H146-7** (600 mg, yield: 98%) as a brown oil. LCMS (ESI): *m*/*z* =429.0 [M+H]⁺.

### Step 8: Synthesis of 4-(5,7-dichloro-6-(2-chloroethoxy)-2-methyl-1,2,3,4-tetrahydroisoquinolin-1-yl)-2-methylanilin e

To a dry flask was added compound **H146-7** (600 mg, 1.4 mmol), methanol/water (40 mL, 1/1), iron powder (468 mg, 8.4 mmol), ammonium chloride (450 mg, 8.4 mmol) sequentially and stirred at 30 °C for 2 h. The solution was filtered, concentrated under reduced pressure, extracted by EA. The organic phase was dried with Na₂SO₄, filtered, and concentrated to obtain a yellow colour solid **H146-8** (520 mg, yield: 93%). LCMS (ESI): *m*/*z* =399.1 [M+H]⁺.

### Step 9: Synthesis of 5,7-dichloro-6-(2-chloroethoxy)-1-(1H-indazol-5-yl)-2-methyl-1,2,3,4-tetrahydroisoquinoline

Compound **H146-8** (350 mg, 0.88 mmol), tetrafluoroboric acid (6 mL, 50% in H₂O), sodium nitrite (75 mg, 1.1 mmol) were added sequentially to a dry flask and stirred at room temperature for 4 h, then trichloromethane (6 mL), water (6 mL), potassium acetate (515 mg, 5.3 mmol), 18-crown-6-ether (116 mg, 0.44 mmol) were added in, and stirred at room temperature for 3 h. The reaction was extracted with DCM, and the organic phase was dried with anhydrous Na₂SO₄, then filtered and concentrated. The residue was purified by flash chromatography to obtain the white solid crude **HANT-146** (yield: 29%). LCMS (ESI): *m*/*z* =410.1[M+H]⁺. Racemic **HANT-146** is subjected to SFC Chiral Separation (mobile phase: Hex:EtOH = 70:30-15 min) to afford two enantiomers **HANT-146A** (53.6 mg, retention time 5.214 min) and the yellow solid **HANT-146B** (50.8 mg, retention time 6.642 min).

**HANT-146A:** ¹H NMR (400 MHz, CD₃OD) δ 8.06 (s, 1H), 7.72 (s, 1H), 7.52 (d, *J* = 8.8 Hz, 1H), 7.19 (dd, *J =* 8.8, 1.6 Hz, 1H), 6.59 (s, 1H), 4.40 (s, 1H), 4.22 (t, *J =* 5.8 Hz, 2H), 3.86 (t, *J =* 5.8 Hz, 2H), 3.25-3.19 (m, 1H), 3.13-2.94 (m, 2H), 2.71-2.63 (m, 1H), 2.23 (s, 3H).

**HANT-146B:** ¹H NMR (400 MHz, CD₃OD) δ 8.06 (d, *J*=1.1 Hz, 1H), 7.72 (s, 1H), 7.52 (d, *J* = 8.8 Hz, 1H), 7.19 (dd, *J* =8.8, 1.6 Hz, 1H), 6.58 (s, 1H), 4.39 (s, 1H), 4.22 (t, *J* =5.8 Hz, 2H), 3.86 (t, *J*=5.8 Hz, 2H), 3.25-3.19 (m, 1H), 3.13-2.93 (m, 2H), 2.70-2.62 (m, 1H), 2.22 (s, 3H).

### Example 29

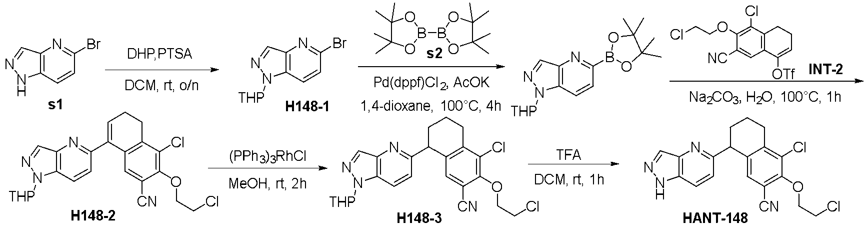

### Step 1: Synthesis of 5-bromo-1-(tetrahydro-2H-pyran-2-yl)-1H-pyrazolo[4,3-b]pyridine

S1 (500 mg, 2.52 mmol), DHP (427 mg, 5.05 mmol) and PTSA (242 mg, 1.26 mmol) were dissolved in DCM (15 mL) and the reaction was stirred under N₂ overnight at room temperature. The reaction was concentrated under reduced pressure and the residue was purified by flash chromatography to obtain compound **H148-1** (520 mg, colorless oil, yield: 73.0%). LCMS (ESI): *m*/*z* =282.0 [M+H]⁺.

### Step 2: Synthesis of 4-chloro-3-(2-chloroethoxy)-8-(1-(tetrahydro-2H-pyran-2-yl)-1H-pyrazolo[4,3-b]pyridin-5-yl)-5,6-dihydronaphthalene-2-carbonitrile

Compound **H148-1** (200 mg, 0.71 mmol), **S2** (360 mg, 1.42 mmol), Pd(dppf)Cl₂ (104 mg, 0.14 mmol), and potassium acetate (208 mg, 2.13 mmol) were dissolved in dioxane (10 mL), and reacted under N₂ at 100 °C for 4 h. The reaction was cooled to room temperature, then **INT-2** (148 mg, 0.35 mmol), sodium carbonate (150 mg, 1.42 mmol) and water (1 mL) were added in, and the reaction was heated to 100 °C and reacted for 1 h under N₂. The reaction was then cooled to room temperature, concentrated under reduced pressure and the residue was purified by flash chromatography to obtain the compound **H148-2** (90 mg, colorless oil, yield: 27.1%). LCMS (ESI): *m*/*z* = 469.1 [M+H]⁺.

### Step 3: Synthesis of 4-chloro-3-(2-chloroethoxy)-8-(1-(tetrahydro-2H-pyran-2-yl)-1H-pyrazolo[4,3-b]pyridin-5-yl)-5,6,7,8-tetrahydronaphthalene-2-carbonitrile

Compound **H148-2** (80 mg) and tris(triphenylphosphine)rhodium chloride (40 mg, 50% wt) were dissolved in methanol (5 mL) and reacted under H₂ at room temperature for 2 h. The reaction was concentrated under reduced pressure, and the residue was purified by flash chromatography to obtain compound **H148-3** (24 mg, colorless oil, yield: 29.9%). LCMS (ESI): *m*/*z* =471.1[M+H]⁺.

### Step 4: Synthesis of 4-chloro-3-(2-chloroethoxy)-8-(1H-pyrazolo[4,3-b]pyridin-5-yl)-5,6,7,8-tetrahydronaphthalene-2-carbonitrile

Compound **H148-3** (24 mg) was dissolved in DCM (2 mL), and trifluoroacetic acid (1 mL) was added dropwise to the reaction solution, and the reaction was stirred at room temperature for 1 h. The reaction solution was concentrated under reduced pressure, and the residue was purified by flash chromatography to obtain the compound **HANT-148** (2.5 mg, white solid, yield: 12.7%). LCMS (ESI): *m*/*z* =387.1[M+H]⁺; ¹H NMR (400 MHz, CD₃OD) δ 8.14 (s, 1H), 8.00 (d, *J =* 8.8 Hz, 1H), 7.26 (d, *J =* 8.8 Hz, 1H), 7.04 (s, 1H), 4.52-4.45 (m, 1H), 4.41 (t, *J* = 5.8 Hz, 2H), 3.91 (t, *J =* 5.6 Hz), 2H), 3.07-2.91 (m, 2H), 2.24-2.15 (m, 1H), 2.11-1.96 (m, 2H), 1.91-1.83 (m, 1H).

### Example 30

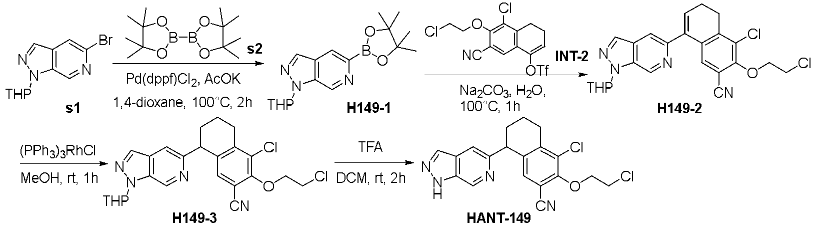

Synthesis was performed using methods described in **Example 29** to afford **HANT-149** as a white solid, yield: 39.3%). LCMS (ESI): *m*/*z* =387.1 [M+H]⁺; ¹H NMR (400 MHz, CD₃OD) δ 8.93 (s, 1H), 8.12 (s, 1H), 7.51 (s, 1H), 7.04 (s, 1H), 4.48-4.34 (m, 3H), 3.91 (t, *J =* 5.6 Hz, 2H), 3.06-2.91 (m, 2H), 2.23-2.07 (m, 2H), 2.03-1.92 (m, 1H), 1.88-1.78 (m, 1H).

### Example 31

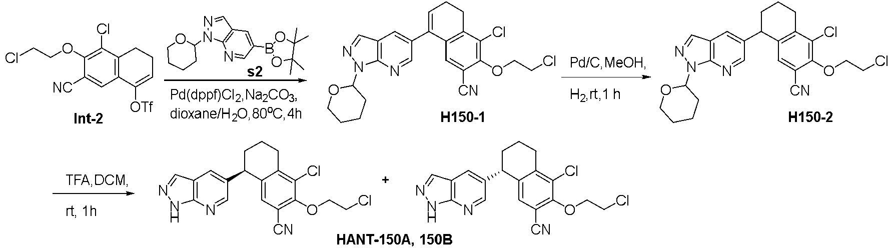

### Step 1: Synthesis of 4-chloro-3-(2-chloroethoxy)-8-(1-(tetrahydro-2H-pyran-2-yl)-1H-pyrazolo[3,4-b]pyridin-5-yl)-5,6-dihydronaphthalene-2-cyanide

Compound **INT-2** (500 mg, 1.2 mmol), **S2** (595 mg, 1.8 mmol), Pd(dppf)Cl₂ (176 mg, 0.24 mmol), and Na₂CO₃ (255 mg, 2.4 mmol) were dissolved in dioxane/water (10 mL/2mL) and stirred under N₂ at 80 °C for 4 h. The reaction was cooled to room temperature, extracted with EA. The organic phase was dried and concentrated, and the residue was purified by flash chromatography to obtain **H150-1** as a solid (520 mg, yield: 92%). LCMS (ESI): *m*/*z* = 469.1 [M+H]⁺.

### Step 2: Synthesis of 4-chloro-3-(2-chloroethoxy)-8-(1-(tetrahydro-2H-pyran-2-yl)-1H-pyrazolo[3,4-b]pyridin-5-yl)-5,6,7,8-tetrahydronaphthalene-2-cyanide

Compound **H150-1** (520 mg, 1.1 mmol) and Pd/C (300 mg) were dissolved in MeOH (10 mL) and reacted under H₂ at room temperature for 1 h. The reaction solution was filtered, concentrated under reduced pressure to obtain compound **H150-2** (500 mg, white solid, yield: 96%). LCMS (ESI): *m*/*z* =471.1 [M+H]⁺.

### Step 3: Synthesis of 4-chloro-3-(2-chloroethoxy)-8-(1H-pyrazolo[3,4-b]pyridin-5-yl)-5,6,7,8-tetrahydronaphthalene-2-carbonitrile

Compound **H150-2** (200 mg, 0.5 mmol) and TFA (5 mL) were dissolved in DCM (10 mL) and stirred at room temperature for 1 h. The reaction solution was adjusted to pH~8 with aq. sodium bicarbonate then extracted with EA. The organic phase was dried and concentrated, and the residue was purified by flash chromatography to obtain the crude product **HANT-150,** LCMS (ESI): *m*/*z* =387.1 [M+H]⁺. The racemic **HANT-150** was subjected to SFC chiral separation (Hex-EtOH-70-30-30MIN) to afford two enantiomers **HANT-150A** (37.4 mg, RT = 8.159 min) and **HANT-150B** (39 mg, RT = 10.662 min).

**HANT-150A:** ¹H NMR (400 MHz, DMSO-*d*₆) δ 13.60 (s, 1H), 8.38 (d, *J =* 2.2 Hz, 1H), 8.06 (s, 1H), 7.80 (d, *J =* 2.2 Hz, 1H), 7.24 (s, 1H), 4.41 (t, *J =* 5.2 Hz, 3H), 4.07-3.89 (m, 2H), 3.03-2.80 (m, 2H), 2.18-2.03 (m, 1H), 1.98-1.74 (m, 3H).

**HANT-150B:** ¹H NMR (400 MHz, DMSO-*d*₆) δ 13.60 (s, 1H), 8.38 (d, *J =* 2.2 Hz, 1H), 8.06 (s, 1H), 7.80 (d, *J* = 2.2 Hz, 1H), 7.24 (s, 1H), 4.49-4.34 (m, 3H), 4.04-3.92 (m, 2H), 3.01-2.78 (m, 2H), 2.11-2.03 (m, 1H), 1.97-1.74 (m, 3H).

### Example 32

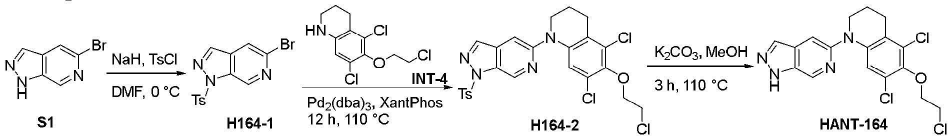

### Step 1: Synthesis of 5-bromo-1-toluenesulfonyl-1H pyrazolo[3,4-c]pyridine

Compound S1 (500 mg, 2.5 mmol), TsCl (675 mg, 3.5 mmol) and NaH (131 mg, 3.3 mmol) were added to DMF (20 mL) in a flask in an ice bath for 1 h. The reaction solution was extracted with EA, and the organic phase was dried and concentrated under reduced pressure. The residue was purified by flash chromatography to obtain a pink solid **H164-1** (650 mg, yield: 68%). LCMS (ESI): *m*/*z* =351.1 [M+H]⁺.

### Step 2: Synthesis of 5,7-dichloro-6-(2-chloroethoxy)-1-(1-toluenesulfonyl-1H-pyrazolo[3,4-c]pyridin-5-yl)-1,2,3,4-t etrahydroquinoline

**H164-1** (230 mg, 0.66 mmol), **INT-4** (92 mg, 0.33 mmol), Pd₂(dba)₃ (91 mg, 0.1 mmol), Xantphos (115 mg, 0.2 mmol), and Cs₂CO₃ (215 mg, 0.65 mmol) were dissolved in toluene (5 mL) under N₂ and the reaction was kept at 110 °C for 12 h. The reaction solution was cooled to room temperature and extracted with EA. The organic phase was concentrated under reduced pressure, and the residue was purified by column chromatography to obtain the yellow oil compound **H164-2** (180 mg, yield: 98%). LCMS (ESI): *m*/*z* =551.1[M+H]⁺.

### Step 3: Synthesis of 5,7-dichloro-6-(2-chloroethoxy)-1-(1H-pyrazolo[3,4-c]pyridin-5-yl)-1,2,3,4-tetrahydroquinolin e

**H164-2** (170 mg, 0.31 mmol) and K₂CO₃ (214 mg, 1.55 mmol) were dissolved in MeOH (30 mL) and reacted at 110 °C for 3 h. The reaction was cooled to room temperature and extracted with EA. The organic phase was concentrated under reduced pressure and the residue was purified by flash chromatography to obtain a white solid **HANT-164** (12 mg, yield: 12%). LCMS (ESI): *m*/*z* =397.0[M+H]⁺; ¹H NMR (400 MHz, CD₃OD) δ 8.88 (s, 1H), 8.14 (s, 1H), 7.56 (s, 1H), 6.53 (s, 1H), 4.16-3.87 (m, 2H), 3.85-3.71 (m, 2H), 3.70-3.30 (m, 2H), 2.89-2.87 (m, 2H), 2.07 (m, 2H).

### Example 33

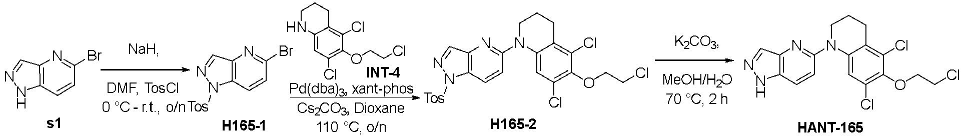

### Step 1: Synthesis of 5-bromo-1-toluenesulfonyl-1H-pyrazolo[4,3-b]pyridine

Compound **S1** (500 mg, 2.53 mmol), DMF (25 mL) and sodium hydride (131 mg, 3.28 mmol) were sequentially added to a dry flask at 0°C. After stirred for 30 min, TosCl (674 mg, 3.54 mmol) was added and stirred overnight at room temperature under N₂. The reaction was quenched with water and extracted with EA. The organic phase was dried with anhydrous Na₂SO₄, filtered, concentrated under reduced pressure, and the residue was purified by flash chromatography to obtain **H165-1** (600 mg, yield: 67%) as a white solid. LCMS (ESI): *m*/*z* =352.0 [M+H]⁺.

### Step 2: Synthesis of 5,7-dichloro-6-(2-chloroethoxy)-1-(1-toluenesulfonyl-1H-pyrazolo[4,3-b]pyridin-5-yl)-1,2,3,4-t etrahydroquinoline

Compound **H165-1** (253 mg, 0.72 mmol), dioxane (10 mL), **INT-4** (100 mg, 0.36 mmol), cesium carbonate (234 mg, 0.72 mmol), **Xant-phos** (83 mg, 0.14 mmol), Pd₂(dba)₃ (66 mg, 0.072 mmol) were sequentially added into a flask and stirred overnight at 110 °C under N₂. The reaction was cooled to room temperature and concentrated under reduced pressure. The residue was purified by flash chromatography to obtain **H165-2** (78 mg, yield: 39%) as a yellow solid. LCMS (ESI): *m*/*z* =551.0 [M+H]⁺.

### Step 3: Synthesis of 5,7-dichloro-6-(2-chloroethoxy)-1-(1H-pyrazolo[4,3-b]pyridin-5-yl)-1,2,3,4-tetrahydroquinolin e

To a dry flask was sequentially added **H165-2** (60 mg, 0.11 mmol), methanol (3 mL), and saturated aq. potassium carbonate (1 mL). The reaction was stirred under N₂ at 70 °C for 2 h. The reaction was cooled to room temperature, concentrated under reduced pressure, diluted with water, and extracted by EA. The organic phase was dried with Na₂SO₄, filtered and concentrated, and the residue was purified by prep-HPLC to obtain **HANT-165** (10.7 mg, yield: 24.5%) as a white solid. LCMS (ESI): *m*/*z* =397.0 [M+H]⁺; ¹H NMR (400 MHz, DMSO-*d*₆): ¹H NMR (400 MHz, DMSO-*d*₆): 1.0 mmol.MHz, DMSO-d6) δ 13.19 (s, 1H), 8.08 (s, 1H), 7.96 (d, *J* = 9.0 Hz, 1H), 7.25 (d, *J =* 9.0 Hz, 1H), 7.08 (s, 1H), 4.18 (t, *J =* 5.2 Hz, 2H), 3.95 (t, *J =* 5.2 Hz, 2H), 3.84-3.77 (m, 2H), 2.80 (t, *J =* 6.8 Hz, 2H), 1.99-1.89 (m, 2H).

### Example 34

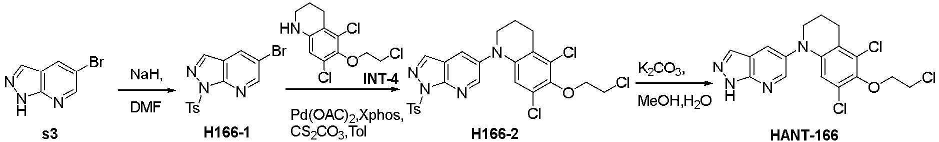

### Step 1: Synthesis of 5-bromo-1-toluenesulfonyl-1H-pyrazolo[3,4-b]pyridine

Compound **S3** (4.0 g, 20.2 mmol) was dissolved in DMF (50 mL), then NaH (1.0 g, 26.3 mmol) was added in and stirred in an ice bath for 30 min under N₂. Then TosCl (5.4 g, 28.3 mmol) was added and reacted for 12 h. The reaction was extracted by EA, washed with saturated aq. NH₄Cl, and the organic phase was dried with anhydrous Na₂SO₄, filtered, concentrated. The residue was slurried with PE to obtain the red solid **H166-1** (4.4 g, yield: 62%). LCMS (ESI): *m*/*z* =352.1 [M+H]⁺.

### Step 2. Synthesis of 5,7-dichloro-6-(2-chloroethoxy)-1-(1-toluenesulfonyl-1H pyrazolo[3,4-b]pyridin-5-yl)-1,2,3,4-t etrahydroquinoline

To a dry flask were sequentially added compounds **H166-1** (100 mg, 0.36 mmol), **INT-4** (190 mg, 0.54 mmol), Pd(OAc)₂ (16 mg, 0.072 mmol), X-Phos (68 mg, 0.15 mmol), and cesium carbonate (350 mg, 1.44 mmol) in Tol (15 mL), and reacted at 110 °C for 12 h. The reaction was cooled to room temperature, filtered and concentrated. The residue was purified by flash chromatography (PE:EA = 2:1) to obtain **H166-2** (40 mg, yield: 21%). LCMS (ESI): *m*/*z* =551.2[M+H]⁺.

### Step 3. Synthesis of 5,7-dichloro-6-(2-chloroethoxy)-1-(1H-pyrazolo[3,4-b]pyridin-5-yl)-1,2,3,4-tetrahydroquinolin e

**H166-2** (40 mg, 0.07 mmol), potassium carbonate (50 mg, 0.35 mmol), methanol/water (5 mL/2 mL) were sequentially added in a dry flask and reacted at 50 °C for 12 h. The reaction was cooled to room temperature, filtered and concentrated. The residue was purified by Prep-HPLC to obtain **HANT-166** (4.5 mg, yield: 15%). LCMS (ESI): *m*/*z* = 397.2[M+H]⁺; ¹H NMR (400 MHz, CDCl₃) δ 8.47 (d, *J =* 2.0 Hz, 1H), 8.09 (s, 1H), 7.93 (d, *J =* 2.4 Hz, 1H), 6.30 (s, 1H), 4.18 (t, *J =* 6.4 Hz, 2H), 3.85 (t, *J =* 6.0Hz, 2H), 3.59 (t, *J =* 6.4 Hz, 2H), 2.91 (t, *J =* 6.8 Hz, 2H), 2.14-2.11 (m, 2H).

### Example 35

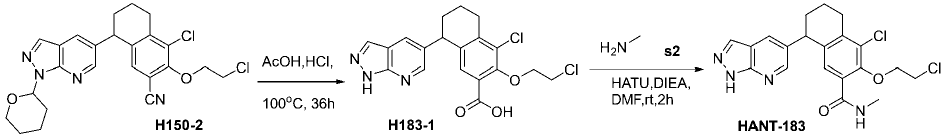

### Step 1: Synthesis of 4-chloro-3-(2-chloroethoxy)-8-(1H-pyrazolo[3,4-b]pyridin-5-yl)-5,6,7,8-tetrahydronaphthalene-2-carboxylic acid

Compound **H150-2** (100 mg, 0.21 mmol) were stirred in AcOH (4 mL) and HCl (2 mL) at 100 °C for 36 h. The reaction was cooled to room temperature,then adjusted to pH~8 with aq. sodium bicarbonate. The reaction mixture was extracted with EA, and the organic phase was dried and concentrated. The residue was purified by flash chromatography to obtain the oily compound **H183-1** (60 mg, yield: 70%). LCMS (ESI): *m*/*z* =406.1[M+H]⁺.

### Step 2: Synthesis of 4-chloro-3-(2-chloroethoxy)-N-methyl-8-(1H-pyrazolo[3,4-b]pyridin-5-yl)-5,6,7,8-tetrahydrona phthalene-2-carboxamide

**H183-1** (60 mg, 0.15 mmol), S2 (0.1 mL), HATU (113 mg, 0.3 mmol) and DIEA (38 mg, 0.3 mmol) were dissolved in DMF (3 mL) and reacted at room temperature for 2 h. The reaction mixture was extracted with EA, and the organic phase was dried and concentrated. The residue was purified by prep-HPLC to obtain compound **HANT-183** (10.4 mg, white solid, yield: 17%). LCMS (ESI): *m*/*z* =419.1 [M+H]⁺; ¹H NMR (400 MHz, CD₃OD) δ 8.39 (d, *J* = 2.2 Hz, 1H), 8.05 (s, 1H), 7.89 (d, *J =* 2.2 Hz, 1H), 7.23 (d, *J* =1.0 Hz, 1H), 4.43 (t, *J =* 6.6 Hz, 1H), 4.33-4.24 (m, 2H), 3.98-3.89 (m, 2H), 3.00 (t, *J =* 6.4 Hz, 2H), 2.87 (s, 3H), 2.27-2.18 (m, 1H), 2.04-1.84 (m, 3H).

### Example 36

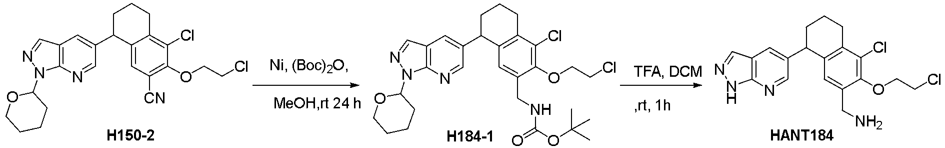

### Step 1: Synthesis of tert-butyl ((4-chloro-3-(2-chloroethoxy)-8-(1-(tetrahydro-2H-pyran-2-yl)-1H-pyrazolo[3,4-b]pyridin-5-yl )-5,6,7,8-tetrahydronaphthalene-2-methyl) carbamate

Compound **H150-2** (100 mg, 0.21 mmol), Ni (50 mg) and (Boc)₂O (93 mg, 0.4 mmol) were dissolved in methanol (3 mL) and stirred at room temperature for 24 h. The reaction solution was filtered and the organic phase was dried and concentrated to obtain the oily compound **H184-1** (100 mg, yield: 82%). LCMS (ESI): *m*/*z=* 575.2 [M+H]⁺.

### Step 2: Synthesis of (4-chloro-3-(2-chloroethoxy)-8-(1-(tetrahydro-2H-pyran-2-yl)-1H-pyrazolo[3,4-b]pyridin-5-yl) -5,6,7,8-tetrahydronaphthalen-2-ylcarboxamide

**H184-1** (30 mg, 0.05 mmol) was dissolved in DCM (3 mL), then TFA (1 mL) was added in and the reaction was stirred at room temperature for 1 h. The reaction solution was concentrated under reduced pressure and the residue was purified by prep-HPLC to obtain a white solid **HANT-184** (14.3 mg, yield: 56%). LCMS (ESI): *m*/*z* =391.1 [M+H]⁺; ¹H NMR (400 MHz, CD₃OD) δ 8.36 (d, *J =* 2.2 Hz, 1H), 8.03 (s, 1H), 7.87 (d, *J =* 2.2 Hz, 1H), 6.89 (s, 1H), 4.42-4.34 (m, 3H), 4.13-4.02 (m, 2H), 4.00-3.91 (m, 2H), 3.00-2.91 (m, 2H), 2.06 - 1.82 (m, 4H).

### Example 37

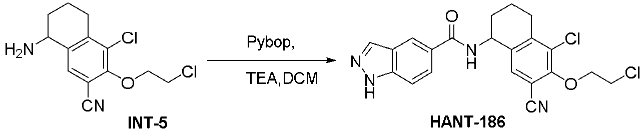

Compound **INT-5** (40 mg, 0.14 mmol), 1H-indazole-5-carboxylic acid (33 mg, 0.16 mmol), PyBOP (110 mg, 0.21 mmol) and TEA (28 mg, 0.28 mmol) were sequentially added to DCM (10 mL) in a dry flask, and the reaction was stirred at room temperature for 12 h. The mixture was filtered, concentrated, and the residue was purified by Prep-HPLC to obtain **HANT-186** (21.3 mg, yield: 36%). LCMS (ESI): *m*/*z* =429.2[M+H]⁺. ¹H NMR (400 MHz, CD₃OD): δ 8.36 (d, *J =* 0.8 Hz, 1H), 8.16 (s, 1H), 7.93-7.90 (m,1H),7.62-7.58 (m, 2H), 5.34 (d, *J =* 3.2 Hz, 1H), 4.42 (t, *J =* 5.6 Hz, 2H), 3.91 (t, *J =* 5.6 Hz, 2H), 2.92-2.91 (m, 2H), 2.11-2.08 (m, 2H), 1.96-1.92 (m, 2H).

### Example 38

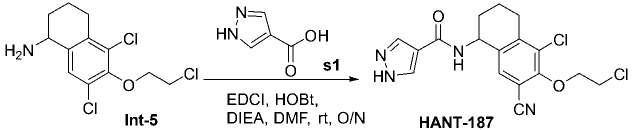

Compound **INT-5** (15 mg, 0.13 mmol), EDCI (40 mg, 0.21 mmol), HOBt (28 mg, 0.18 mmol) and DIEA (54 mg, 0.42 mmol) were sequentially added to DMF (5 mL) in a dry flask and stirred for 30 min at room temperature. Then compound S1 (40 mg, 0.14 mmol) was added and stirred overnight at room temperature. The reaction solution was poured into EA, washed with aq. NH₄Cl. The organic phases were combined and concentrated under reduced pressure. The residue was purified by reversed-phase prep-HPLC to obtain **HANT-187** (37.8 mg, yield: 73%) as a white solid. LCMS (ESI): *m*/*z* =379.1 [M+H]⁺; ¹H NMR (400 MHz, DMSO-*d*₆) δ 13.14 (br s, 1H), 8.37 (d, *J* = 8.3 Hz, 1H), 8.08 (br s, 2H), 7.56 (s, 1H), 5.18-5.14 (m, 1H), 4.40-4.37 (m, 2H), 3.99-3.96 (m, 2H), 2.83-2.80(m, 2H), 2.00-1.77 (m, 4H).

### Example 39

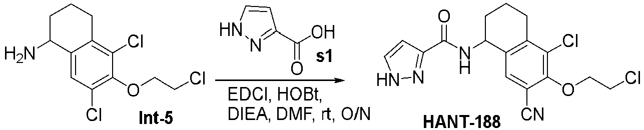

Compound **INT-5** (12 mg, 0.1 mmol), EDCI (29 mg, 0.15 mmol), HOBt (20 mg, 0.15 mmol) and DIEA (39 mg, 0.3 mmol) were sequentially added to DMF (5 mL) in a dry flask, and the reaction was stirred for 30 min at room temperature. Compound S1 (30 mg, 0.1 mmol) was added in and stirred at room temperature overnight. The reaction solution was poured into EA, washed with aq. NH₄Cl. The organic phases were combined and concentrated under reduced pressure. The residue was purified by reversed-phase prep-HPLC to obtain **HANT-188** (29.7 mg, yield: 74%) as a white solid. LCMS (ESI): *m*/*z* =379.1 [M+H]⁺; ¹H NMR (400 MHz, DMSO-*d*₆) δ 13.26 (s, 1H), 8.47 (d, *J =* 8.7 Hz, 1H), 7.84 (s, 1H), 7.51 (s, 1H), 6.71 (s, 1H), 5.19-5.14 (m, 1H), 4.39 (t, *J* = 4.8 Hz 2H), 3.97 (t, *J* = 5.0 Hz.2H), 2.85-2.75 (m, 2H), 2.07-1.67 (m, 4H).

### Example 40

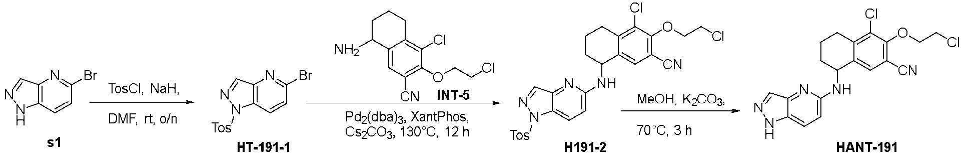

### Step 1: Synthesis of 5-bromo-1-toluenesulfonyl-1H-pyrazolo[4,3-b]pyridine

Compound **S1** (1 g, 5.05 mmol), TosCl (1.38 g, 7.07 mmol) and NaH (262 mg, 6.56 mmol) were added to DMF (30 mL) in a flask, and the reaction was stirred at room temperature for 1 h. The reaction solution was extracted with EA, and dried with Na₂SO₄. The organic phase was concentrated under reduced pressure, and the residue was purified by flash chromatography to afford the yellow oily compound **H191-1** (1.13 g, yield: 64%). LCMS (ESI): *m*/*z* =352.1[M+H]⁺.

### Step 2: Synthesis of 4-chloro-3-(2-chloroethoxy)-8-(1-toluenesulfonyl-1H-pyrazolo[4,3-b]pyridin-5-yl)amino)-5,6,7 ,8-tetrahydronaphthalene-2-carbonitrile

**H191-1** (360 mg, 1.02 mmol), **INT-5** (100 mg, 0.34 mmol), Pd₂(dba)₃ (94 mg, 0.1 mmol), Xantphos (119 mg, 0.2 mmol), and Cs₂CO₃ (223 mg, 0.68 mmol) were dissolved in Tol (5 mL) and reacted for 12 h at 130 °C under N₂. The reaction was cooled to room temperature then extracted with EA. The organic phase was concentrated under reduced pressure, and the residue was purified by flash chromatography to obtain the yellow oily compound **H191-2** (30 mg, yield: 16%). LCMS (ESI): *m*/*z* =556.1[M+H]⁺.

### Step 3: Synthesis of 8-((1H-pyrazolo[4,3-b]pyridin-5-yl)amino)-4-chloro-3-(2-chloroethoxy)-5,6,7,8-tetrahydronaph thalene-2-carbonitrile

Compound **H191-2** (30 mg, 0.31 mmol) and K₂CO₃ were dissolved in MeOH (30 mL) and reacted at 70 °C for 3 h. The reaction solution was extracted with EA and the organic phase was concentrated under reduced pressure. The residue was purified by flash chromatography to obtain the crude white solid **HANT-191** (20 mg, yield: 90%). LCMS (ESI): *m*/*z* =402.1[M+H]⁺. ¹H NMR (400 MHz, CD₃OD) δ 7.81 (s, 1H), 7.70 (d, *J =* 9.2 Hz, 1H), 7.61 (s, 1H), 6.71 (d, *J =* 9.2 Hz, 1H), 5.27 (d, *J* = 6.4 Hz, 1H), 4.40 (t, *J* = 5.6 Hz, 2H), 3.90 (t, *J* = 5.6 Hz, 2H), 2.92-2.88 (m, 2H), 2.07-2.01 (m, 2H), 1.93-1.87 (m, 2H).

### Example 41

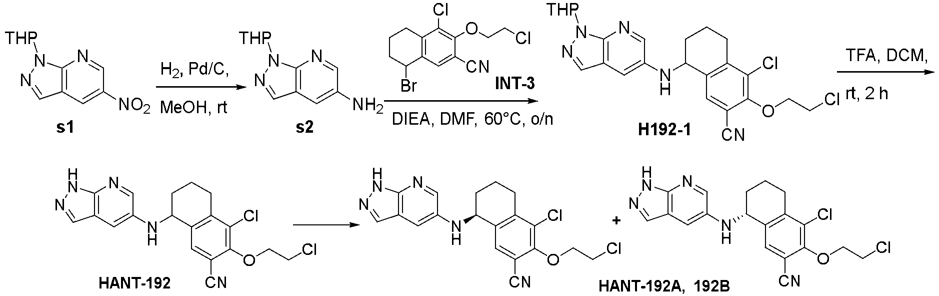

### Step 1: Synthesis of 1-(tetrahydro-2H-pyran-2-yl)-1H-pyrazolo[3,4-b]pyridin-5-amine

Compound **S1** (1.25 g, 5.05 mmol) and Pd/C (625 mg) were dissolved in MeOH (10 mL) and hydrogenated at room temperature for 2 h. The reaction solution was extracted with EA, and the organic phase was dried and concentrated under reduced pressure. The residue was purified by flash chromatography to obtain the yellow oily compound **S2** (1.0 g, yield: 90%). LCMS (ESI): *m*/*z* =219.1 [M+H]⁺.

### Step 2: Synthesis of 4-chloro-3-(2-chloroethoxy)-8-((1-(tetrahydro-2H-pyran-2-yl)-1H-pyrazolo[3,4-b]pyridin-5-yl) amino)-5,6,7,8-tetrahydronaphthalene-2-carbonitrile

Compound **S2** (754 mg, 3.45 mmol), **INT-3** (600 mg, 1.75 mmol) and DIEA (1128 mg, 8.75 mmol) were dissolved in DMF (20 mL) and the reaction was stirred at 60 °C overnight. The reaction was cooled to room temperature and extracted with EA. The organic phase was concentrated under reduced pressure, and the residue was purified by flash chromatography to obtain **H192-1** (530 mg, yield: 16%) as a white solid. LCMS (ESI): *m*/*z* =486.1[M+H]⁺.

### Step 3: Synthesis of 8-((1H-pyrazolo[3,4-b]pyridin-5-yl)amino)-4-chloro-3-(2-chloroethoxy)-5,6,7,8-tetrahydronaph thalene-2-carbonitrile

Compound **H192-1** (530 mg), TFA (1 mL) were dissolved in DCM (30 mL) and the reaction was stirred at room temperature for 2 h. The reaction solution was extracted with EA, and the organic phase was concentrated under reduced pressure. The residue was purified by prep-HPLC to obtain the white solid crude **HANT-192** (51 mg, yield: 23%). LCMS (ESI): *m*/*z* =402.1[M+H]⁺. The racemic **HANT-192** was subjected to SFC chiral separation (mobile phase: Hex-EtOH-30-70-0.2-30 min) to afford two enantiomers HANT-192A (retention time: 6.635 min) and HANT-192B (retention time: 8.971 min).

**HANT-192A:** ¹H NMR (400 MHz, CD₃OD) δ 8.17 (d, *J* = 2.6 Hz, 1H), 7.89 (s, 1H), 7.72 (d, *J =* 1.8 Hz, 1H), 7.39 (d, *J =* 2.6 Hz, 1H), 4.67-4.68 (m, 1H), 4.40-4.43 (m, 2H), 3.90-3.93 (m, 2H), 3.04-2.72 (m, 2H), 2.12-1.77 (m, 4H).

**HANT-192B:** ¹H NMR (400 MHz, CD₃OD) δ 8.17 (d, *J* = 2.6 Hz, 1H), 7.89 (s, 1H), 7.72 (d, *J =* 1.8 Hz, 1H), 7.39 (d, *J =* 2.6 Hz, 1H), 4.67-4.68 (m, 1H), 4.40-4.43 (m, 2H), 3.90-3.93 (m, 2H), 3.04-2.72 (m, 2H), 2.12-1.77 (m, 4H).

### Example 42

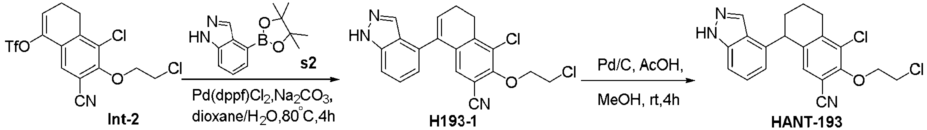

### Step 1: Synthesis of 4-chloro-3-(2-chloroethoxy)-8-(1H-indazol-7-yl)-5,6-dihydronaphthalene-2-carbonitrile

Compound **INT-2** (150 mg, 0.36 mmol), **S2** (132 mg, 0.54 mmol), Pd(dppf)Cl₂ (53 mg, 0.07 mmol) and Na₂CO₃ (77 mg, 0.73 mmol) were dissolved in dioxane/water (3 mL/0.5 mL), and stirred at 80 °C for 4 h. The reaction was cooled to room temperature, then extracted by EA. The organic phase was dried and concentrated, and the residue was purified by flash chromatography to obtain **H193-1** as solid (120 mg, yield: 87%). LCMS (ESI): *m*/*z* =384.1[M+H]⁺.

### Step 2: Synthesis of 4-chloro-3-(2-chloroethoxy)-8-(1H-indazol-7-yl)-5,6,7,8-tetrahydronaphthalene-2-cyanide

Compound **H193-1** (120 mg, 0.31 mmol), Pd/C (50 mg) and AcOH (0.1 mL) were dissolved in MeOH (5 mL) and reacted under H₂ at room temperature for 4 h. The reaction solution was filtered and concentrated under reduced pressure. The residue was purified by prep-HPLC to obtain compound **HANT-193** (21.1 mg, white solid, yield: 18%). LCMS (ESI): *m*/*z* = 386.1 [M+H]⁺; ¹H NMR (400 MHz, DMSO-*d*₆) δ 13.11 (s, 1H), 7.90 (d, *J =* 1.4 Hz, 1H), 7.42 (d, *J =* 1.4 Hz, 1H), 7.42 (d, *JJ* 1.4 Hz, 1H).7.42 (d, *J =* 8.4 Hz, 1H), 7.26 (dd, *J* = 8.4, 7.0 Hz, 1H), 7.13 (d, *J =* 0.8 Hz, 1H), 6.61 (d, *J =* 7.0 Hz, 1H), 4.63 (t, *J =* 6.6 Hz, 1H), 4.45-4.37 (m, 2H), 3.97 (dd, *J* = 6.0,4.4 Hz, 2H), 3.03-2.85 (m, 2H), 2.13-1.98 (m, 2H), 1.84-1.75 (m, 2H).

### Example 43

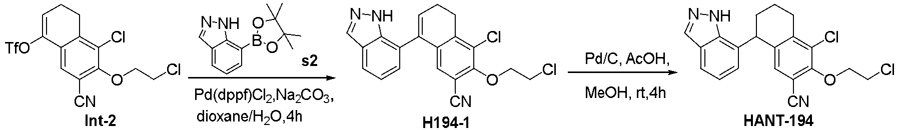

Synthesis was performed using methods described in Example **42** to obtain **HANT-194** (18.7 mg, yield: 19%) as a white solid. LCMS (ESI): *m*/*z* = 386.1 [M+H]⁺; ¹H NMR (400 MHz, CD₃OD) δ 8.08 (s, 1H), 7.68 (dd, *J =* 8.2, 1.0 Hz, 1H), 7.12-7.03 (m, 2H), 6.77 (d, *J =* 7.0 Hz, 1H), 4.65 (t, *J =* 6.2 Hz, 1H), 4.42 (t, *J =* 5.6 Hz, 2H), 3.91 (t, *J =* 5.6 Hz, 2H), 3.11-2.91 (m, 2H), 2.19-2.04 (m, 2H), 1.96-1.84 (m, 2H).

### Example 44

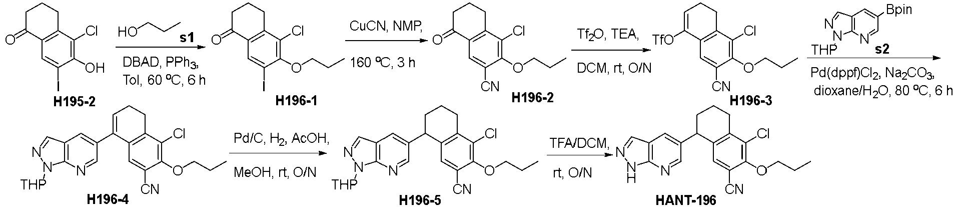

### Step 1: Synthesis of 5-chloro-7-iodo-6-propoxy-3,4-dihydronaphthalen-1(2H)-one

Compounds **H195-2** (2.0 g, 6.2 mmol), **S1** (740 mg, 12.4 mmol), DBAD (2.1 g, 9.3 mmol) and PPh₃ (2.4 g, 9.3 mmol) were sequentially added to toluene (30 mL) in a dry flask and stirred at 60 °C for 6 h. The reaction was cooled to room temperature, and the solvent toluene was removed by concentration under reduced pressure. The resulting crude product was poured into EA, washed three times with saturated brine, and the organic phases were combined and concentrated under reduced pressure. The residue was purified by flash chromatography to obtain the yellow oily compound **H196-1** (850 mg, yield: 38%). LCMS (ESI): *m*/*z* =295.0 [M+H]⁺.

### Step 2: Synthesis of 4-chloro-8-oxo-3-propoxy-5,6,7,8-tetrahydronaphthalene-2-carbonitrile

To a dry sealed tube was sequentially added compounds **H196-1** (850 mg, 2.34 mmol) and CuCN (1 g, 11.7 mmol) in NMP (15 mL), and the reaction was stirred at 160 °C for 3 h. The reaction was cooled to room temperature, then the reaction solution was poured into EA, washed three times with aq. NH₄Cl. The organic phases were combined, concentrated under reduced pressure, and purified by flash chromatography to obtain the yellow solid **H196-2** (260 mg, yield: 43%). LCMS (ESI): *m*/*z* =264.1 [M+H]⁺.

### Step 3: Synthesis of 5-chloro-7-cyano-6-propoxy-3,4-dihydronaphthalen-1-yl trifluoromethane sulfonates

Compound **H196-2** (200 mg, 0.76 mmol) and TEA (230 mg, 2.3 mmol) were dissolved in DCM (10 mL) in a dry flask, followed by addition of Tf₂O (0.7 mL, 3.8 mmol) in an ice bath. The reaction was stirred at room temperature overnight, then poured into saturated brine and extracted with DCM. The organic phases were combined, concentrated under reduced pressure, and purified by flash chromatography to obtain the yellow oily compound **H196-3** (200 mg, yield: 67%). LCMS (ESI): *m*/*z* =396.0 [M+H]⁺.

### Step 4: Synthesis of 4-chloro-3-propoxy-8-(1-(tetrahydro-2H-pyran-2-yl)-1H-pyrazolo[3,4-b]pyridin-5-yl)-5,6-dihy dronaphthalene-2-carbonitrile

Compound **H196-3** (100 mg, 0.25 mmol), **S2** (125 mg, 0.38 mmol), Pd(dppf)Cl₂ (18 mg, 0.025 mmol), and sodium carbonate (54 mg, 0.5 mmol) were sequentially added to dioxane/water (8/2 mL) in a dry flask and stirred for 6h at 80 °C under N₂. The reaction was cooled to room temperature, then poured into saturated brine and extracted with EA. The organic phases were combined, concentrated under reduced pressure, and purified by flash chromatography to obtain white solid **H196-4** (100 mg, yield: 74%). LCMS (ESI): *m*/*z* =449.1 [M+H]⁺.

### Step 5: Synthesis of 4-chloro-3-propoxy-8-(1-(tetrahydro-2H-pyran-2-yl)-1H-pyrazolo[3,4-b]pyridin-5-yl)-5,6-dihy dronaphthalene-2-carbonitrile

To a dry flask was added **H196-4** (90 mg, 0.2 mmol) in methanol (10 mL), followed by Pd/C (9 mg, 10% wt) and AcOH (5 drops). The reaction was stirred under H₂ overnight at room temperature, then filtered, combined and concentrated under reduced pressure. The residue was purified by flash chromatography to obtain **H196-5** (65 mg, yield: 72%) as a white solid. LCMS (ESI): *m*/*z* =451.1 [M+H]⁺.

### Step 6: Synthesis of 4-chloro-3-propoxy-8-(1H-pyrazolo[3,4-b]pyridin-5-yl)-5,6,7,8-tetrahydronaphthalene-2-carbo nitrile

To a dry flask was added compound **H196-5** (65 mg, 0.14 mmol) in DCM (5 mL), then TFA (2 mL) was added in and the reaction was stirred at room temperature overnight. The reaction was concentrated under reduced pressure and the residue was purified by reversed-phase prep-HPLC to obtain **HANT-196** (19.7 mg, yield: 37%) as a white solid. LCMS (ESI): *m*/*z* =367.1 [M+H]⁺; ¹H NMR (400 MHz, DMSO-*d*₆) δ 13.59 (s, 1H), 8.37 (d, *J =* 2.2 Hz, 1H), 8.05(s, 1H), 7.79 (d, *J =* 2.1 Hz, 1H), 7.21 (s, 1H), 4.41-4.37 (m, 1H), 4.09 (t, *J =* 6.5 Hz, 2H), 3.00-2.78 (m, 2H), 2.08-2.05 (m, 1H), 1.91-1.75 (m, 5H), 1.03 (t, *J* = 7.4 Hz, 3H).

### Example 45

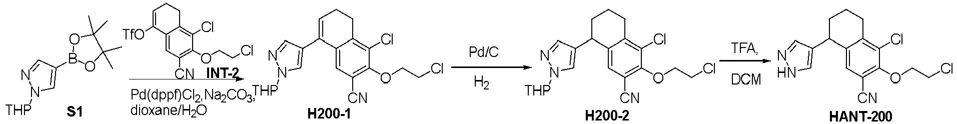

Synthesis was performed using methods described in Example 31 to obtain **HANT-200** (7.5 mg, yield: 31%). LCMS (ESI): *m*/*z* = 336.2 [M+H]⁺. ¹H NMR (400 MHz, CD₃OD) δ 7.35 (s, 2H), 7.26 (s, 1H), 4.39 (t, *J* = 5.6 Hz, 2H), 4.16 (t, 1H), 4.16 (t, 2H).*J* = 5.6 Hz, 1H), 3.91 (t, *J* = 6.0 Hz, 2H), 2.92-2.87 (m, 2H), 2.04-2.03 (m, 1H), 1.93-1.86 (m, 3H).

### Example 46

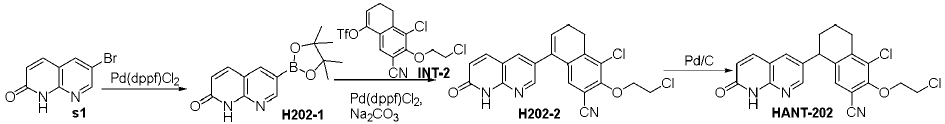

Synthesis was performed using methods described in Example 42 to obtain **HANT-202** (1.2 mg, yield: 1.5%). LCMS(ESI): *m*/*z* =414.2[M+H]⁺. ¹H NMR(400 MHz, CDCl₃) δ 9.95 (s, 1H), 8.37 (d, *J =* 2.0 Hz, 1H), 7.64 (d, *J =* 10 Hz, 1H), 7.47 (d, *J =* 2.0 Hz, 1H), 7.00 (s,1H), 6.73 (d, *J =* 9.6 Hz, 1H), 4.43 (t, *J =* 6.8 Hz, 2H), 4.20 (s, 1H), 3.90 (t, *J =* 6.4 Hz, 2H), 2.96 (d, *J* = 4.0 Hz, 2H), 2.22-2.20 (m, 1H), 1.31-1.26 (m, 3H).

### Example 47

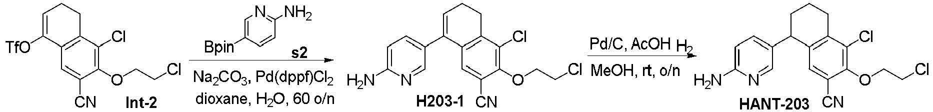

Synthesis was performed using methods described in Example 42 to obtain **HANT-203** (11 mg, yield: 44 %) as a white solid. LCMS (ESI): *m*/*z* = 362.1 [M+H]⁺; ¹H NMR (400 MHz, CD₃OD) δ 7.64 (d, *J =* 2.4 Hz, 1H), 7.18 (dd, *J =* 8.6, 2.5 Hz, 1H), 7.10 (d, *J =* 1.0 Hz, 1H), 6.58 (dd, *J =* 8.6, 0.8 Hz, 1H), 4.41 (t, *J =* 5.0 Hz, 0.8 Hz), 4.41 (t, *J =* 5.0 Hz, 1H)7.10 (d, *J* = 1.0 Hz, 1H), 6.58 (dd, *J =* 8.6, 0.8 Hz, 1H), 4.41 (t, *J =* 5.6 Hz, 2H), 4.01-4.05 (m, 1H), 3.91 (t, *J* = 5.6 Hz, 2H), 2.92-2.95 (m, 2H), 2.12-2.01 (m, 1H),2.01-1.90 (m, 1H), 1.79-1.86 (m, 2H).

### Example 48

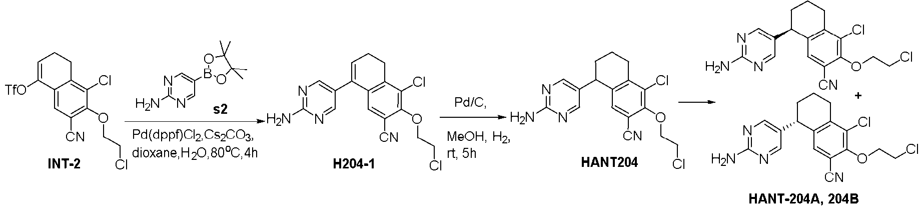

Synthesis was performed using methods described in Example 42 to obtain **HANT-204** (14.2 mg, white solid), yield: 36%. LCMS (ESI): *m*/*z* = 363.1 [M+H]⁺; ¹H NMR (400 MHz, CD₃OD) δ 8.00 (s, 2H), 7.17 (d, *J =* 0.9 Hz, 1H), 4.41 (t, *J* =5.6 Hz, 2H), 4.07 (t, *J =* 6.2 Hz, 1H), 3.91 (t, *J =* 5.6 Hz, 2H), 3.00-2.88 (m, 2H), 2.14-2.02 (m, 1H), 1.97-1.76 (m, 3H).

The racemic **HANT-204** was subjected to SFC chiral separation (mobile phase: Hex-EtOH-DEA-60-40-0.2-30MIN) to afford two enantiomers **HANT-204A** (16.8 mg, RT=8.103min), **HANT-204B** (14.6 mg, RT=10.902min).

**HANT-204A:** ¹H NMR (400 MHz, DMSO-*d*₆) δ 7.96 (s, 2H), 7.28 (d, *J =* 0.9 Hz, 1H), 6.52 (s, 2H), 4.46-4.33 (m, 2H), 4.07-3.91 (m, 3H), 2.93-2.76 (m, 2H), 2.03-1.88 (m, 1H), 1.86-1.72 (m, 3H).

**HANT-204B:** ¹H NMR (400 MHz, DMSO-*d*₆) δ 7.96 (s, 2H), 7.29 (s, 1H), 6.55 (s, 2H), 4.46-4.31 (m, 2H), 4.06-3.92 (m, 3H), 2.97-2.77 (m, 2H), 2.03-1.89 (m, 1H), 1.88-1.66 (m, 3H).

### Example 49

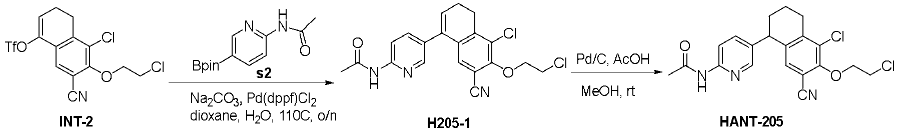

Synthesis was performed using methods described in Example **42** to obtain **HANT-205** (13.9 mg, yield: 9%) as a white solid. LCMS (ESI): *m*/*z* = 404.1 [M+H]⁺; ¹H NMR (400 MHz, CD₃OD) δ 8.08-7.97 (m, 2H), 7.44-7.47 (m, 1H), 7.09 (s, 1H), 4.41 (t, *J =* 5.5 Hz, 2H), 4.20 (t, *J* = 6.3 Hz, 1H), 3.91 (t, *J =* 5.6 Hz, 2H), 2.96 (t, *J =* 6.1 Hz, 2H), 2.16 (s, 3H), 2.11-2.13 (m, 1H), 1.84-1.94 (m, 3H).

### Example 50

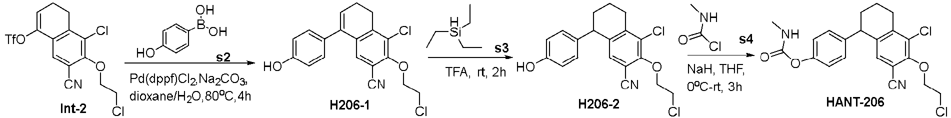

### Step 1: Synthesis of 4-chloro-3-(2-chloroethoxy)-8-(4-hydroxyphenyl)-5,6-dihydronaphthalene-2-carbonitrile

Compound **INT-2** (200 mg, 0.5 mmol), **S2** (100 mg, 0.72 mmol), Pd(dppf)Cl₂ (70 mg, 0.1 mmol), and Na₂CO₃ (102 mg, 1.0 mmol) were dissolved in dioxane/water (10 mL/2 mL) and stirred at 80 °C for 4 h. The reaction was cooled to room temperature, extracted with EA. The organic phase was dried and concentrated, and the residue was purified by flash chromatography to obtain **H206-1** as solid (130 mg, yield: 75%). LCMS (ESI): *m*/*z* = 360.0 [M+H]⁺.

### Step 2: Synthesis of 4-chloro-3-(2-chloroethoxy)-8-(4-hydroxyphenyl)-5,6,7,8-tetrahydronaphthalene-2-carbonitrile

Compound **H206-1** (130 mg, 0.36 mmol) and **S3** (84 mg, 0.72 mmol) were dissolved in TFA (5 mL) and reacted under N₂ at room temperature for 2 h. The reaction solution was concentrated under reduced pressure to obtain compound **H206-2** (100 mg, yellow oil, yield: 77%). LCMS (ESI): *m*/*z* =362.1 [M+H]⁺.

### Step 3: Synthesis of 4-(5-chloro-6-(2-chloroethoxy)-7-cyano-1,2,3,4-tetrahydronaphthalen-1-yl)phenyl methylcarbamate

**H206-2** (100 mg, 0.2 8 mmol) was dissolved in THF (5 mL), and NaH (22.2 mg, 0.56 mmol) was added at 0 °C and stirred for 1 h. Then **S4** (39 mg, 0.42 mmol) was added, and the reaction was stirred at room temperature for 3 h. The reaction was extracted with DCM, and the organic phase was dried and concentrated. The residue was purified by prep-HPLC to obtain the solid **HANT-206** (15.0 mg, yield: 13%). LCMS (ESI): *m*/*z* = 419.1[M+H]⁺; ¹H NMR (400 MHz, DMSO-*d*₆) δ 7.59 (d, *J* = 4.8 Hz, 1H), 7.17 (s, 1H), 7.09-6.99 (m, 4H), 4.40 (t, *J =* 5.2)Hz, 2H), 4.21 (t, *J =* 6.2 Hz, 1H), 3.97 (t, *J =* 5.2 Hz, 2H), 2.95-2.79 (m, 2H), 2.65 (d, *J =* 4.6 Hz, 3H), 2.14-1.96 (m, 1H), 1.88-1.70 (m, 3H).

### Example 51

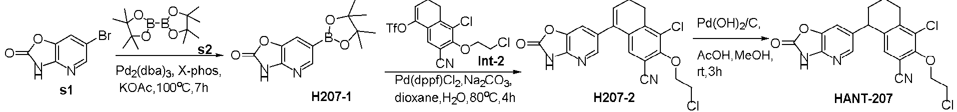

### Step 1: Synthesis of 6-(4,4,5,5-tetramethyl-1,3,2-dioxaborolan-2-yl)oxazolo[4,5-b]pyridin-2(3H)-one

Compounds **S1** (500 mg, 2.3 mmol), **S2** (890 mg, 3.5 mmol), Pd₂(dba)₃ (211 mg, 0.23 mmol), Xphos (219 mg, 0.46 mmol), and potassium acetate (676 mg, 6.9 mmol) were dissolved in Tol (15 mL), and stirred under N₂ at 100 °C for 7 h. The reaction was cooled to room temperature, extracted with EA, and the organic phase was concentrated under reduced pressure. The residue was purified by flash chromatography to obtain compound **H207-1** (100 mg, white solid, yield: 16%). LCMS (ESI): *m*/*z* =263.1[M+H]⁺.

### Step 2: Synthesis of 4-chloro-3-(2-chloroethoxy)-8-(2-oxo-2,3-dihydrooxazolo[4,5-b]pyridin-6-yl)-5,6-dihydronaph thalene-2-carbonitrile

Compounds **H207-1** (100 mg, 0.38 mmol), **INT-2** (237 mg, 0.57 mmol), Pd(dppf)Cl₂ (56 mg, 0.08 mmol), and Na₂CO₃ (81 mg, 0.8 mmol) were dissolved in dioxane/water (3 mL/0.5 mL), and the reaction solution was stirred under N₂ at 80 °C for 4 h. The reaction was cooled to room temperature, extracted with EA, and the organic phase was dried and concentrated. The residue was purified by flash chromatography to obtain **H207-2** as solid (50 mg, yield: 33%). LCMS (ESI): *m*/*z* =402.0[M+H]⁺.

### Step 3: Synthesis of 4-chloro-3-(2-chloroethoxy)-8-(2-oxo-2,3-dihydrooxazolo[4,5-b]pyridin-6-yl)-5,6,7,8-tetrahydr onaphthalene-2-carbonitrile

Compound **H207-2** (30 mg, 0.07 mmol), Pd(OH)₂/C (20 mg) and AcOH (20 mg) were dissolved in MeOH (3 mL) and reacted under H₂ at room temperature for 3 h. The reaction solution was filtered, concentrated under reduced pressure, and the residue was purified by prep-HPLC to obtain the compound **HANT-207** (2.5 mg, white solid, yield: 8%). LCMS (ESI): *m*/*z* = 404.0 [M+H]⁺; ¹H NMR (400 MHz, CD₃OD) δ 7.82 (s, 1H), 7.26 (s, 1H), 7.11 (s, 1H), 4.41 (t, *J =* 5.6 Hz, 2H), 4.25 (t, *J =* 6.4 Hz, 1H), 3.91 (t, *J* =5.6 Hz, 2H), 2.97 (t, *J =* 6.2 Hz, 2H), 2.23-2.11 (m, 1H), 1.99-1.81 (m, 3H).

### Example 52

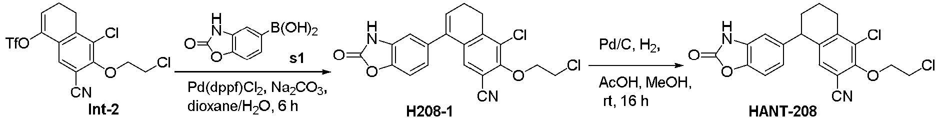

Synthesis was performed using methods described in Example 27 to obtain **HANT-208** (24.4 mg, yield: 33%) as a white solid. LCMS (ESI): *m*/*z* =403.0 [M+H]⁺; ¹H NMR (400 MHz, DMSO-*d*₆) δ 7.22-7.19 (m, 1H), 7.15 (s, 1H), 6.79-6.76 (m, 2H).), 4.41-4.38 (m, 2H), 4.21-4.19 (m, 1H), 3.97 (dd, *J =* 6.2, 4.0 Hz, 2H), 2.88 (br s, 2H), 2.04-1.98 (m, 1H), 1.85-1.72 (m, 3H).

### Example 53

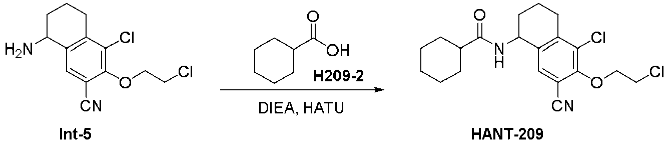

Compounds **INT-5** (30 mg, 0.103 mmol), **H209-2** (16 mg, 0.124 mmol), and HATU (51 mg, 0.134 mmol) were dissolved in DMF (5 mL) in a dry flask, then DIEA (40 mg, 0.31 mmol) was added in. The reaction solution was stirred at room temperature overnight, then extracted with water and EA. The organic phase was dried with anhydrous Na₂SO₄, filtered, and concentrated under reduced pressure to obtain the crude product **HANT-209** (13.7 mg, white solid, yield: 32.9%). LCMS (ESI): *m*/*z* = 395.1 [M+H]⁺. ¹H NMR (400 MHz, DMSO-*d*₆) δ 8.11 (d, *J* = 8.4 Hz, 1H), 7.46 (s, 1H), 4.92 (dd, *J =* 12.8, 8.0 Hz, 1H), 4.43-4.34 (m, 2H), 4.01-3.93 (m, 2H), 2.77 (t, *J* = 6.2 Hz, 2H), 2.15 (m, 1H), 1.86-1.58 (m, 8H), 1.45-1.13 (m, 6H).

### Example 54

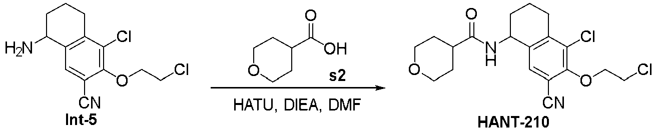

In a dry flask, compounds **INT-5** (30 mg, 0.105 mmol), **S2** (15 mg, 0.115 mmol), and HATU (51 mg, 0.134 mmol) were sequentially dissolved in DMF (5 mL), followed by addition of DIEA (0.054 mL, 0.31 mmol). The reaction solution was stirred at room temperature overnight, then extracted with water and EA. The organic phase was dried with anhydrous Na₂SO₄, filtered, and concentrated under reduced pressure. The crude product was purified by by Prep-HPLC (NH₄HCO₃) to afford the compound **HANT-210** (14.8 mg, white solid, yield: 47%). LCMS (ESI): *m*/*z* = 397.2 [M+H]⁺; ¹H NMR (400 MHz, DMSO-*d*₆) δ 8.20 (d, *J =* 8.4 Hz, 1H), 7.50 (s1H), 4.96-4.91 (m, 1H), 4.38 (t, *J* = 4.8 Hz, 2H), 3.97 (t, *J =* 5.2 Hz, 2H), 3.89-3.85 (m, 2H), 3.29 (s, 1H), 3.27 (d, *J* = 9.6 Hz, 1H), 2.77 (t, *J =* 6.4 Hz, 2H), 2.45-2.37 (m, 1H), 1.91-1.88 (m, 1H), 1.84-1.76 (m, 2H), 1.70-1.58 (m, 5H).

### Example 55

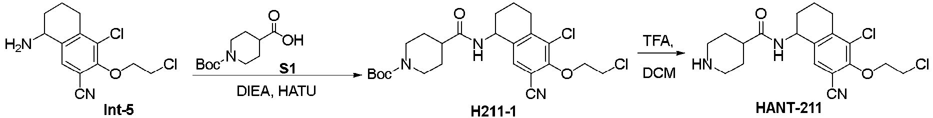

### Step 1: Synthesis of tert-butyl 4-((5-chloro-6-(2-chloroethoxy)-7-cyano-1,2,3,4-tetrahydronaphthalen-1-yl)carbamoyl)piperidi ne-1-carboxylate

In a dry flask, compounds **INT-5** (42 mg, 0.18 mmol), HATU (70 mg, 0.18 mmol), and **S1** (40 mg, 0.14 mmol) were sequentially dissolved in DMF (5 mL), followed by addition of DIEA (55 mg, 0.43 mmol). The reaction solution was stirred at 100 °C overnight. The reaction was cooled to room temperature, quenched with saturated aq. NH₄Cl and extracted with EA. The organic phase was dried with anhydrous Na₂SO₄ and filtered. The filtrate was concentrated under reduced pressure to obtain the crude product **H211-1** (50 mg, yellow oily material, yield: 64%). LCMS (ESI): *m*/*z* = 440.1 [M-56+H]⁺.

### Step 2: Synthesis of 4-((5-chloro-6-(2-chloroethoxy)-7-cyano-1,2,3,4-tetrahydronaphthalen-1-yl)carbamoyl)piperidi ne

Compound **H211-1** (45 mg, 0.09 mmol) was dissolved in DCM (10 mL) in a dry flask then trifluoroacetic acid (1.5 mL) was added and stirred at room temperature for 3 h. The crude product was concentrated under reduced pressure, and the residue was purified by prep-HPLC (NH₄HCO₃) to obtain **HANT-211** (23.1 mg, white solid, yield: 63%). LCMS (ESI): *m*/*z* = 396.1 [M+H]⁺. ¹H NMR (400 MHz, DMSO-*d*₆) δ 8.52 (s, 1H), 7.48 (s, 1H), 5.07-5.04 (m, 1H), 4.41 (t, *J* = 5.6 Hz, 2H), 3.90 (t, *J =* 6.0 Hz, 2H), 3.49-3.42 (m, 2H), 3.04-2.97 (m, 2H), 2.89-2.85 (m, 2H), 2.60-2.52 (m, 1H), 2.07-1.84 (m, 8H),1.84-1.75 (m, 1H).

### Example 56

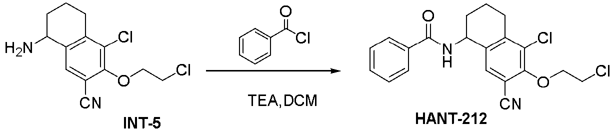

Compound **INT-5** (20 mg, 0.07 mmol), benzoyl chloride (12 mg, 0.09 mmol) triethylamine (14 mg, 0.14 mmol), and DCM (3 mL) were stirred at room temperature for 2 h. The reaction was concentrated and purified by prep-HPLC to obtain **HANT-212** (15.1 mg, Yield: 56%). LCMS (ESI): *m*/*z* = 389.2 [M+H]⁺. ¹H NMR (400 MHz, CD₃OD) δ 7-86-7.84 (m, 2H), 7.57-7.53 (m, 2H), 7.49-7.45 (m,2H), 5.31 (d, *J =* 4.0 Hz, 1H), 4.41 (t, *J =* 4.2 Hz, 2H), 3.90 (t, *J* =4.2 Hz, 2H), 2.92-2.90 (m, 2H), 2.09-2.05 (m, 2H), 1.94-1.89 (m, 2H).

### Example 57

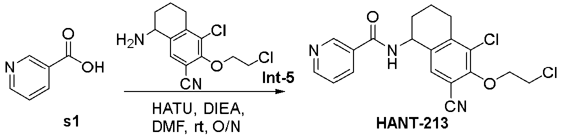

Compound **S1** (30 mg, 0.25 mmol) and HATU (145 mg, 0.38 mmol) were dissolved in DMF (5 mL) and stirred for ten minutes, then compound **INT-5** (70 mg, 0.25 mmol) and DIEA (96 mg, 0.75 mmol) were added in. The reaction was stirred at room temperature overnight. The reaction solution was poured into EA and washed with saturated aq. NH₄Cl. The resulting organic phase was dried with anhydrous Na₂SO₄ and concentrated under reduced pressure, and the resulting residue was purified by reversed-phase prep-HPLC to obtain **HANT-213** (42.6 mg, yield: 47%) as a white solid. LCMS (ESI): *m*/*z* =390.1 [M+H]⁺; ¹H NMR (400 MHz, DMSO-*d*₆) δ 9.04 (dd, *J* = 2.3, 0.9 Hz, 1H), 8.97 (d, *J =* 8.0 Hz, 1H), 8.71 (dd, *J =* 4.8, 1.7 Hz, 1H), 8.23 (dt, *J =* 8.0, 1.9 Hz, 1H), 7.70 (s, 1H), 7.51 (ddd, *J =* 7.9, 4.8, 0.9 Hz.1H), 5.24-5.21 (m, 1H), 4.40 (t, *J* = 5.0 Hz, 2H), 3.98 (t, *J* = 5.0 Hz, 2H), 2.94-2.76 (m, 2H), 2.04-1.91 (m, 2H), 1.91-1.79 (m, 2H).

### Example 58

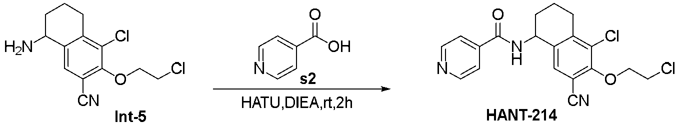

Compounds **INT-5** (30 mg, 0.11 mmol), **S2** (13 mg, 0.11 mmol), HATU (60 mg, 0.16 mmol) and DIEA (20.5 mg, 0.16 mmol) were dissolved in DMF (3 mL) and stirred at room temperature for 2 h. The reaction solution was extracted with EA and the organic phase was dried and concentrated. The residue was purified by prep-HPLC to afford **HANT-214** as a solid (19.8 mg, yield: 48%). LCMS (ESI): *m*/*z* = 390.1[M+H]⁺; ¹H NMR (400 MHz, DMSO-*d*₆) δ 9.07 (d, *J =* 8.2 Hz, 1H), 8.73 (d, *J* = 5.2 Hz, 2H), 7.84-7.77 (m, 2H), 7.68 (d, *J =* 0.8 Hz, 1H), 5.24-5.15 (m, 1H), 4.43-4.35 (m, 2H), 4.01-3.95 (m, 2H), 2.86-2.79 (m, 2H), 2.03-1.80 (m, 4H).

### Example 59

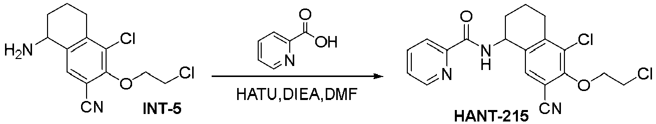

Compound **INT-5** (20 mg, 0.07 mmol), pyridine carboxylic acid (11 mg, 0.09 mmol), HATU (35 mg, 0.09 mmol) and DIEA (23 mg, 0.18 mmol) were dissolved in DMF (5 mL) and stirred at room temperature for 12 h. The reaction was extracted with EA and washed with saturated NH₄Cl. The organic phase was dried, concentrated and purified by prep-HPLC to obtain **HANT-215** (10.1 mg, yield: 37%). LCMS (ESI): *m*/*z* = 390.2[M+H]⁺.¹H NMR (400 MHz, CD₃OD) δ 8.62 (d, *J =* 4.0 Hz, 1H), 8.15 (d, *J* = 4.0 Hz, 1H), 8.01-7.97 (m,1H),7.58-7.53 (m, 2H), 5.30 (d, *J =* 3.6 Hz, 1H), 4.41 (t, *J =* 5.6 Hz, 2H), 3.90 (t, *J =* 5.6 Hz, 2H), 2.94-2.91 (m, 2H), 2.10-2.06 (m, 2H), 1.97-1.92 (m, 2H).

### Example 60

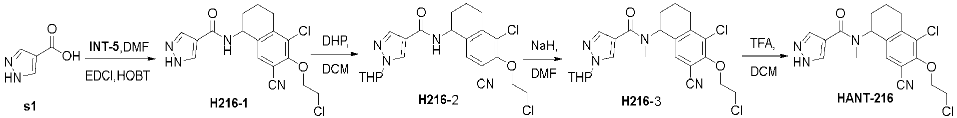

### Step 1: Synthesis of N-(5-chloro-6-(2-chloroethoxy)-7-cyano-1,2,3,4-tetrahydronaphthalen-1-yl)-1H-pyrazole-4-car boxamide

Compound **S1** (67 mg, 0.6 mmol), EDCI (171 mg, 0.9 mmol), HOBT (121 mg, 0.9 mmol) and DIEA (230 mg, 1.8 mmol) were added to DMF (5 mL) and stirred at room temperature for 0.5 h. **INT-5** (170 mg, 0.6 mmol) was added to the reaction, and stirred at room temperature for 2 h. The reaction was then extracted with EA, washed with saturated NH₄Cl, and the organic phase was dried and concentrated to obtain **H216-1** (160 mg, yield: 71%). LCMS (ESI): *m*/*z* =379.2[M+H]⁺.

### Step 2. Synthesis of N-(5-chloro-6-(2-chloroethoxy)-7-cyano-1,2,3,4-tetrahydronaphthalen-1-yl)-1-(tetrahydro-2H-p yran-2-yl)-1H-pyrazole-4-carboxamide

Compound **H216-1** (160 mg, 0.4 mmol), DHP (71 mg, 0.8 mmol) and PTSA (40 mg, 0.2 mmol) were dissolved in DCM (5 mL) and stirred at room temperature for 12 h. The organic phase was concentrated, and the residue was purified by flash chromatography (PE:EA=2:1) to obtain **H216-2** (160 mg, yield: 71%). LCMS (ESI): *m*/*z* = 463.2 [M+H]⁺.

### Step 3. Synthesis of N-(5-chloro-6-(2-chloroethoxy)-7-cyano-1,2,3,4-tetrahydronaphthalen-1-yl)-N-methyl-1-(tetrah ydro-2H-pyran-2-yl)-1H-pyrazole-4-carboxamide

Compound **H216-2** (100 mg, 0.22 mmol) was dissolved in DMF (5 mL), then NaH (17 mg, 0.43 mmol) was added in and stirred for 0.5 h at 0 °C. Iodomethane (46 mg, 0.32 mmol) was added to the reaction and stirred at room temperature for 2 h. The reaction was extracted with EA, washed with saturated NH₄Cl, and the organic phase was dried and concentrated. The residue was purified by flash chromatography (PE:EA=1:1) to obtain **H216-3** (40 mg, yield: 39%). LCMS (ESI): *m*/*z* =477.2[M+H]⁺.

### Step 4. Synthesis of N-(5-chloro-6-(2-chloroethoxy)-7-cyano-1,2,3,4-tetrahydronaphthalen-1-yl)-N-methyl-1H-pyra zole-4-carboxamide

Compound **H216-3** (40 mg, 0.08 mmol) was added to DCM (5 mL) at 0 °C followed by TFA (0.5 mL). The reaction was stirred at room temperature for 3 h then concentrated. The residue was purified by prep-HPLC to obtain **HANT-216** (18 mg, yield: 55%). LCMS (ESI): *m*/*z* = 393.2 [M+H]⁺; ¹H NMR (400 MHz, CD₃OD) δ 8.01 (d, *J =* 63.5 Hz, 2H), 7.38 (d, *J =* 29.2 Hz, 1H), 5.59 (d, *J =* 201.0 Hz, 1H), 4.42 (t, *J =* 5.6 Hz, 2H), 3.89 (dd, *J =* 22.4, 16.8 Hz, 2H), 3.10-2.90 (m, 3H), 2.76 (d, *J =* 11.4 Hz, 2H), 2.23-1.81 (m, 4H).

### Example 61

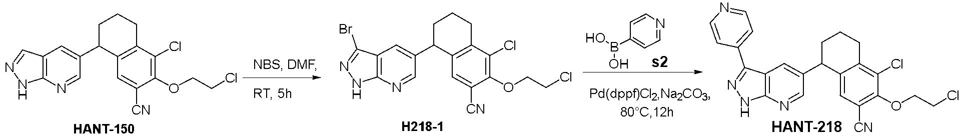

### Step 1: Synthesis of 8-(3-bromo-1H-pyrazolo[3,4-b]pyridin-5-yl)-4-chloro-3-(2-chloroethoxy)-5,6,7,8-tetrahydrona phthalene-2-carbonitrile

**HANT-150** (180 mg, 0.47 mmol), NBS (100 mg, 0.56 mmol), DMF (3 mL) were sequentially added to a 10 mL flask and stirred under N₂ at room temperature for 5 h. The reaction was extracted with EA. The organic phase was dried with anhydrous Na₂SO₄, filtered, and concentrated under reduced pressure to obtain compound **H218-1** (150 mg, yield:69%). LCMS (ESI): *m*/*z* =465.0 [M+H]⁺.

### Step 2: Synthesis of 4-chloro-3-(2-chloroethoxy)-8-(3-(pyridin-4-yl)-1H-pyrazolo[3,4-b]pyridin-5-yl)-5,6,7,8-tetrah ydronaphthalene-2-carbonitrile

**H218-1** (50 mg, 0.1 mmol), **S2** (133 mg, 1.08 mmol), Pd(dppf)Cl₂ (27.6 mg, 0.038 mmol) and sodium carbonate (40 mg, 0.38 mmol), dioxane, and water (3 mL, 5 :1) were added sequentially to a 10 mL three-necked flask, and the reaction was stirred at 80 °C under N₂ for 12 h. The reaction was cooled to room temperature and extracted with aq. NH₄Cl and EA. The organic phase was dried with anhydrous Na₂SO₄, filtered, and concentrated under reduced pressure. The residue was purified by prep-HPLC to obtain compound **HANT-218** (3.5 mg, white solid, yield: 7.0%). LCMS (ESI): *m*/*z* =464.1 [M+H]⁺; ¹H NMR (400 MHz, CD₃OD) δ 8.67 (d, *J =* 6.2 Hz, 2H), 8.41 (d, *J =* 2.0 Hz, 1H), 8.38 (d, *J =* 2.0 Hz, 1H), 8.08 (dd, *J =* 4.8, 1.6 Hz, 2H), 7.14 (s, 1H), 4.51-4.41 (m, 3H), 3.95 (t, *J =* 5.6 Hz, 2H), 3.07 (d, *J =* 9.6 Hz, 2H), 2.35-2.01 (m, 4H).

### Example 62

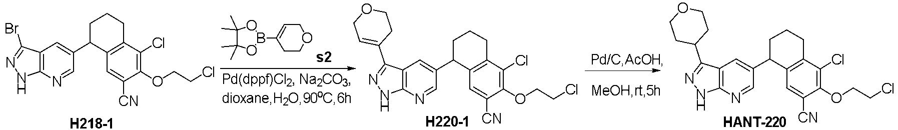

### Step 1: Synthesis of 4-chloro-3-(2-chloroethoxy)-8-(3-(3,6-dihydro-2H-pyran-4-yl)-1H-pyrazolo[3,4-b]pyridin-5-yl) -5,6,7,8-tetrahydronaphthalene-2-cyanide

Compound **H218-1** (100 mg, 0.1 mmol), **S2** (91 mg, 0.2 mmol), Pd(dppf)Cl₂ (32 mg, 0.04 mmol), and Na₂CO₃ (46 mg, 0.4 mmol) were added to dioxane/water (5 mL/1 mL), and stirred under N₂ at 90 °C for 6 h. The reaction was cooled to room temperature and extracted with EA. The organic phase was dried and concentrated, and the residue was purified by flash chromatography to obtain **H220-1** as solid (50 mg, yield: 50%). LCMS (ESI): *m*/*z =* 469.1[M+H]⁺.

### Step 2: Synthesis of 4-chloro-3-(2-chloroethoxy)-8-(3-(tetrahydro-2H-pyran-4-yl)-1H-pyrazolo[3,4-b]pyridin-5-yl)-5,6,7,8-tetrahydronaphthalene-2-cyanide

Compound **H220-1** (20 mg, 0.04 mmol), Pd/C (20 mg) and AcOH (0.1 mL) were dissolved in MeOH (3 mL) and reacted under H₂ at room temperature for 5 h. The reaction solution was filtered, concentrated under reduced pressure and the residue was purified by prep-HPLC to obtain compound **HANT-220** (4.8 mg, white solid, yield: 24%) LCMS (ESI): *m*/*z* = 471.1 [M+H]⁺; ¹H NMR (400 MHz, CD₃OD) δ 8.27 (s, 1H), 8.00 (d, *J =* 2.0 Hz, 1H), 7.08 (s, 1H), 4.43 (t, *J =* 5.6 Hz, 2H), 4.38 (t, *J =* 6.8 Hz, 1H), 4.08-4.01 (m, 2H), 3.91 (t, *J =* 5.6 Hz, 2H), 3.66-3.56 (m, 2H), 3.01 (t, *J =* 6.4 Hz, 2H), 2.23-2.16 (m, 1H), 2.06-1.87 (m, 8H).

### Example 63

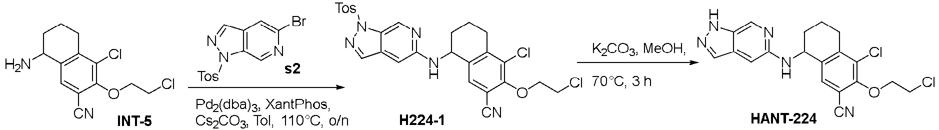

### Step 1: Synthesis of 4-chloro-3-(2-chloroethoxy)-8-(1-toluenesulfonyl-1H-pyrazolo[3,4-c]pyridin-5-yl)amino)-5,6,7 ,8-tetrahydronaphthalene-2-carbonitrile

**INT-5** (50 mg, 0.18 mmol), **S2** (93 mg, 0.26 mmol), Pd₂(dba)₃ (48 mg, 0.05 mmol), Xantphos (61 mg, 0.105 mmol), and Cs₂CO₃ (58 mg, 0.36 mmol) were dissolved in toluene (3 mL) and reacted overnight in a sealed tube under N₂ at 110 °C. The reaction solution was extracted with EA, and the organic phase was dried and concentrated under reduced pressure. The residue was purified by flash chromatography to obtain **H224-1** (80 mg, yield: 78%) as a yellow solid. LCMS (ESI): *m*/*z* =556.1[M+H]⁺.

### Step 2: Synthesis of 8-(1H-pyrazolo[3,4-c]pyridin-5-yl)amino)-4-chloro-3-(2-chloroethoxy)-5,6,7,8-tetrahydronapht halene-2-carbonitrile

Compound **H224-1** (80 mg, 0.14 mmol), K₂CO₃ (96 mg, 0.7 mmol) were dissolved in methanol (3 mL) and reacted under N₂ at 70 °C for 3 h. The reaction was cooled to room temperature, extracted with EA, and the organic phase was concentrated under reduced pressure. The residue was purified by flash chromatography to obtain the yellow oily compound **HANT-224** (13.7 mg, yield: 23%). LCMS (ESI): *m*/*z* =402.1[M+H]⁺; ¹H NMR (400 MHz, CD₃OD) δ 8.60 (s, 1H), 7.89 (s, 1H), 7.69 (s, 1H), 6.82 (s, 1H), 5.04-5.05 (m, 1H), 4.40 (t, *J =* 5.6 Hz, 2H), 3.91(t, *J =* 5.6 Hz, 2H), 3.01-2.69 (m, 2H), 2.01-2.05 (m, 2H), 1.97-1.80 (m, 2H).

### Example 64

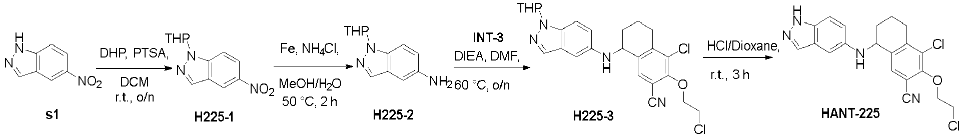

### Step 1: Synthesis of 5-nitro-1-(tetrahydro-2H-pyran-2-yl)-1H indazole

**S1** (1.0 g, 6.1 mmol), DCM (20 mL), DHP (1.03 g, 12.3 mmol), and PTSA (582 mg, 3.07 mmol) were sequentially added into a dry flask and stirred overnight at room temperature under N₂. The reaction mixture was concentrated under reduced pressure, and the residue was purified by flash chromatography to obtain **H225-1** (1.2 g, yield: 80%) as an orange solid. LCMS (ESI): *m*/*z* =248.1 [M+H]⁺.

### Step 2: Synthesis of 1-(tetrahydro-2H-pyran-2-yl)-1H-indazol-5-amine

To a dry flask was added compound **H225-1** (500 mg, 2.0 mmol), methanol/water (20 mL), iron powder (680 mg, 12.0 mmol), NH₄Cl (642 mg, 12.0 mmol) sequentially, and stirred at 50°C for 2 h. The reaction was cooled to room temperature, filtered and concentrated under reduced pressure. The residue was diluted with water, extracted by EA, and the organic phase was dried with Na₂SO₄, filtered and concentrated to obtain the orange solid **H225-2** (430 mg, yield: 99%). LCMS (ESI): *m*/*z* =218.1 [M+H]⁺.

### Step 3: Synthesis of 4-chloro-3-(2-chloroethoxy)-8-((1-(tetrahydro-2H-pyran-2-yl)-1H-indazol-5-yl)amino)-5,6,7,8-tetrahydronaphthalene-2-carbonitrile

Compound **H225-2** (54 mg, 0.25 mmol), DMF (4 mL), **INT-3** (175 mg, 0.5 mmol), and DIEA (97 mg, 0.75 mmol) were added sequentially into a dry flask and stirred overnight at 60 °C under N₂. The reaction was cooled to room temperature, diluted with water and extracted with EA. The organic phase was dried with Na₂SO₄, filtered and concentrated. The residue was purified by flash chromatography to obtain a yellow solid (100 mg, yield: 82%). LCMS (ESI): *m*/*z* =485.1 [M+H]⁺.

### Step 4: Synthesis of 8-((1H-indazol-5-yl)amino)-4-chloro-3-(2-chloroethoxy)-5,6,7,8-tetrahydronaphthalene-2-carb onitrile

**H225-3** (90 mg, 0.19 mmol) and HCl in dioxane (4 M, 7 mL) were sequentially added to a dry flask and stirred at room temperature for 3 h. The reaction was concentrated under reduced pressure, then pH was adjusted to alkaline with saturated sodium bicarbonate solution. The reaction mixture was extracted by DCM, and the organic phase was dried with Na₂SO₄, filtered, and concentrated. The residue was purified by prep-HPLC to obtain a white solid **HANT-225** (20.1 mg, yield: 27%). LCMS (ESI): *m*/*z* =401.1 [M+H]⁺; ¹H NMR (400 MHz, CD₃OD) δ 7.80 (s, 1H), 7.71 (s, 1H), 7.36 (d, *J* = 9.0 Hz, 1H), 6.98 (dd, *J* = 9.0, 2.2 Hz, 1H), 6.93(s, 1H), 4.66-4.63 (m, 1H), 4.41 (t, *J* = 6.0 Hz, 2H), 3.91 (t, *J* = 6.0 Hz, 2H), 3.00-2.92 (m, 1H), 2.86-2.78 (m, 1H), 2.07-1.79 (m, 4H).

### Example 65

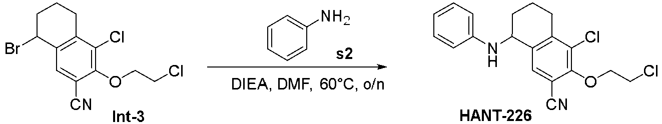

**INT-3** (50 mg, 0.14 mmol), **S2** (27 mg, 0.27 mmol), DIEA (93 mg, 0.72 mmol ) were dissolved in DMF (2 mL) and reacted at 60 °C under N₂ overnight. The reaction was cooled to room temperature and extracted with EA. The organic phase was dried and concentrated under reduced pressure, and the residue was purified by flash chromatography to obtain **HANT-226** (9.0 mg, yield: 17%) as a white solid. LCMS (ESI): *m*/*z* = 362.1[M+H]⁺; ¹H NMR (400 MHz, CD₃OD) δ 7.64 (d, *J =* 0.8 Hz, 1H),7.16-7.03 (m, 2H), 6.73-6.66 (m, 2H), 6.61-6.65 (m, 1H), 4.60-4.63 (m, 1H), 4.39 (t, *J* = 5.6 Hz, 2H), 3.90 (t, *J* = 5.6 Hz, 2H), 2.99-2.73 (m, 2H), 1.93-1.99 (m,2H), 1.91-1.77 (m, 2H).

### Example 66

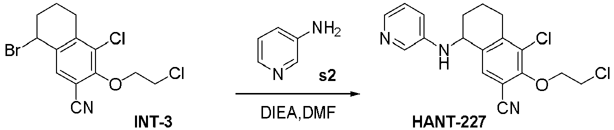

Synthesis was performed using methods described in Example 65 to afford the compound **HANT-227** (11.9 mg, yield: 10%). LCMS (ESI): *m*/*z* = 362.2 [M+H]⁺. ¹H NMR (400 MHz, CD₃OD) δ 8.00 (s, 1H), 7.80 (s, 1H), 7.64 (s, 1H), 7.21-7.13 (m, 2H), 4.69 (s, 1H), 4.41 (t, *J=* 5.6 Hz, 2H), 3.91 (t, *J =* 5.6 Hz, 2H), 2.98-2.80 (m, 2H), 2.03-1.88 (m, 4H).

### Example 67

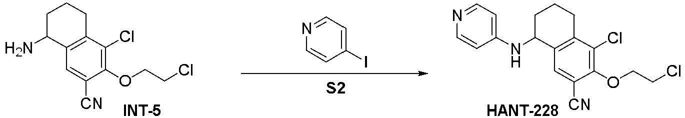

**INT-5** (50 mg, 0.177 mmol), **S2** (48 mg, 0.212 mmol), Pd₂(dba)₃ (48 mg, 0.053 mmol), Xantphos (61.5 mg, 0.106 mmol), and Cs₂CO₃ (115 mg, 0.354 mmol) were dissolved in 4 mL of toluene in a sealed tube and stirred at 100 °C overnight. The reaction was cooled to room temperature, quenched with aq. NH₄Cl and extracted with DCM. The organic phase was washed with saturated brine, dried over Na₂SO₄, filtered and concentrated under reduced pressure. The residue was purified by prep-HPLC to obtain compound **HANT-228** (13.5 mg, white solid), yield: 21.1%. LCMS (ESI): *m*/*z* = 362.0 [M+H]⁺; ¹H NMR (400 MHz, CD₃OD) δ 8.04 (d, *J =* 5.8 Hz, 2H), 7.56 (s, 1H), 7.52-7.31(m, 1H), 6.74-6.65 (m, 2H), 4.80 (d, *J =* 5.4 Hz, 1H), 4.42 (td, *J =* 5.6, 1.2 Hz, 2H), 3.91 (t, *J =* 5.6 Hz, 2H), 3.00-2.91 (m, 1H), 2.86 (s, 1H), 1.94 (ddt, *J =* 25.2, 1H).8.6, 6.0 Hz, 3H), 1.31 (d, *J =* 16.4 Hz, 1H).

### Example 68

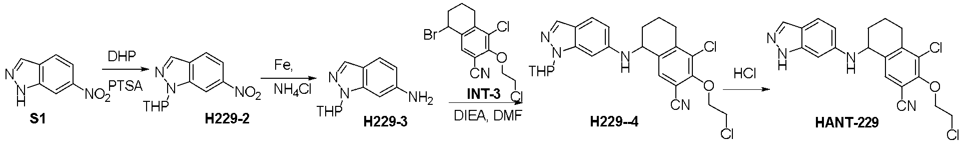

### Step 1: Synthesis of 6-nitro-1-(tetrahydro-2H-pyran-2-yl)-1H indazole

Compound **S1** (1 g, 6.1 mmol), DHP (1.03 g, 12.26 mmol) and PTSA (582 mg, 3.0 mmol) were dissolved in DCM (10 mL) and reacted under N₂ at room temperature for 12 h. The organic phase was washed with saturated brine and dried with Na₂SO₄, then filtered and concentrated under reduced pressure. The residue was purified by flash chromatography to obtain compound **H229-2** (1.1 g, yellow oil), yield: 73.3%. LCMS (ESI): *m*/*z* =248 [M+H]⁺.

### Step 2: Synthesis of 1-(tetrahydro-2H-pyran-2-yl)-1H-indazol-6-amine

**H229-2** (500 mg, 2.0 mmol), iron powder (680 mg, 12.0 mmol) and NH₄Cl (642 mg, 12.0 mmol) were dissolved in methanol/water (100 mL/10 mL) and stirred at 50 °C for 2 h. The reaction was cooled to room temperature then the iron powder was filtered off and methanol was removed by reduced pressure concentration. The reaction solution was extracted with EA and aq. NH₄Cl, and the organic phase was washed with saturated saline, then dried with Na₂SO₄, filtered and concentrated under reduced pressure to obtain crude **H229-3** (430 mg, brown oil). yield: 98%. LCMS (ESI): *m*/*z* =218 [M+H]⁺.

### Step 3: Synthesis of 4-chloro-3-(2-chloroethoxy)-8-((1-(tetrahydro-2H-pyran-2-yl)-1H-indazol-6-yl)amino)-5,6,7,8-tetrahydronaphthalene-2-carbonitrile

**H229-3** (50 mg, 0.23 mmol, crude) and compound **INT-3** (160 mg, 0.46 mmol) were dissolved in DMF (5 mL), then DIEA (89.2 mg, 0.69 mmol) was added in dropwise and the reaction was stirred at 60 °C overnight. The reaction was cooled to room temperature, quenched by pouring into icy aq. NH₄Cl. The reaction mixture was extracted with EA, and the organic phase was washed with water and saturated brine, then dried with anhydrous Na₂SO₄, filtered and concentrated under reduced pressure. The residue was purified by preparative TLC plate to obtain the compound **H229-4** (60 mg, yellow oil), yield: 49.6%. LCMS (ESI): *m*/*z* =485 [M+H]⁺.

### Step 4: Synthesis of 8-((1H-indazol-6-yl)amino)-4-chloro-3-(2-chloroethoxy)-5,6,7,8-tetrahydronaphthalene-2-carb onitrile

**H229-4** (60 mg, 0.124 mmol, crude) was dissolved in dioxane (5 mL) saturated with HCl and reacted at room temperature for 1 h. The reation was adjusted to pH~7 with saturated aq. Bicarbonate and extracted with EA. The organic phase was washed with water and saturated brine, then dried with Na₂SO₄, filtered and concentrated under reduced pressure. The residue was purified by neutral prep-HPLC to obtain compound **HANT-229** (12.2 mg, white solid), yield: 24.6%. LCMS (ESI): *m*/*z* =401 [M+H]⁺; ¹H NMR (400 MHz, CD₃OD) δ 7.78 (s, 1H), 7.67 (s, 1H), 7.47 (d, *J =* 8.8 Hz, 1H), 6.68-6.59 (m, 2H), 4.69 (s, 1H), 4.41 (t, *J =* 5.6 Hz, 2H), 3.91 (t, *J =* 5.6 Hz, 2H), 2.93 (d, *J =* 6.0 Hz, 1H), 2.84 (d, *J =* 18.6 Hz, 1H), 2.01 (d, *J =* 17.8 Hz, 4H).

### Example 69

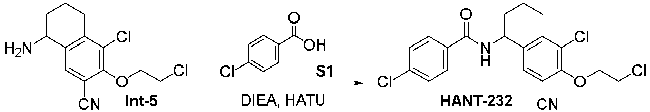

Compounds **INT-5** (30 mg, 0.106 mmol), **S1** (20 mg, 0.127 mmol), and HATU (52 mg, 0.137 mmol) were dissolved in DMF (5 mL) in a dry flask, then DIEA (41 mg, 0.317 mmol) was added and stirred at room temperature overnight. The reaction mixture was extracted with water and EA, and the organic phase was dried with anhydrous Na₂SO₄, filtered, and concentrated under reduced pressure. The residue was purified by flash chromatography to obtain compound **HANT-232** (23 mg, white solid, yield: 51.1%). LCMS (ESI): *m*/*z* = 424.0 [M+H]⁺. ¹H NMR (400 MHz, DMSO-*d*₆) δ 8.86 (d, *J =* 8.2 Hz, 1H), 7.96-7.89 (m, 2H), 7.63(s, 1H), 7.58-7.51 (m, 2H), 5.25-5.14 (m, 1H), 4.45-4.35 (m, 2H), 3.98 (dd, *J =* 5.8, 4.6 Hz, 2H), 2.82 (t, *J =* 5.6 Hz, 2H), 2.04-1.77 (m, 4H).

### Example 70

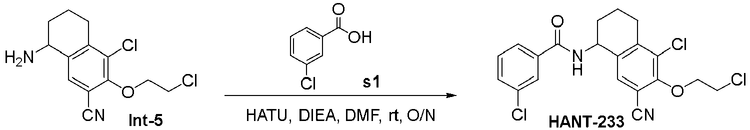

Compound S1 (30 mg, 0.19 mmol), compound **INT-5** (54 mg, 0.19 mmol) and HATU (110 mg, 0.29 mmol) were dissolved in DMF (5 mL), then DIEA (74 mg, 0.57 mmol) was added and stirred at room temperature overnight. The reaction solution was poured into EA and washed with saturated aq. NH₄Cl. The resulting organic phase was dried with anhydrous Na₂SO₄ and concentrated under reduced pressure, and the resulting residue was purified by reversed-phase prep-HPLC to obtain **HANT-233** (31.3 mg, yield: 39%) as a white solid. LCMS (ESI): *m*/*z* =423.0 [M+H]⁺; ¹H NMR (400 MHz, DMSO-*d*₆) δ 8.91 (d, *J =* 8.0 Hz, 1H), 7.94 (br s, 1H), 7.86 (d, *J =* 7.8 Hz, 1H), 7.66 (s, 1H), 7.63-7.61 (m, 1H), 7.53-7.49 (m, 1H), 5.19-5.18 (m, 1H), 4.44-4.32 (m, 2H),3.98 (dd, *J =* 5.9, 4.3 Hz, 2H), 2.81 (br s, 2H), 2.07-1.64 (m, 4H).

### Example 71

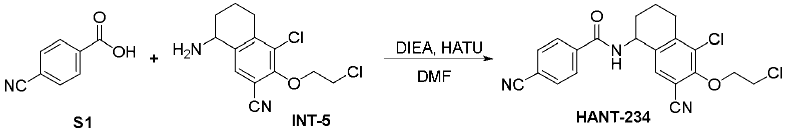

Compounds **INT-5** (30 mg, 0.106 mmol), **S1** (19 mg, 0.127 mmol), and HATU (52 mg, 0.137 mmol) were dissolved in DMF (5 mL) in a dry flask and DIEA (41 mg, 0.317 mmol) was added in and stirred at room temperature overnight. The reaction was extracted with water and EA, and the organic phase was dried with anhydrous Na₂SO₄, filtered, and the filtrate was concentrated under reduced pressure to afford the crude product. The residue was purified by flash chromatography to obtain compound **HANT-234** (20.3 mg, white solid, yield: 46.14%). LCMS (ESI): *m*/*z* = 414.1 [M+H]⁺.¹H NMR (400 MHz, DMSO-*d*₆) δ 9.03 (d, *J =* 8.0 Hz, 1H), 8.05 (d, *J* = 8.6 Hz, 2H), 7.97 (d, *J* = 8.6 Hz, 2H), 7.68 (s, 1H), 5.20 (d, *J* = 5.4 Hz, 1H), 4.47-4.32 (m, 2H), 4.06-3.92 (m, 2H), 2.81 (d, *J =* 5.6 Hz, 2H), 2.07-1.75 (m, 4H).

### Example 72

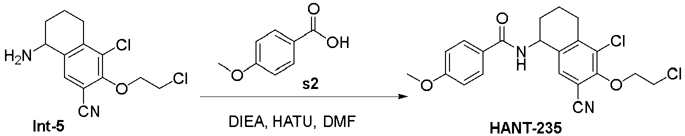

**INT-5** (19.3 mg, 0.12 mmol), HATU (48.2 mg, 0.12 mmol), DIEA (34.2 mg, 0.26 mmol) were dissolved in DMF (3 mL), then **S1** (30 mg, 0.1 mmol) was added, and the reaction was stirred at room temperature for 3 h. The reaction was extracted with NH₄Cl and EA, and the organic phases were dried with anhydrous Na₂SO₄ and concentrated. The residue was purified by prep-HPLC to obtain compound **HANT-235** (20.1 mg, white solid, yield: 45.5%) LCMS (ESI): *m*/*z* = 419.0 [M+H]⁺; ¹H NMR (400 MHz, CD₃OD) δ 7.84 (d, *J =* 9.0 Hz, 2H), 7.52 (s, 1H), 6.99 (d, *J =* 9.0 Hz, 2H), 5.28 (s, 1H), 4.41 (t, *J =* 5.6 Hz, 2H), 3.91 (t, *J =* 5.6 Hz, 2H), 3.85 (s, 3H), 2.91 (s, 2H), 2.13-1.99 (m, 2H), 1.95-1.83 (m, 2H).

### Example 73

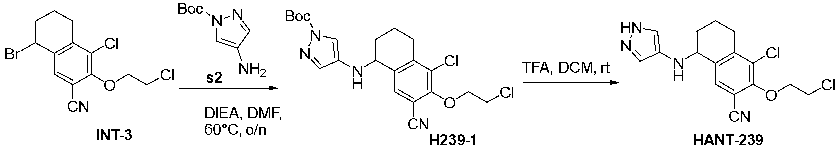

### Step 1: Synthesis of tert-butyl 4-((5-chloro-6-(2-chloroethoxy)-7-cyano-1,2,3,4-tetrahydronaphthalen-1-yl)amino)-1H-pyrazol e-1-carboxylate

Compounds **INT-3** (50 mg, 0.14 mmol), **S2** (53 mg, 0.28 mmol), DIEA (93 mg, 0.72 mmol) were dissolved in DMF (3 mL) and stirred at 60 °C overnight. The reaction was cooled to room temperature, extracted with EA, and the organic phase was dried and concentrated under reduced pressure. The residue was purified by flash chromatography to obtain **H239-1** (50 mg, yield: 78%) as a yellow solid. LCMS (ESI): *m*/*z* =451.1[M+H]⁺.

### Step 2: Synthesis of 8-((1H-pyrazol-4-yl)amino)-4-chloro-3-(2-chloroethoxy)-5,6,7,8-tetrahydronaphthalene-2-carb onitrile

Compound **H239-1** (50 mg), TFA (0.05 mL) was dissolved in DCM (3 mL) and reacted under N₂ at room temperature for 3 h. The reaction solution was extracted with EA, and the organic phase was concentrated under reduced pressure. The residue was purified by flash chromatography to obtain a white solid **HANT-239** (19.8 mg, yield: 62%). LCMS (ESI): *m*/*z* =351.1 [M+H]⁺; ¹H NMR (400 MHz, DMSO-*d*₆) δ 7.76 (s, 1H), 7.11 (s, 2H), 4.68 (d, *J =* 8.8 Hz, 1H), 4.43-4.31 (m, 2H), 4.21-4.12 (m, 1H), 3.96-3.98 (m, 2H), 2.86-2.67 (m, 2H), 1.90-1.94 (m, 1H), 1.87-1.62 (m, 3H).

### Example 74

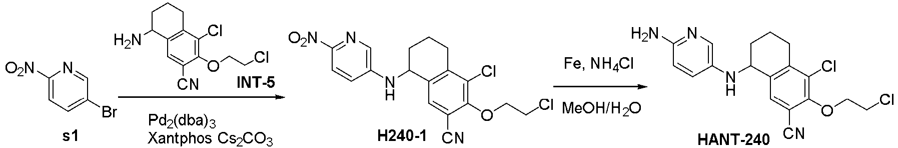

### Step 1: Synthesis of 4-chloro-3-(2-chloroethoxy)-8-((6-nitropyridin-3-yl)amino)-5,6,7,8-tetrahydronaphthalene-2-ca rbonitrile

In a sealed tube, compound **S1** (47 mg, 0.23 mmol) and compound **INT-5** (50 mg, 0.177 mmol) were dissolved in 4 mL of Tol, then Pd₂(dba)₃ (48.6 mg, 0.053 mmol), Xantphos (61 mg, 0.106 mmol) and Cs₂CO₃ (57.6 mg,0.353 mmol) were added in, and the reaction was allowed to react at 110 °C for 12 h under N₂. The reaction was cooled to room temperature, then poured into icy aq. NH₄Cl and extracted with EA. The organic phase was washed with saturated brine and then dried with Na₂SO₄, filtered and concentrated under reduced pressure. The residue was purified by flash chromatography to obtain the compound **H240-1** (50 mg, yellow oil), yield: 69.7%. LCMS (ESI): *m*/*z* =407 [M+H]⁺.

### Step 2: Synthesis of 8-((6-aminopyridin-3-yl)amino)-4-chloro-3-(2-chloroethoxy)-5,6,7,8-tetrahydronaphthalene-2-c arbonitrile

Compound **H240-1** (40 mg, 0.099 mmol), iron powder (33 mg, 0.591 mmol) and NH₄Cl (32 mg, 0.591 mmol) were dissolved in 3 mL/1mL of MeOH/H₂O and stirred at 50 °C for 6 h. The mixture was cooled to room temperature, filtered and concentrated to remove solvent. The residue was extracted with EA and with aq. NH₄Cl, and the organic phase was washed with saturated brine, dried with Na₂SO₄, and filtered. The filtrate was concentrated under reduced pressure, and the residue was purified by flash chromatography to obtain compound **HANT-240** (3.2 mg, gray solid), yield: 8.6%. LCMS (ESI): *m*/*z* =377 [M+H]⁺; ¹H NMR (400 MHz, CD₃OD) δ 7.68 (s, 1H), 7.34 (d, *J =* 3.0 Hz, 1H), 7.30 (dd, *J =* 9.0, 2.8 Hz, 1H), 6.71 (d, *J =* 9.0 Hz, 1H), 4.52-4.45 (m, 1H), 4.41 (t, *J = 5.6* Hz, 2H), 3.91 (t, *J =* 5.6 Hz, 2H), 3.00-2.88 (m, 1H), 2.86-2.74 (m, 1H),1.91 (s, 3H), 2.02-1.81 (m, 1H).

### Example 75

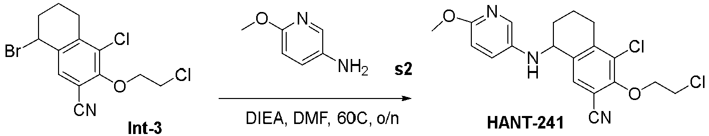

Synthesis was performed using methods described in Example **65** to afford **HANT-241** (15.8 mg, yield: 28%) as a pink solid. LCMS (ESI): *m*/*z* = 392.1 [M+H]⁺; ¹H NMR (400 MHz, CD₃OD) δ 7.68 (s, 1H), 7.57 (d, *J =* 2.9 Hz, 1H), 7.21 (dd, *J =* 8.9, 3.0 Hz, 1H), 6.69 (d, *J* = 2.9 Hz, 1H), 4.55-4.56 (m, 1H), 4.41 (t, *J* = 5.6 Hz, 2H), 3.91 (t, *J* = 5.6 Hz, 2H), 3.82 (s, 3H), 2.90-2.93 (m, 1H), 2.82-2.85 (m, 1H),2.07-1.91 (m, 2H), 1.90-1.79 (m, 2H).

### Example 76

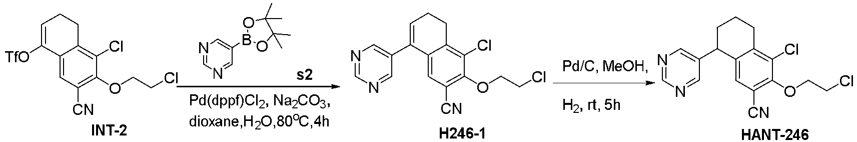

Synthesis was performed using methods described in Example **42** to afford **HANT-246** (20.7 mg, yield: 30%) as a white solid. LCMS (ESI): *m*/*z* =348.1 [M+H]⁺; ¹H NMR (400 MHz, DMSO-*d*₆) δ 9.08 (s, 1H), 8.56 (s, 2H), 7.32 (s, 1H), 4.41 (t, *J* = 5.2 Hz, 2H), 4.30 (t, *J =* 6.2 Hz, 1H), 3.97 (t, *J =* 5.2 Hz, 2H), 2.98 - 2.79 (m, 2H), 2.12 - 2.00 (m, 1H), 1.90 - 1.71 (m, 3H).

### Example 77

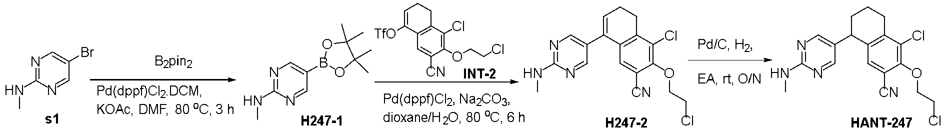

### Step 1: Synthesis of N-methyl-5-(4,4,5,5-tetramethyl-1,3,2-dioxobenzofuran-2-yl)pyrimidin-2-amine

**S1** (200 mg, 1.06 mmol), (Bpin)2 (320 mg, 1.27 mmol), Pd(dppf)Cl₂ (40 mg, 0.05 mmol), and KOAc (311 mg, 3.18 mmol) were sequentially added to DMF (10 mL) in a dry flask and stirred at 80 °C for 3 h. The reaction was cooled to room temperature then poured into EA, washed with saturated brine. The organic phases were combined and concentrated under reduced pressure, and purified by flash chromatography to obtain **H247-1** (120 mg, yield: 48%) as a white solid. LCMS (ESI): *m*/*z* =236.1 [M+H]⁺.

### Step 2: Synthesis of 4-chloro-3-(2-chloroethoxy)-8-(2-(methylamino)pyrimidin-5-yl)-5,6-dihydronaphthalene-2-car bonitrile

Compound **H247-1** (100 mg, 0.43 mmol), **INT-2** (136 mg, 0.33 mmol), Pd(dppf)Cl2 (24 mg, 0.033 mmol), and Na₂CO₃ (70 mg, 0.66 mmol) were sequentially dissolved in dioxane/water (8/2 mL) in a dry flask and stirred for 6 h under N₂ at 80 °C. The reaction solution was cooled to room temperature then poured into saturated brine, extracted with EA. The organic phases were combined, concentrated under reduced pressure, and purified by flash chromatography to obtain **H247-2** (50 mg, yield: 32%) as a white solid. LCMS (ESI): *m*/*z* =375.0 [M+H]⁺.

### Step 3: Synthesis of 4-chloro-3-(2-chloroethoxy)-8-(2-(methylamino)pyrimidin-5-yl)-5,6,7,8-tetrahydronaphthalene -2-carbonitrile

To a dry flask was added compound **H247-2** (50 mg, 0.13 mmol) in EA (10 mL) and Pd/C (10 mg, 10% wt). The reaction was stirred overnight at room temperature under H₂, then filtered, combined and concentrated under reduced pressure. The residue was purified by reversed-phase prep-HPLC to obtain **HANT-247** (8.1 mg, yield: 16%) as a white solid. LCMS (ESI): *m*/*z* = 377.0 [M+H]⁺; ¹H NMR (400 MHz, DMSO-*d*₆) δ 8.01 (s, 2H), 7.28 (s, 1H), 7.00 (q, *J* =4.8 Hz, 1H), 4.39 (t, *J =* 5.2 Hz, 2H), 4.04-3.99 (m, 1H), 3.99-3.93 (m, 2H), 2.86 (q, *J =* 6.8 Hz, 2H), 2.77 (d, *J =* 4.7 Hz, 3H), 2.08-1.95 (m, 1H), 1.80-1.76 (m, 3H).

### Example 78

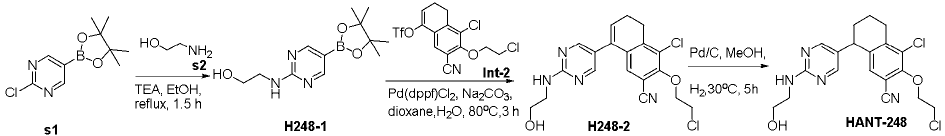

### Step 1: Synthesis of 2-((5-(4,4,5,5-tetramethyl-1,3,2-dioxobenzofuran-2-yl)pyrimidin-2-ylamino)ethan-1-ol

Compounds **S1** (200 mg, 0.83 mmol), **S2** (76 mg, 1.25 mmol), TEA (252 mg, 2.5 mmol) were dissolved in ethanol (5 mL) and reacted at 78 °C for 1.5 h. The reaction was cooled to room temperature, then extracted with EA. The organic phase was dried and concentrated under reduced pressure, and the residue was purified by flash chromatography to obtain the white solid **H248-1** (200 mg, yield: 91%) LCMS (ESI): *m*/*z* =266.2[M+H]⁺.

### Step 2: Synthesis of 4-chloro-3-(2-chloroethoxy)-8-(2-((2-hydroxyethyl)amino)pyrimidin-5-yl)-5,6-dihydronaphthal ene-2-carbonitrile

Compound **H248-1** (66 mg, 0.36 mmol), **INT-2** (100 mg, 0.24 mmol), Pd(dppf)Cl₂ (35 mg, 0.05 mmol), Na₂CO₃ (51 mg, 0.48 mmol) were dissolved in dioxane/water (3 mL/0.5 mL) and stirred at 80 °C for 3 h. The reaction was cooled to room temperature then extracted with EA. The organic phase was dried and concentrated, and the residue was purified by flash chromatography to obtain **H248-2** as a solid (45 mg, yield: 46%). LCMS (ESI): *m*/*z* =405.1[M+H]⁺.

### Step 3: Synthesis of 4-chloro-3-(2-chloroethoxy)-8-(2-((2-hydroxyethyl)amino)pyrimidin-5-yl)-5,6,7,8-tetrahydrona phthalene-2-carbonitrile

Compound **H248-2** (45 mg, 0.11 mmol) and Pd/C (90 mg) were dissolved in MeOH (3 mL) and reacted under H₂ at 30 °C for 5 h. The reaction solution was filtered, concentrated under reduced pressure, and the residue was purified by prep-HPLC to obtain **HANT-248** (5.2 mg, yield: 12%) as a white solid. LCMS (ESI): *m*/*z=* 407.1 [M+H]⁺; ¹H NMR (400 MHz, CD₃OD) δ 8.01 (s, 2H), 7.17 (s, 1H), 4.41 (t, *J =* 5.6 Hz, 2H), 4.06 (d, *J =* 6.8 Hz, 1H), 3.91 (t, *J =* 5.6 Hz, 2H), 3.69 (t, *J =* 5.7 Hz, 2H), 3.49 (t, *J =* 5.7 Hz, 2H), 2.94 (t, *J =* 6.2 Hz, 2H), 2.14-1.99 (m, 1H), 1.98-1.75 (m, 3H).

### Example 79

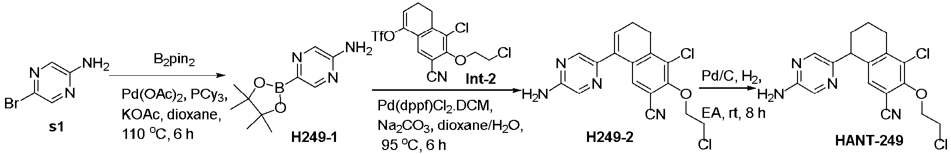

Synthesis was performed using methods described in Example **51** to afford **HANT-249** (4.9 mg, yield: 31%) as a white solid. LCMS (ESI): *m*/*z* =363.0 [M+H]⁺; ¹H NMR (400 MHz, DMSO-*d*₆) δ 7.80 (s, 1H), 7.72 (s, 1H), 7.22 (s, 1H), 6.29 (s, 2H), 4.50-4.29 (m, 2H), 4.13 (t, *J* = 6.2 Hz, 1H), 3.96 (dd, *J* = 6.0, 4.3 Hz, 2H), 2.84 (t, *J* = 6.2 Hz, 2H), 1.93-1.86 (m, 3H), 1.82-1.64 (m, 1H).

### Example 80

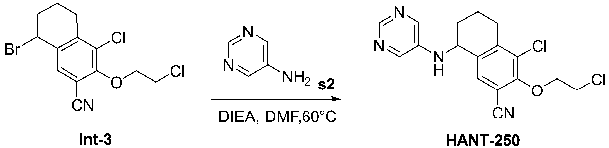

Synthesis was performed using methods described in Example **65** to afford **HANT-250** (3.3 mg, white solid, yield: 6.3%). LCMS (ESI): *m*/*z* =363.0 [M+H]⁺; ¹H NMR (400 MHz, CD₃OD) δ 8.42 (s, 1H), 8.23 (s, 2H), 7.66 (s, 1H), 4.76 (s, 1H), 4.42 (t, *J =* 5.6 Hz, 2H), 3.92 (t, *J* = 5.6 Hz, 2H), 2.97 (d, *J =* 18.4 Hz, 1H), 2.83 (d, *J =* 18.4 Hz, 1H), 1.95 (dd, *J* = 14.6, 7.4 Hz, 4H).

### Example 81

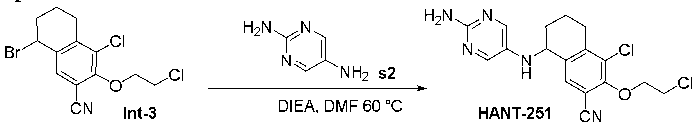

Synthesis was performed using methods described in Example **65** to afford **HANT-251** (17.6 mg, white solid), yield: 33%. LCMS (ESI): *m*/*z* = 378.1 [M+H]⁺; ¹H NMR (400 MHz, DMSO-*d*₆) δ 7.89 (s, 2H), 7.79 (s, 1H), 5.82 (s, 2H), 5.19(d, *J =* 9.4 Hz, 1H), 4.55-4.43 (m, 1H), 4.42-4.31 (m, 2H), 4.01-3.87 (m, 2H), 2.92-2.78 (m, 1H), 2.70 (dd, *J =* 18.6, 7.0 Hz, 1H), 1.87 (d, *J =* 5.2 Hz, 1H), 1.75 (dt, *J =* 17.8, 10.4 Hz, 3H).

### Example 82

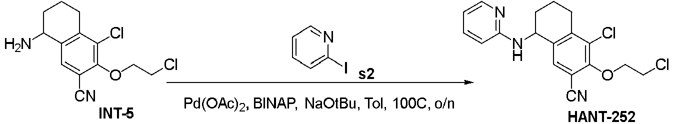

Compounds **INT-5** (50 mg, 0.18 mmol), **S2** (54 mg, 0.26 mmol), Pd(OAc)₂ (8 mg, 0.03 mmol), BINAP (22 mg, 0.03 mmol), and NaOtBu (35 mg, 0.36 mmol) were added to 3 mL of toluene in a sealed tube, and the reaction was stirred at 100 °C overnight. The reaction was cooled to room temperature, and extracted with EA. The organic phase was dried and concentrated under reduced pressure, and the residue was purified by flash chromatography to obtain **HANT-252** (3.3 mg, yield: 4 %) as a white solid. LCMS (ESI): *m*/*z* =362.1[M+H]⁺; ¹H NMR (400 MHz, DMSO-*d*₆) δ 8.04-7.97 (m, 1H), 7.62 (s, 1H), 7.40 (td, *J =* 8.3, 7.7, 2.0 Hz, 1H), 6.92 (d, *J* = 8.3 Hz, 1H), 6.51-6.54 (m, 2H), 5.29-5.09 (m, 1H), 4.45-4.32 (m, 2H), 4.03-3.92 (m, 2H), 2.77-2.83 (m,2H), 1.75-1.93 (m, 4H).

### Example 83

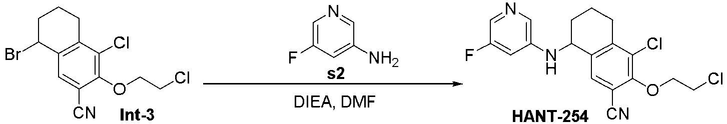

Synthesis was performed using methods described in Example **65** to afford **HANT-254** (2.1 mg, white solid), yield: 5%. LCMS (ESI): *m*/*z* = 380 [M+H]⁺; ¹H NMR (400 MHz, CD₃OD) δ 7.85 (s, 1H), 7.69-7.61 (m, 2H), 6.94 (dt, *J =* 11.8,2.6 Hz, 1H), 4.69 (d, *J =* 4.4 Hz, 1H), 4.42 (t, *J* = 5.6 Hz, 2H), 3.91 (t, *J =* 5.6 Hz, 2H), 3.01-2.90 (m, 1H), 2.84 (s, 1H), 2.19 (t, *J =* 7.8 Hz, 1H), 1.99-1.86 (m, 3H).

### Example 84

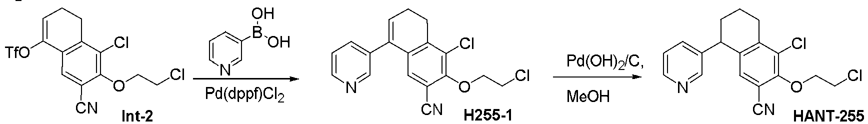

Synthesis was performed using methods described in Example **27** to afford **HANT-255** (10.4 mg, yield: 26%). LCMS (ESI): *m*/*z* = 347.2[M+H]⁺. ¹H NMR (400 MHz, CD₃OD) δ 8.43 (dd, *J* = 4.8, 1.4 Hz, 1H), 8.34 (d, *J =* 4.8, 1.4 Hz, 1H) 8.34 (d, *J =* 2.0 Hz, 1H), 7.53 (d, *J =* 8.0 Hz, 1H), 7.39 (dd, *J =* 8.0, 5.0 Hz, 1H), 7.07 (s, 1H), 4.42 (t, *J=* 5.6 Hz, 2H), 4.28 (t, *J =* 6.2 Hz, 1H), 3.91 (t, *J =* 5.6 Hz, 2H), 2.97 (t, *J =* 6.2 Hz, 2H), 2.27-2.08 (m, 1H), 1.96-1.77 (m, 1H), 2.27-2.08 (m, 1H), 2.27-2.08 (m, 1H), 2.27-2.08 (m, 2H) 1.96-1.77 (m, 3H).

### Example 85

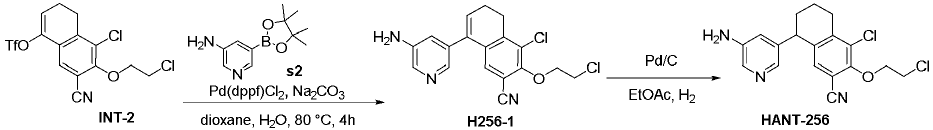

Synthesis was performed using methods described in Example **42** to afford **HANT-256** (2.3 mg, white solid), yield: 8%. LCMS (ESI): *m*/*z* = 362.0 [M+H]⁺; ¹H NMR (400 MHz, CD₃OD) δ 7.84 (d, *J =* 2.6 Hz, 1H), 7.59 (d, *J =* 2.2 Hz, 1H), 7.10 (s, 1H), 6.71 (t, *J =* 2.2 Hz, 1H), 4.41 (t, *J* = 5.6 Hz, 2H), 4.12 (t, *J =* 5.6 Hz, 2H), 4.12 (t, *J =* 5.6 Hz, 2H)7.10 (s, 1H), 6.71 (t, *J* = 2.2 Hz, 1H), 4.41 (t, *J =* 5.6 Hz, 2H), 4.12 (t, *J =* 6.4 Hz, 1H), 3.91 (t, *J =* 5.6 Hz, 2H), 2.95 (t, *J =* 6.2 Hz, 2H), 2.17-2.02 (m, 1H), 1.96-1.78 (m, 3H).

### Example 86

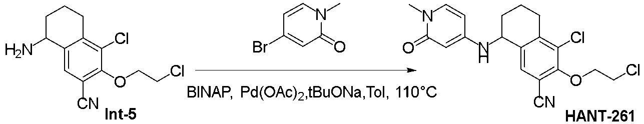

**INT-5** (20 mg, 0.07 mmol), 4-bromo-1-methylpyridin-2-one (19 mg, 0.1 mmol), sodium tert-butoxide (20 mg, 0.21 mmol), Pd(OAc)₂ (3 mg, 0.015 mmol) and BINAP (17 mg, 0.03 mmol) were added to toluene (10 mL) in a flask and refluxed at 110 °C under N₂ overnight. The reaction was cooled to room temperature, quenched with NH₄Cl (aq.) and extracted with EA. The organic phase was washed with saturated brine, dried over anhydrous Na₂SO₄, filtered and concentrated under reduced pressure. The residue was purified by reversed-phase prep-HPLC to obtain **HANT-261** (5 mg, 19% yield) as a yellow solid. LCMS (ESI): *m*/*z* =392 [M+H]⁺; ¹H NMR (400 MHz, CD₃OD) δ 7.53 (s, 1H), 7.32 (d, *J =* 7.6 Hz, 1H), 5.95 (dd, *J =* 7.4, 2.4 Hz, 1H), 5.63 (d, *J =* 2.6 Hz, 1H), 4.66 (s, 1H), 4.41 (t, *J =* 5.6 Hz, 2H), 3.91 (t, *J =* 5.6 Hz, 2H), 3.43 (s3H), 2.92 (dd, *J =* 15.2, 9.4 Hz, 1H), 2.88- 2.80 (m, 1H), 1.99-1.84 (m, 4H).

### Example 87

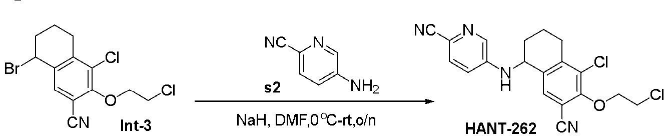

In a dry flask compound **S2** (25 mg, 0.22 mmol) was dissolved in 3 mL of DMF and cooled to 0 °C, then NaH (5 mg, 0.22 mmol) was added in and reacted at 0 °C for 30 min,. **INT-3** (50 mg, 0.14 mmol) in 1 mL of DMF was added slowly dropwise, then the reaction was allowed to room temperature and stirred overnight. The reaction was extracted by EA (20 mL*3), then the organic phase was dried with Na₂SO₄, filtered, and concentrated under reduced pressure. The residue was purified to obtain **HANT-262** (7 mg, yield: 13%) as a pale yellow solid. LCMS (ESI): *m*/*z* =387.0 [M+H]⁺; ¹H NMR (400 MHz, DMSO-*d*₆) δ 8.16 (d, *J* = 2.8 Hz, 1H), 7.77 (s, 1H), 7.72 (d, *J =* 2.8 Hz, 1H)7.72 (d, *J =* 8.6 Hz, 1H), 7.36 (d, *J =* 8.0 Hz, 1H), 7.20-7.13 (m, 1H), 4.87 (s, 1H), 4.45-4.40 (m, 2H), 4.03-3.99 (m, 2H), 2.90-2.75 (m, 2H), 2.02 (d, *J* = 7.2 Hz, 1H), 1.87 (d, *J =* 7.0 Hz, 3H).

### Example 88

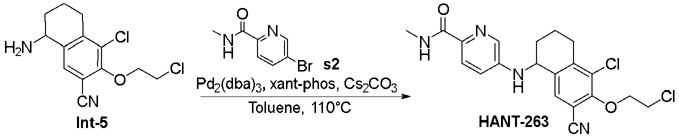

Compound **INT-5** (30 mg, 0.105 mmol) and **S2** (34 mg, 0.157 mmol) were dissolved in 8 mL of toluene, and then Pd₂(dba)₃ (29 mg, 0.032 mmol), XantPhos (37 mg, 0.063 mmol) and Cs₂CO₃(103 mg, 0.316 mmol) were added in and reacted at 110 °C overnight under N₂. The reaction was cooled to room temperature, concentrated and extracted with water and EA. The organic phase was dried with anhydrous Na₂SO₄, filtered, and concentrated under reduced pressure to obtain the crude product. The crude product was purified by flash chromatography to obtain compound **HANT-263** (4.4 mg, white solid) yield: 10%. LCMS (ESI): *m*/*z* =419.0 [M+H]⁺; ¹H NMR (400 MHz, DMSO-*d*₆) δ 8.29 (q, *J =* 4.8 Hz, 1H), 8.03 (d, *J =* 2.8 Hz, 1H), 7.78 (d, *J =* 2.8 Hz, 1H), 7.78 (d, *J=* 2.8 Hz, 1H).7.78 (d, *J =* 8.6 Hz, 1H), 7.72 (s, 1H), 7.14 (dd, *J =* 8.8, 2.8 Hz, 1H), 6.81 (d, *J =* 8.6 Hz, 1H), 4.78 (d, *J =* 7.4 Hz, 1H), 4.45-4.34 (m, 2H), 3.98 (dd, *J =* 5.8, 4.4 Hz, 2H), 2.92-2.81 (m, 1H), 2.77 (d, *J =* 4.8 Hz, 4H), 1.84 (td, *J =* 12.0, 10.6, 5.8 Hz, 4H).

### Example 89

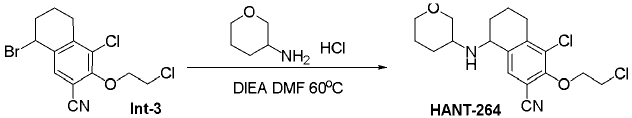

Synthesis was performed using methods described in Example **65** to afford **HANT-264** (5.5 mg, white solid, 4.7% yield). LCMS (ESI): *m*/*z* =369 [M+H]⁺; ¹H NMR (400 MHz, CD₃OD) δ 8.26 (br s, 1H), 7.75 (d, *J =* 5.4 Hz, 1H), 4.43 (td, *J =* 5.6, 2.4 Hz, 2H), 4.10 (dd, *J =* 18.6, 11.4 Hz, 1H), 3.91 (t, *J =* 5.6 Hz, 3H), 3.81 (d, *J =* 11.4 Hz, 1H), 3.48 (t, *J =* 9.2 Hz, 1H), 3.35 (dd, *J =* 12.0, 6.0 Hz, 1H), 3.07-2.93 ( m, 2H), 2.78 (d, *J =* 18.8 Hz, 1H), 2.09-1.84 (m, 5H), 1.78 (s, 1H), 1.66 (dt, *J =* 27.0, 16.4 Hz, 1H), 1.54 (dd, *J =* 19.4, 9.2 Hz, 1H).

### Example 90

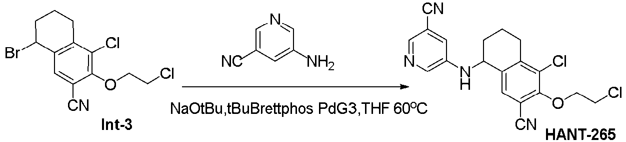

**INT-3** (20 mg, 0.07 mmol), 5-aminonicotinonitrile (26 mg, 0.14 mmol), NaO^{t}Bu (21 mg, 0.21 mmol), and tBuBrettphos-Pd-G3 (7 mg, 0.21 mmol) were dissolved in THF (5 mL) and stirred at 60 °C under N₂ overnight. The reaction was cooled to room temperature, quenched with NH₄Cl (aq.) and extracted with EA. The organic phase was washed with saturated brine, dried with anhydrous Na₂SO₄ sodium sulfate, filtered and concentrated under reduced pressure. The residue was purified by reversed-phase prep-HPLC to obtain **HANT-265** (5.5 mg, 20.3% yield as a white solid). LCMS (ESI): *m*/*z* =387[M+H]⁺; ¹H NMR (400 MHz, CD₃OD) δ 8.22 (d, *J = 2.8* Hz, 1H), 8.08 (d, *J =* 1.2 Hz, 1H), 7.63 (s, 1H), 7.41 (d, *J =* 1.6 Hz, 1H), 4.73 (br s, 1H), 4.42 (t, *J= 5.6* Hz, 2H), 3.92 (t, *J =* 5.6 Hz, 2H), 2.95-2.84(m, 2H), 1.97-1.88 (m, 4H).

### Example 91

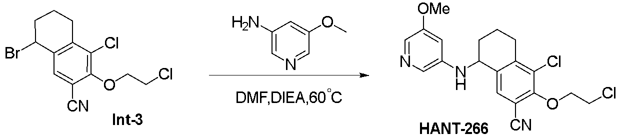

Synthesis was performed using methods described in Example **65** to afford **HANT-266** (4 mg, white solid, 9.8% yield). LCMS (ESI): *m*/*z* = 392[M+H]⁺; ¹H NMR (400 MHz, DMSO-*d*₆) δ 7.69 (d, *J* = 4.0 Hz, 2H), 7.54 (d, *J =* 2.4 Hz, 1H), 6.55 (t*J* = 2.0 Hz, 1H), 6.24 (d, *J =* 8.8 Hz, 1H), 5.33 (br s, 1H), 4.71 (t, *J* = 8.8 Hz, 2H), 4.41 (t, *J =* 4.8 Hz, 2H), 3.96 (s, 3H), 3.10-2.72 (m, 2H), 2.03-1.78 (m, 4H).

### Example 92

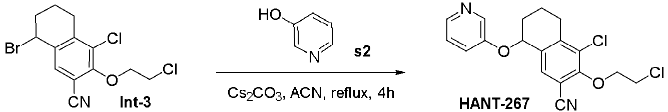

Compound **INT-3** (50 mg, 0.143 mmol) and **S2** (18 mg, 0.186 mmol) were dissolved in 20 mL of acetonitrile, then Cs₂CO₃ (140 mg, 0.430 mmol) was added and refluxed for 4 h. The reaction was cooled to room temperature, concentrated, and the residue was purified by flash chromatography to afford compound **HANT-267** (16.1 mg, white solid, 31% yield). LCMS (ESI): *m*/*z* =363.0 [M+H]⁺; ¹H NMR (400 MHz, CDCl₃) δ 8.32 (s, 1H), 8.24 (s, 1H), 7.51 (s, 1H), 7.23 (q, *J* = 3.2, 2.8 Hz 2H), 5.32-5.23 (m, 1H), 4.37 (t, *J =* 6.2 Hz, 2H), 3.83 (t, *J =* 6.2 Hz, 2H), 2.95 (dt, *J =* 19.0, 5.8 Hz, 1H), 2.79-2.64 (m, 1H), 2.12-1.82 (m, 4H).

### Example 93

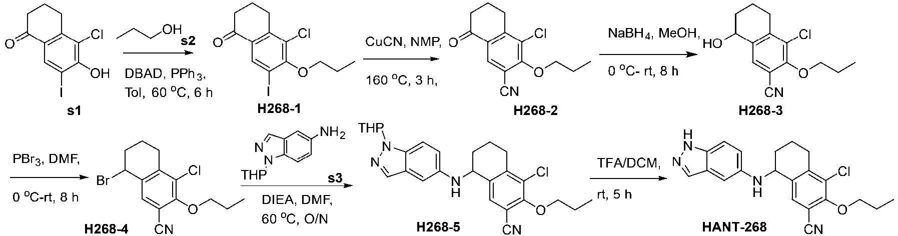

### Step 1: Synthesis of 5-chloro-7-iodo-6-propoxy-3,4-dihydronaphthalen-1(2H)-one

**S1** (1 g, 3.1 mmol), **S2** (370 mg, 6.2 mmol), DBAD (1.05 g, 4.6 mmol), and PPh3 (1.2 g, 4.6 mmol) were sequentially dissolved in toluene (15 mL) in a dry flask, and stirred at 60 °C for 6 h. The reaction was cooled to room temperature and concentrated under reduced pressure to remove solvent. The crude product was poured into the EA, washed with saturated brine, and the organic phases were combined and concentrated under reduced pressure. The residue was purified by flash chromatography to obtain the yellow oily compound **H268-1** (270 mg, yield: 25%). LCMS (ESI): *m*/*z* =365.0 [M+H]⁺.

### Step 2: Synthesis of 4-chloro-8-oxo-3-propoxy-5,6,7,8-tetrahydronaphthalene-2-carbonitrile

**H268-1** (220 mg, 0.6 mmol) and CuCN (270 mg, 3.0 mmol) were dissolved in NMP (8 mL) in a dry sealed tube and stirred at 160 °C for 3 h. The reaction was cooled to room temperature, quenched with ammonia, then poured into the EA and washed with aq. NH₄Cl. The organic phases were combined and concentrated under reduced pressure, and the residue was purified by flash chromatography to obtain yellow solid **H268-2** (100 mg, yield: 63%). LCMS (ESI): *m*/*z* =264.1 [M+H]⁺.

### Step 3: Synthesis of 4-chloro-8-hydroxy-3-propoxy-5,6,7,8-tetrahydronaphthalene-2-carbonitrile

Compound **H268-2** (100 mg, 0.38 mmol) was dissolved in MeOH (10 mL) in a dry flask, and NaBH₄ (58 mg, 3.8 mmol) was added in with an ice bath and the reaction was stirred at room temperature for 8 h. The reaction was quenched with water, concentrated under reduced pressure, and purified by flash chromatography to obtain the white solid **H268-3** (80 mg, yield: 66%). LCMS (ESI): *m*/*z* =266.0 [M+H]⁺.

### Step 4: Synthesis of 8-bromo-4-chloro-3-propoxy-5,6,7,8-tetrahydronaphthalene-2-carbonitrile

Compound **H268-3** (70 mg, 0.26 mmol) was dissolved in DMF (8 mL) in a dry flask, then PBr₃ (350 mg, 1.3 mmol) was added in at 0 °C, and the reaction was stirred at room temperature for 8 h. The reaction was quenched with aq. NH₄Cl, then reaction solution was poured into the EA and washed with saturated brine. The organic phases were combined, dried and concentrated under reduced pressure. The residue was purified by flash chromatography to obtain white solid **H268-4** (50 mg, yield: 51%). LCMS (ESI): *m*/*z* =328.0 [M+H]⁺.

### Step 5: Synthesis of 4-chloro-3-propoxy-8-(1-(tetrahydro-2H-pyran-2-yl)-1H-indazol-5-yl)amino)-5,6,7,8-tetrahydr onaphthalene-2-carbonitrile

Compounds **H268-4** (40 mg, 0.12 mmol) and **S3** (52 mg, 0.24 mmol) were dissolved in DMF (5 mL) in a dry flask, then DIEA (77 mg, 0.6 mmol) was added, and the reaction was stirred at 60 °C overnight. The reaction was cooled to room temperature, then poured into EA, washed with saturated brine. The organic phases were combined, dried and concentrated under reduced pressure, and the residue was purified by flash chromatography to obtain **H268-5** (35 mg, yield: 63%) as a white solid. LCMS (ESI): *m*/*z* =465.2 [M+H]⁺.

### Step 6: Synthesis of 8-((1H-indazol-5-yl)amino)-4-chloro-3-propoxy-5,6,7,8-tetrahydronaphthalene-2-carbonitrile

Compound **H268-5** (35 mg, 0.075 mmol) was dissolved in DCM (5 mL) in a dry flask and TFA (2 mL) was added, and the reaction was stirred at room temperature for 5 h. The reaction solution was concentrated under reduced pressure and purified by reversed-phase prep-HPLC to obtain a white solid **HANT-268** (7.4 mg, yield: 26%). LCMS (ESI): *m*/*z* =381.1 [M+H]⁺; ¹H NMR (400 MHz, DMSO-*d*₆) δ 12.63 (s, 1H), 7.78 (s, 1H), 7.71 (s, 1H), 7.31 (d, *J =* 8.8 Hz, 1H), 6.91 (dd, *J =* 8.9, 2.1 Hz, 1H), 6.85 (s, 1H), 5.68 (d, *J =* 8.9 Hz, 1H).1H), 4.62 (t, *J =* 4.9 Hz, 1H), 4.08 (t, *J =* 6.4 Hz, 2H), 2.91-2.85 (m, 1H), 2.78-2.71 (m, 1H), 1.96-1.69 (m, 6H), 1.04 (t, *J* = 7.4 Hz, 3H).

### Example 94

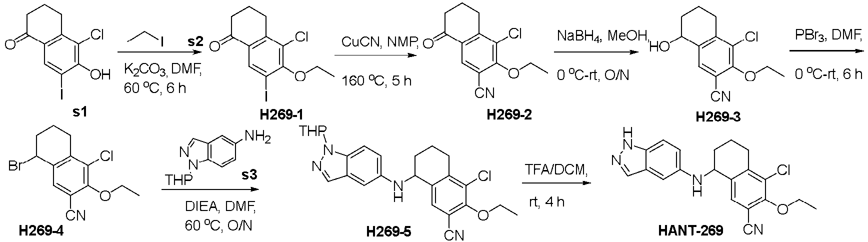

### Step 1: Synthesis of 5-chloro-6-ethoxy-7-iodo-3,4-dihydronaphthalen-1(2H)-one

Compound **S1** (1 g, 3.1 mmol) and **S2** (960 mg, 6.2 mmol) were sequentially dissolved in DMF (15 mL) in a dry flask, then K₂CO₃ (1.3 g, 9.3 mmol) was added and stirred at 60 °C for 6 h. The reaction was cooled to room temperature, poured into EA and washed three times with saturated brine. The organic phases were combined and concentrated under reduced pressure, and the residue was purified by flash chromatography to obtain a yellow solid **H269-1** (370 mg, yield: 34%). LCMS (ESI): *m*/*z* =351.0 [M+H]⁺.

### Step 2: Synthesis of 4-chloro-3-ethoxy-8-oxo-5,6,7,8-tetrahydronaphthalene-2-carbonitrile

Compounds H269-1 (320 mg, 0.91 mmol) and CuCN (410 mg, 4.6 mmol) were sequentially dissolved in NMP (15 mL) in a dry sealed tube and stirred at 160 °C for 5 h. The reaction was cooled to room temperature, quenched with ammonia, then poured into EA, and washed three times with aq. NH₄Cl. The organic phases were combined and concentrated under reduced pressure, and the residue was purified by flash chromatography to obtain yellow solid **H269-2** (140 mg, yield: 54%). LCMS (ESI): *m*/*z* =250.1 [M+H]⁺.

### Step 3: Synthesis of 4-chloro-3-ethoxy-8-hydroxy-5,6,7,8-tetrahydronaphthalene-2-carbonitrile

Compound **H269-2** (140 mg, 0.56 mmol) was dissolved in MeOH (10 mL) in a dry flask, then NaBH4 (85 mg, 2.2 mmol) was added in with an ice bath and the reaction was stirred overnight at room temperature. The reaction was quenched with water, concentrated under reduced pressure and the residue was purified by flash chromatography to obtain **H269-3** (110 mg, yield: 78%) as a white solid. LCMS (ESI): *m*/*z* =251.1 [M+H]⁺.

### Step 4: Synthesis of 8-bromo-4-chloro-3-ethoxy-5,6,7,8-tetrahydronaphthalene-2-carbonitrile

Compound **H269-3** (110 mg, 0.44 mmol) was dissolved in DMF (10 mL) in a dry flask, then PBr₃ (590 mg, 2.2 mmol) was added at 0 °C and stirred at room temperature for 6 h. The reaction solution was quenched with aqueous NH₄Cl, poured into the EA, and washed three times with saturated brine. The organic phases were combined, dried and concentrated under reduced pressure, and the residue was purified by flash chromatography to obtain white solid **H269-4** (80 mg, yield: 51%) was obtained. LCMS (ESI): *m*/*z* =314.0 [M+H]⁺.

### Step 5: Synthesis of 4-chloro-3-ethoxy-8-(1-(tetrahydro-2H-pyran-2-yl)-1H-indazol-5-yl)amino)-5,6,7,8-tetrahydron aphthalene-2-carbonitrile

Compounds **H269-4** (70 mg, 0.22 mmol) and **S3** (95 mg, 0.44 mmol) were dissolved in DMF (5 mL) in a dry flask and DIEA (143 mg, 1.1 mmol) was added. The reaction was stirred at 60 °C overnight. The reaction was cooled to room temperature, poured into EA and washed with saturated brine. The organic phases were combined, dried and concentrated under reduced pressure, and the residue was purified by flash chromatography obtain **H269-5** (80 mg, yield: 70%) as a white solid. LCMS (ESI): *m*/*z* =451.1 [M+H]⁺.

### Step 6: Synthesis of 8-((1H-indazol-5-yl)amino)-4-chloro-3-ethoxy-5,6,7,8-tetrahydronaphthalene-2-carbonitrile

Compound **H269-5** (80 mg, 0.18 mmol) was dissolved in DCM (5 mL) in a dry flask and TFA (2 mL) was added and the reaction was stirred at room temperature for 4 h. The reaction solution was concentrated under reduced pressure and the residue was purified by reversed-phase prep-HPLC to obtain **HANT-269** (12.8 mg, yield: 20%) as a white solid. LCMS (ESI): *m*/*z* =367.1 [M+H]⁺; ¹H NMR (400 MHz, DMSO-*d*₆) δ 12.66 (s, 1H), 7.78 (s, 1H), 7.72 (s, 1H), 7.31 (d, *J* = 8.9 Hz, 1H), 6.90 (dd, *J =* 8.8, 2.0 Hz, 1H), 6.85 (s, 1H), 5.72-5.69 (d, *J* =8.9 Hz, 1H), 4.64-4.62 (m, 1H), 4.17 (q, *J =* 7.0 Hz, 2H), 2.84-2.73 (m, 2H), 2.07-1.83 (m, 4H), 1.39 (t, *J =* 7.0 Hz, 3H).

### Example 95

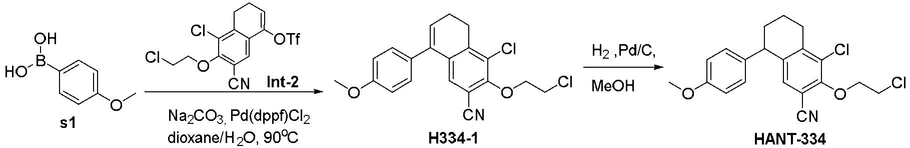

Synthesis was performed using methods described in Example 27 to afford the compound **HANT-334** (8.5 mg, colorless oil, yield: 33.8%). LCMS : *m*/*z* =393.2 [M+NH₃+H]⁺; ¹H NMR (400 MHz, CD₃OD) δ 7.01 (s, 1H), 6.99-6.94 (m, 2H), 6.88-6.82 (m, 2H), 4.38 (t, *J =* 5.6 Hz, 2H), 4.06 (t, *J =* 6.4 Hz, 1H), 3.89 (t, *J =* 5.6 Hz, 2H), 3.89 (t, *J =* 5.6 Hz, 2H)2H), 4.38 (t, *J =* 5.6 Hz, 2H), 4.06 (t, *J* = 6.4 Hz, 1H), 3.89 (t, *J =* 5.6 Hz, 2H), 3.76 (s, 3H), 3.00-2.81 (m, 2H), 2.10-2.01 (m, 1H), 1.97-1.88 (m, 1H), 1.87-1.74 (m, 2H).

### Example 96

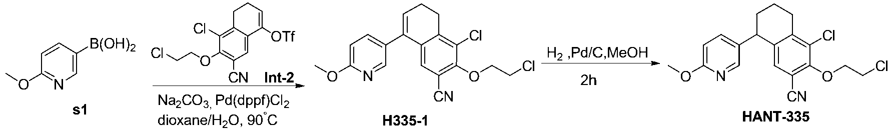

Synthesis was performed using methods described in Example **27** to afford **HANT-335** (21 mg, white solid, 41.8% yield). LCMS (ESI): *m*/*z* = 377 [M+H]⁺; ¹H NMR (400 MHz, MeOD) δ 7.87 (d, *J =* 1.8 Hz, 1H), 7.37 (dd, *J =* 8.6, 2.2 Hz, 1H), 7.05 (s, 1H), 6.76 (d, *J =* 5.6 Hz, 1H), 4.40 (t, *J =* 5.6 Hz, 2H), 4.14 (d, *J =* 5.6 Hz, 2H), 6.76 (d, *J =* 5.6 Hz, 1H) 7.05 (s, 1H), 6.76 (d, *J* = 8.6 Hz, 1H), 4.40 (t, *J =* 5.6 Hz, 2H), 4.14 (t, *J =* 6.2 Hz, 1H), 3.92 - 3.88 (m, 5H), 2.95 (d, *J* = 4.8 Hz, 2H), 2.09 (t, *J =* 11.8 Hz, 1H), 1.98 - 1.90 (m, 1H), 1.84 (dd, *J =* 9.8, 6.4 Hz, 2H).

### Example 97

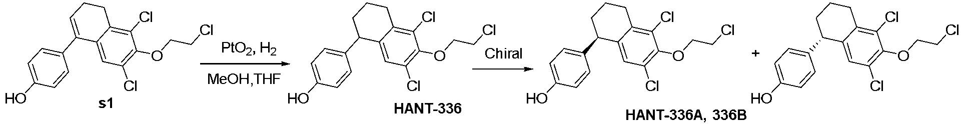

### Step 1: Synthesis of 4-(5,7-dichloro-6-(2-chloroethoxy)-1,2,3,4-tetrahydronaphthalen-1-yl)phenol

Compound **S1** (400 mg, 1.08 mmol) was dissolved in a mixed solution of methanol and THF (4.5 mL, 2 :1), then PtO₂ (40 mg, 0.1 eq) was added, and the reaction was stirred at room temperature under H₂ for 2.5 h. The reaction was filtered and concentrated under reduced pressure to obtain the crude product HANT-336 (150 mg, yield: 40.9%). LCMS: *m*/*z* =371.0 [M+H]⁺, which is subjected to SFC Chiral Separation (mobile phase: Hex-EtOH-95-5-20MIN) to afford two enantiomers HANT-336A (36 mg, retention time 8.810 min) and HANT- 336B (46.4 mg, retention time 10.433 min).

**HANT-336A:¹H** NMR (400 MHz, CDCl₃) δ 6.93 - 6.90 (m, 2H), 6.82 (s, 1H), 6.78 - 6.74 (m, 2H), 4.73 (s, 1H), 4.25 (t, *J* = 6.4 Hz, 2H), 3.99 - 3.94 (m, 1H), 3.87 (t, *J =* 6.4 Hz, 2H), 2.82 (td, *J* = 6.1, 2.5 Hz, 2H), 2.09 - 1.99 (m, 1H), 1.94 - 1.85 (m, 1H), 1.82- 1.71 (m, 2H).

**HANT-336B:** ¹H NMR (400 MHz, CDCl₃) δ 6.93 - 6.89 (m, 2H), 6.82 (s, 1H), 6.79 - 6.75 (m, 2H), 4.75 (s, 1H), 4.25 (t, *J =* 6.4 Hz, 2H), 3.96 (t, *J =* 6.2 Hz, 1H), 3.87 (t, *J =* 6.4 Hz, 2H), 2.82 (dt, *J* = 6.4, 2.9 Hz, 2H), 2.08 - 1.99 (m, 1H), 1.94 - 1.85 (m, 1H), 1.82 - 1.71 (m, 2H).

### Example 98

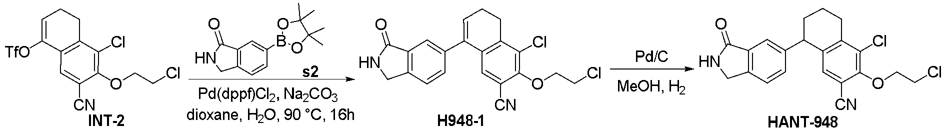

Synthesis was performed using methods described in Example **42** to afford the white solid compound **HANT-948** (46.6 mg, yield: 26.7%) at RT= 1.32 min (1.80 min). LCMS (ESI): *m*/*z* = 401.0 [M+H]⁺; ¹H NMR (400 MHz, DMSO-*d*₆) δ 8.56 (s, 1H), 7.52 (d, *J* = 7.8 Hz, 1H), 7.38 - 7.31 (m, 2H), 7.15 (s, 1H), 4.41 (t, *J =* 4.8 Hz, 2H), 4.35 - 4.32 (m, 3H), 3.97 (t, *J =* 4.8 Hz, 2H), 2.96-2.84 (m, 2H), 2.07-2.01 (m, 1H),1.89-1.76 (m, 3H).

### Example 99

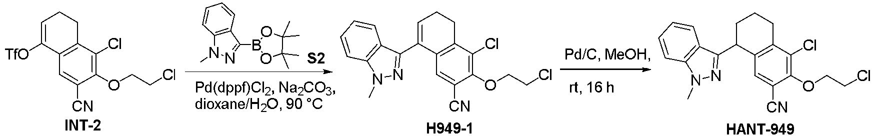

Synthesis was performed using methods described in Example **42** to afford **HANT-949** (46.3 mg, white solid), yield: 57.9 %. LCMS (ESI): *m*/*z* =402.1 [M+H]⁺; ¹H NMR (400 MHz, DMSO-*d₆*) δ 7.60 (d, *J =* 8.5 Hz, 1H), 7.49 (d, *J =* 8.2 Hz, 1H), 7.43 - 7.33 (m, 1H), 7.19 (s, 1H), 7.07-7.05 (m, 1H), 4.66 (t, *J* = 6.4 Hz, 1H), 4.40 (t, *J =* 4.8 Hz, 2H), 3.98 - 3.95 (m, 5H), 2.95-2.91 (m, 2H), 2.10 - 2.05 (m, 2H), 1.94-1.82 (m, 2H).

### Example 100

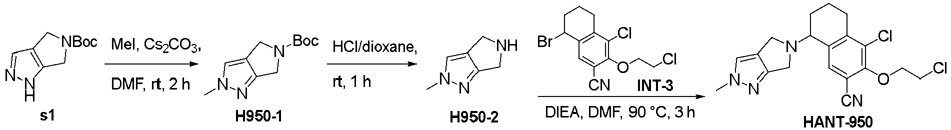

### Step 1: tert-Butyl 2-methyl-2,6-dihydropyrrolo[3,4-c]pyrazole-5(4H)-carboxylate

Compound S1 (5 g, 23.9 mmol) and iodomethane (4.4 g, 31.1 mmol) were dissolved in DMF (30 mL), followed by Cs₂CO₃ (15.5 g, 47.8 mmol). The reaction was stirred at room temperature for 2 hours, then poured into ethyl acetate for extraction. The organic phase was washed with saturated brine, dried with Na₂SO₄, filtered and concentrated under reduced pressure to obtain the crude product. The residue was purified by flash chromatography (PE/EA=1/1) to obtain a white solid compound **H950-1** (2 g, yield: 37.7%). LCMS (ESI): *m*/*z* =224.1[M+H]⁺; RT=1.313 min (1.80 min).

### Step 2: 2-Methyl-2,4,5,6-tetrahydropyrrolo[3,4-c]pyrazole

Compound **H950-1** (2 g, 8.9 mmol) was dissolved in dioxane (10 mL), then HCl/dioxane (11 mL, 44.5 mmol, 4.0 M) was added in and the reaction was stirred at room temperature for 1 hour. The reaction was concentrated under reduced pressure to afford crude H950-2 (1 g, yield: 100%) which was used directly in next step. LCMS (ESI): *m*/*z* =124.1[M+H]⁺; RT= 0.195 min (1.80 min).

### Step 3: 4-Chloro-3-(2-chloroethoxy)-8-(2-methyl-2,6-dihydropyrrolo[3,4-c]pyrazol-5(4H)-yl)-5,6,7,8-t etrahydronaphthalene-2-carbonitrile

Compound **INT-3** (70 mg, 0.2005 mmol) was dissolved in DMF (5 mL) and **H950-2** (49 mg, 0.401 mmol) and DIEA (77 mg, 0.6015 mmol) were added and the reaction was stirred at 90 °C for 3 hours. The organic phase was extracted with ethyl acetate, washed with saturated brine, dried over Na₂SO₄ and purified by prep-HPLC to obtain compound **HANT-950** (6 mg, white solid), yield: 7.3%. LCMS (ESI): *m*/*z* = 391.1 [M+H]⁺; RT= 1.11 min (1.80 min). ¹H NMR (400 MHz, DMSO-*d*₆) δ 7.87 (s, 1H), 7.35 (s, 1H), 4.50 - 4.35 (m, 2H), 4.03 - 3.91 (m, 3H), 3.78-3.75 (m, 5H), 3.59-3.57 (m, 2H), 2.87 - 2.69 (m, 2H), 2.10-2.06 (m 1H), 1.86-1.71 (m, 3H).

### Example 101

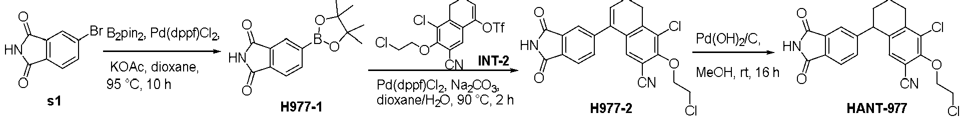

Synthesis was performed using methods described in Example **51** to afford **HANT-977** (11.4 mg, white solid), yield: 14.2 %. LCMS (ESI): *m*/*z* = 415.0 [M+H]⁺; ¹H NMR (400 MHz, DMSO-*d*₆) δ 11.28 (s, 1H), 7.77 (d, *J =* 7.6 Hz, 1H), 7.54-7.52 (m, 2H), 7.18 (s, 1H), 4.46 - 4.29 (m, 3H), 3.97 (t, *J =* 5.2 Hz, 2H), 3.01 - 2.78 (m, 2H), 2.08-2.05 (m, 1H), 1.93- 1.54 (m, 3H).

### Example 102

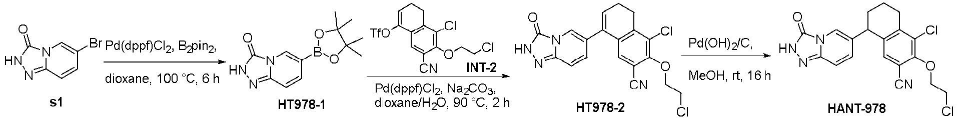

Synthesis was performed using methods described in Example **51** to afford **HANT-978** (12.1 mg, white solid), yield: 13.11 %. LCMS (ESI): *m*/*z=* 403.1 [M+H]⁺; ¹H NMR (400 MHz, DMSO-*d*₆) δ 12.00 (s, 1H), 7.48 (s, 1H), 7.40 (s, 1H), 7.17 (d, *J* = 9.7 Hz, 1H), 6.92 (dd, *J* = 9.7, 1.5 Hz, 1H), 4.40 (t, *J* = 4.7, 1.5 Hz, 1H)7.17 (d, *J =* 9.7 Hz, 1H), 6.92 (dd, *J* = 9.7, 1.5 Hz, 1H), 4.40 (t, *J* = 4.8 Hz, 2H), 4.18 - 4.04 (m, 1H), 3.97 (t, *J =* 5.2 Hz, 2H), 3.00 - 2.75(m, 2H), 2.03 - 1.65 (m, 4H).

### Example 103

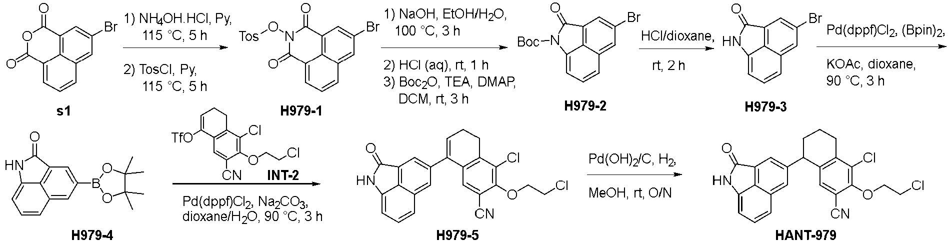

### Step 1: 5-Bromo-1,3-dioxo-1H-benzo[de]isoquinoline-2(3H)-4-methylbenzenesulfonate

Compound **S1** (2 g, 7.2 mmol) and hydroxylamine hydrochloride (500 mg, 7.2 mmol) were dissolved in pyridine (15 mL) and refluxed at 115 °C for 5 h. The reaction was cooled to 80 °C, then TosCl (2.75 g, 14.4 mmol) was added and the reaction was reheated to reflux at 115 °C for 5 h. The reaction was cooled to room temperature, then water (100 mL) was added, the the reaction was stirred at room temperature until solid precipitated. The crude mixture was filtered, and the resulting filter cake was washed with water and dried to obtain **H979-1** (brown solid) and was used directly in next step. LCMS (ESI): *m*/*z* =446.0 [M+H]⁺.

### Step 2: tert-Butyl 4-bromo-2-oxobenz[cd]indole-1(2H)-carboxylate

To a mixture of **H979-1** (2 g, 4.5 mmol) and ethanol/water (6 mL/8 mL) was slowly added an aq. NaOH (1.34 g, 33.6 mmol, 24 mL) and refluxed at 100 °C for 3 h. The reaction was cooled to room temperature, followed by addition of concentrated hydrochloric acid (36%, 4 mL) and stirred for 1 h. The resulting solid precipitator was filtered, and the filter cake was washed with water and dried. The filter cake was dissolved in dichloromethane (30 mL), then Boc₂O (2.2 g, 10.1 mmol), TEA (2.04 g, 20.2 mmol) and DMAP (60 mg, 0.5 mmol) were added and stirred at room temperature for 3 hours. The crude reaction was concentrated under reduced pressure and the resulting residue was purified by flash chromatography (PE/EA=3/1) to afford the yellow solid compound **H979-2** (1.3 g, total yield: 52%). LCMS (ESI): *m*/*z* = 292.0 [M+H-56]⁺.

### Step 3: 4-Bromobenzo[cd]indol-2(1H)-one

Compound **H979-2** (1.3 g, 3.7 mmol) was dissolved in dioxane (15 mL) and HCl in dioxane (3.7 mL, 14.8 mmol, 4.0 M) was added and reacted for 2 h at room temperature. The reaction was concentrated under reduced pressure to obtain the crude product **H979-3** (800 mg, yield: 73%), which was directly used in next step. LCMS (ESI): *m*/*z* =248.0[M+H]⁺.

### Step 4: 4-(4,4,5,5-tetramethyl-1,3,2-dioxaborolan-2-yl)benzo[cd]indol-2(1H)-one

Compounds **H979-3** (330 mg, 1.33 mmol) and (Bpin)₂ (675 mg, 2.66 mmol) were dissolved in dioxane (20 mL), then Pd(dppf)Cl₂ (97 mg, 0.133 mmol) and KOAc (392 mg, 3.99 mmol) were added, and the reaction was heated to 90°C for 3 hours. The reaction was filtered and concentrated under reduced pressure to obtain the crude product **H979-4** (black oil) and used directly in next step. LCMS (ESI): *m*/*z* =296.2 [M+H]⁺.

### Step 5: 4-Chloro-3-(2-chloroethoxy)-8-(2-oxo-1,2-dihydrobenzo[cd]indol-4-yl)-5,6-dihydronaphthalen e-2-carbonitrile

**INT-2** (300 mg, 0.721 mmol) and crude **H979-4** (319 mg, 1.08 mmol) were dissolved in a mixture of 20 mL of dioxane and 4 mL of water, then Na₂CO₃ (229 mg, 2.16 mmol) and Pd(dppf)Cl₂ (53 mg, 0.0721 mmol) were added to the reaction, and the reaction was stirred at 90 °C under N₂ for 3 h. The reaction was cooled to room temperature, then water was added in and the mixture was extracted with ethyl acetate. The organic phase was washed with saturated brine, dried over Na₂SO₄, filtered and concentrated under reduced pressure. The resulting residue was purified by flash chromatography (PE/EA=1/1) to obtain compound **H979-5** (120 mg, yellow solid) in 36.3% yield. LCMS (ESI): *m*/*z* = 435.2 [M+M+S].435.2 [M+H]⁺.

### Step 6: 4-Chloro-3-(2-chloroethoxy)-8-(2-oxo-1,2-dihydrobenzo[cd]indol-4-yl)-5,6,7,8-tetrahydronapht halene-2-carbonitrile

Compound **H979-5** (100 mg, 0.230 mmol) was dissolved in 15 mL of methanol, then Pd(OH)₂/C (50 mg, 50% wt) was added, and the reaction was stirred overnight at room temperature under H₂. The reaction was filtered and concentrated under reduced pressure, and the residue was purified by prep-HPLC to obtain compound **HANT-979** (8.6 mg, white solid), yield: 8.14 %. LCMS (ESI): *m*/*z* =437.1[M+H]⁺; ¹H NMR (400 MHz, DMSO-*d*₆) δ 10.74 (s, 1H), 7.86 (s, 1H), 7.76(s, 1H), 7.56 - 7.42 (m, 2H), 7.20 (s, 1H), 6.94 (d, *J =* 6.5 Hz, 1H), 4.53 (t, *J =* 6.6 Hz, 1H), 4.47 - 4.37 (m, 2H), 4.01 -3.93 (m, 2H), 3.05 - 2.87 (m, 2H), 2.19 - 2.07 (m, 1H), 2.01 - 1.72 (m, 3H).

### Example 104

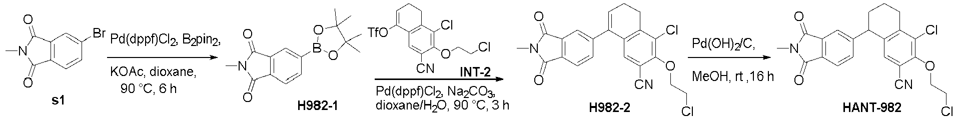

Synthesis was performed using methods described in Example **51** to afford **HANT-982** (12.8 mg, white solid, yield: 18.61 %). LCMS (ESI): *m*/*z* = 429.0 [M+H]⁺; ¹H NMR (400 MHz, DMSO-*d*₆) δ 7.80 (d, *J* = 7.7 Hz, 1H), 7.59 (s, 1H), 7.53 (dd, *J* = 7.7, 1.4 Hz, 1H), 7.15 (s, 1H), 4.50 - 4.35 (m, 3H), 4.02 - 3.92 (m, 2H), 3.02 (s, 3H), 2.98 - 2.85 (m, 2H), 2.13 - 2.01 (m, 1H), 1.94 - 1.68 (m, 3H).

### Example 105

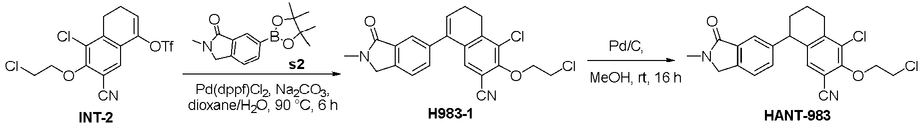

Synthesis was performed using methods described in Example **42** to afford white solid compound **HANT-983** (24.1 mg, yield: 23.9%). LCMS (ESI): *m*/*z* = 415.1 [M+H]⁺; RT= 1.33 min (1.80 min). ¹H NMR (400 MHz, DMSO-*d*₆) δ 7.53 (d, *J =* 8.3 Hz, 1H1H), 7.35-7.32 (m, 2H), 7.11 (s, 1H), 4.43 (s, 2H), 4.41 (t, *J* = 4.8 Hz, 2H), 4.33 (t, *J =* 6.0 Hz, 1H), 3.97 (t, *J* = 5.2 Hz, 2H), 3.06 (s, 3H), 2.93-2.88 (m, 2H), 2.10- 2.01 (m, 1H), 1.87-1.75 (m, 3H).

### Biological Tests:

### Androgen receptor (AR) reporter gene assay

**Experimental reagents:** 11-Ketodihydrotestosterone (11-KDHT) was purchased from MCE (cat.HY-135794); Enzalutamide (MDV3100) was purchased from Selleck (cat.S1250); Bright-Lite Luciferase Assay kit was purchased from Vazyme (cat.DD1204-01);

### Experimental Methods:

1. dissolve the described compounds in 100% DMSO at a final concentration of 10 mM. 100 nL each of 400X compound to be tested and 400X 11-KDHT (40 nM) were added to a 384-well assay plate with Echo, followed by 20 µL of complete medium, and centrifuged at 1000 rpm for 1 min, and oscillated for 20-30 min at room temperature.
2. 20 µL of AR+ARE/Luc HEK293T cells (10,000 cells/well) were inoculated into the assay plate containing the compounds and 11-KDHT, and centrifuged at 1000 rpm for 1 min. The assay plate was incubated in a 37 °C CO₂ incubator for 24h.
3. 40 µL of Bright-Lite assay reagent was transferred to each well of the assay plate, centrifuged at 1000 rpm for 1 min, and shaken for 2 min at room temperature protected from light. Luminescence values (RLU) were measured using Envision. Based on the results, the IC₅₀ values of the compounds described were calculated.

**Table 1 HEK293-Luc% inhibition IC₅₀ of representative compounds**

| **No.** | **HEK293 Luc IC₅₀ (nM)** | **No.** | **HEK293 Luc IC₅₀ (nM)** | **No.** | **HEK293 Luc IC₅₀ (nM)** |
|---|---|---|---|---|---|
| HANT-100 | **** | HANT-101 | ** | HANT-112 | *** |
| HANT-102 | *** | HANT-103 | *** | HANT-113 | *** |
| HANT-105 | **** | HANT-107 | ** | HANT-114 | **** |
| HANT-106 | **** | HANT-108 | **** | HANT-115 | **** |
| HANT-109 | ** | HANT-110 | **** | HANT-117 | ** |
| HANT-118 | **** | HANT-123 | *** | HANT-124 | * |
| HANT-121 | **** | HANT-125 | **** | HANT-131 | * |
| HANT-122 | **** | HANT-126 | *** | HANT-133 | ** |
| HANT-134 | **** | HANT-127 | *** | HANT-132 | * |
| HANT-135 | **** | HANT-146 | **** | HANT-164 | * |
| HANT-137 | ** | HANT-148 | *** | HANT-165 | * |
| HANT-144A | * | HANT-149 | **** | HANT-166 | *** |
| HANT-144B | **** | HANT-150B | * | HANT-170 | *** |
| HANT-173 | **** | HANT-150A | **** | HANT-171 | ** |
| HANT-175 | ** | HANT-187 | **** | HANT-194 | **** |
| HANT-183 | ** | HANT-188 | *** | HANT-196 | **** |
| HANT-184 | *** | HANT-191 | **** | HANT-200 | *** |
| HANT-186 | **** | HANT-192 | **** | HANT-202 | **** |
| HANT-204 | **** | HANT-193 | **** | HANT-203 | **** |
| HANT-205 | **** | HANT-204A | * | HANT-204B | **** |
| HANT-206 | **** | HANT-207 | *** | HANT-209 | *** |
| HANT-213 | ** | HANT-208 | **** | HANT-212 | **** |
| HANT-218 | **** | HANT-214 | *** | HANT-215 | **** |
| HANT-225 | **** | HANT-220 | * | HANT-224 | **** |
| HANT-226 | **** | HANT-228 | * | HANT-227 | **** |
| HANT-233 | **** | HANT-229 | **** | HANT-232 | **** |
| HANT-239 | *** | HANT-235 | *** | HANT-234 | **** |
| HANT-246 | **** | HANT-241 | **** | HANT-240 | *** |
| HANT-247 | **** | HANT-248 | **** | HANT-251 | **** |
| HANT-250 | **** | HANT-249 | *** | HANT-255 | **** |
| HANT-254 | **** | HANT-252 | **** | HANT-257 | **** |
| HANT-262 | **** | HANT-256 | ** | HANT-261 | *** |
| HANT-266 | **** | HANT-263 | **** | HANT-264 | * |
| HANT-269 | **** | HANT-267 | **** | HANT-268 | **** |
| HANT-336A | *** | HANT-334 | **** | HANT-335 | **** |
| HANT-336B | **** | HANT-948 | *** | HANT-949 | ** |
| HANT-950 | ** | HANT-977 | **** | HANT-978 | **** |
| HANT-979 | *** | HANT-982 | **** | HANT-983 | * |

| | | | | | |
|---|---|---|---|---|---|
| Annotation: ****: 1-500 nM, ***: 0.5-1 µM, **: 1-2 µM, *: >2 µM. | | | | | |

## Claims

1. A compound of formula (I), or a stereoisomer, a geometrical isomer, a tautomer, a solvate, a hydrate or a pharmaceutically acceptable salt thereof, 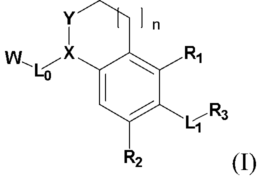
wherein, n is 0 or 1;
X is N or CH;
Y is NR₄ or CHR₄;
L₀ is a bond, O, S, -NR₅-, -C(O)-NR₅-, -NR₅-C(O)- or -NR₅-(CH₂)ₘ-; m is 1 or 2; preferably, L₀ is a bond, O, S, -NR₅-, -NH-C(O)-, -C(O)-NH-, -NR₅-(CH₂)₂- or -NR₅-CH₂-; more preferably, L₀ is a bond, O, S, -NR₅-, -NHC(O)- or -C(O)-NH-;
L₁ is a bond, O, S or -NR₅-; preferably, L₁ is a bond, O or S;
R₁, R₂ are each independently selected from H, C₁-C₆ alkyl, halogen, haloC₁-C₆ alkyl, CN, C₁-C₆ alkoxy, -C₁-C₆ alkyl-NR₆R₇ and -C(O)-NR₆R₇; preferably, R₁, R₂ are each independently selected from H, halogen, CF₃, CN, C₁-C₆ alkyl, C₁-C₆ alkoxy, -CH₂-NH₂ and -C(O)NH-CH₃;
wherein R₄, R₅, R₆, R₇ are each independently selected from H, C₁-C₆ alkyl and acyl;
R₃ is C₁-C₆ alkyl, C₃-C₈ cycloalkyl, C₆-C₁₂ aryl, 5- to 12-membered heteroaryl or 4- to 12-membered heterocycloalkyl, and the alkyl, the cycloalkyl, the aryl, the heteroaryl and the heterocycloalkyl are each optionally substituted with one or more substituents independently selected from halogen and hydroxyl; preferably, R₃ is C₁-C₆ alkyl, C₃- C₇ cycloalkyl, C₆-C₁₀ aryl, 5- to 10-membered heteroaryl or 5- to 7-membered heterocycloalkyl, each of which is optionally substituted with one or more substituents selected from halogen and hydroxyl; more preferably, R₃ is C₁-C₆ alkyl, C₃-C₇ cycloalkyl, C₆-C₁₀ aryl, 5- to 10-membered heteroaryl or 5-to 7-membered heterocycloalkyl, each of which is optionally substituted with one or more F, Cl or OH;W is C₅-C₈ cycloalkyl, C₆-C₁₂ aryl, 5- to 12-membered heteroaryl or 4- to 12-membered heterocycloalkyl, each of which is optionally substituted with one or more substituents independently selected from halogen, CN, oxo, -NR₇R₈, -OR₈, -C(O)-NHR₈, R₉, -OC(O)-NHR₉, -NHC(O)-R₁₀, -SO₂R₉, -SO₂NHR₈, -NR₇SO₂R₉, -NR₇SO₂NR₈R₁₀, substituted or unsubstituted C₃-C₈ cycloalkyl, 4- to 8-membered heterocycloalkyl, C₆-C₁₂ aryl and 5- to 10-membered heteroaryl; wherein the C₃-C₈ cycloalkyl, the 4- to 8-membered heterocycloalkyl, the C₆-C₁₂ aryl and the 5- to 10-membered heteroaryl are each optionally further substituted with one or more substituents selected from halogen, oxo, NH₂, hydroxyls, C₁-C₄ alkyl and C₁-C₄ alkoxy;
wherein R₈ is H or is C₁-C₆ alkyl optionally substituted with one or more substituents selected from halogen and hydroxyl; preferably, R₈ is H or C₁-C₆ alkyl optionally substituted with F or OH;
R₉ is C₁-C₆ alkyl optionally substituted with one or more substituents selected from halogen, hydroxyl and NH₂;
R₁₀ is C₁-C₆ alkyl or C₁-C₆ alkoxy;
preferably, the heterocycloalkyl comprises 1-2 heteroatoms selected from N, NR_{c}, O and S(O)ₚ, and R_{c} is each independently selected from hydrogen and C₁-C₄ alkyl, and p is 1 or 2.

2. The compound of formula (I), or the stereoisomer, the geometrical isomer, the tautomer, the solvate, the hydrate or the pharmaceutically acceptable salt thereof as said in claim 1, wherein W is C₅-C₈ cycloalkyl, C₆-C₁₂ aryl, 5- to 10-membered heteroaryl or 4- to 12-membered heterocycloalkyl, each of which is optionally substituted with one or more substituents independently selected from -OH, F, Cl, CN, oxo, -NH₂, C₁-C₆ alkyl, C₁-C₆ alkoxy, haloC₁-C₆ alkyl, haloC₁-C₆ alkoxy, -(C₁-C₆ alkyl)OH, -(C₁-C₆ alkoxy)OH, -NH(C₁-C₃ alkyl), -NH(C₁-C₄ alkyl)OH, -NHC(O)(C₁-C₃ alkyl), -NHC(O)(C₁-C₄ alkoxy), -OC(O)NH(C₁-C₃ alkyl), -C(O)NH(C₁-C₃ alkyl), -O(C₁-C₄ alkyl)OH, -C(O)NH₂, -SO₂NH₂, -SO₂(C₁-C₄ alkyl), -SO₂(C₁-C₃ alkyl)NH₂, -SO₂NH(C₁-C₄ alkyl), -NHSO₂(C₁-C₄ alkyl), -N(CH₃)SO₂(C₁-C₃ alkyl), -NHSO₂(C₁-C₃ alkyl)NH₂, -NHSO₂CF₃, C₃-C₇ cycloalkyl, 5- to 8-membered heterocycloalkyl, C₆-C₁₀ aryl, and 5- to 10-membered heteroaryl; wherein the C₃-C₇ cycloalkyl, the 5- to 8-membered heterocycloalkyl, the C₆-C₁₀ aryl and the 5- to 10-membered heteroaryl are each optionally substituted with one or more substituents independently selected from halogen, OH, NH₂, C₁-C₄ alkyl, and C₁-C₄ alkoxy.

3. The compound of formula (I), or the stereoisomer, the geometrical isomer, the tautomer, the solvate, the hydrate or the pharmaceutically acceptable salt thereof as said in claim 1, wherein W is C₅-C₈ cycloalkyl, C₆-C₁₂ aryl, 5- to 10-membered heteroaryl, or 4- to 12-membered heterocycloalkyl, each of which is optionally substituted with one or more substituents independently selected from: -OH, F, Cl, CN, oxo, -NH₂, -NHCH₃, -NH(CH₂)₂OH, C₁-C₆ alkyl, C₁-C₆ alkoxy, haloC₁-C₆ alkyl, haloC₁-C₆ alkoxy, -NHC(O)CH₃, -NHC(O)OCH₃, -OC(O)NH-CH₃, -C(O)NH-CH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentyl, cyclohexyl, pyridinyl, phenyl, morpholinyl, 1,3-oxazinylalkyl, tetrahydrofuranyl, hexahydropyrimidinyl, piperazinyl, pyrrole, imidazole, pyrazole, 4-methylpiperazin-1-yl, piperidinyl and 4-hydroxy-piperidin-1-yl.

4. The compound of formula (I), or the stereoisomer, the geometrical isomer, the tautomer, the solvate, the hydrate or the pharmaceutically acceptable salt thereof as said in claim 1, wherein W is cyclopentane, cyclohexane, phenyl, pyrrole, pyrazole, imidazole, thiazole, oxazole, isoxazole, oxadiazole, pyridine, pyrimidine, pyrazine, pyridazine, tetrahydrofuran, pyrrolidine, pyrazolidine, imidazolidine, tetrahydropyran, piperidine, piperazine, hexahydropyrimidine, morpholine, octahydropyrrolo[3,2-b]pyrrole, indole, benzopyran, benzimidazole, benzoxazole, benzotriazole, 4-azaindole, 5-azaindole, 6-azaindole, 7-azaindole, quinoline, isoquiline, quinazoline, cinnoline, quinoxaline, naphthyl, indenyl, 1H-pyrazolo[4,3-b]pyridine, 1*H*-pyrazolo[4,3-c]pyridine, 1H-pyrazol[3,4-c]pyridine, 1H-pyrazolo[3,4-b]pyridine, 2H-pyrazolo[3,4-b]pyridine, 2,3-dihydro-oxazolo[4,5-b]pyridine, isoindoline, indoline, dihydrobenzofuran, 2,3-dihydro-1H-benzo[d]imidazole, 2,3-dihydro-benzo[d]oxazole, 2,3-dihydrobenzo[d]thiazole, 2,3-dihydro-1H-indazole, 1,2 dihydroquinoline, tetrahydroquinoline, tetrahydroisoquinoline, tetrahydronaphthalene, dihydroindene, chromane, isochromane, dihydrobenzofuran, dihydroisobenzofuran, 1,2-dihydro-1,8-naphthyridine, oxazolo[4,5-b]pyridine, pyridin-2(1H)-one, 2-indolinone, 2-benzoxazolone, quinolin-2(1H)-one, 1,4-dihydro-3(2H)-isoquinolinone, 1,3-dihydrobenzimidazol-2-one, 2,3-dihydrochromen-4-one, 5,8-dihydro-6H-[1,6]naphthyridin-7-one, 7,8-dihydro-6*H*-[1,6]naphthyridin-5-one, 1,8-naphthyridin-2(1*H*)-one, 3a,4,5,6,7,7a-hexahydro-1H-pyrazolo[4,3-c]pyridine, 3a,4,5,6,7,7a-hexahydro-1H-pyrazolo[3,4-c]pyridine, 2H-chromene, oxazolo[5,4-c]pyridin-2(1*H*)-one or oxazolo[4,5-b]pyridin-2(3H)-one, each of which is optionally substituted with one or more substituents independently selected from the group consisting of: halogen, CN, oxo, -NR₇R₈, hydroxy, C₁-C₆ alkyl, C₁-C₆ alkoxy, haloC₁-C₆ alkyl, haloC₁-C₆ alkoxy, hydroxy-C₁-C₆ alkyl, hydroxy-C₁-C₆ alkoxy, hydroxy-C₁-C₆ alkyl-NR₈-, -C(O)-NHR₈, -OC(O)-NHR₉, -NHC(O)-R₁₀, -SO₂R₉, -SO₂NHR₈, -NR₇SO₂R₉, - NR₇SO₂NHR₈, C₃-C₈ cycloalkyl, 4- to 8-membered heterocycloalkyl, C₆-C₁₂ aryl and 5- to 10-membered heteroaryl, wherein the C₃-C₈ cycloalkyl, the 4- to 8-membered heterocycloalkyl, the C₆-C₁₂ aryl and the 5- to 10-membered heteroaryl are each optionally further substituted with one or more substituents of halogen, oxo, NH₂, hydroxy, C₁-C₄ alkyl or C₁-C₄ alkoxy;
wherein, R₈ is H, or C₁-C₆ alkyl optionally substituted with one or more substituents of halogen or hydroxyl;
R₉ is C₁-C₆ alkyl optionally substituted with one or more substituents of halogen or NH₂;
R₁₀ is C₁-C₆ alkyl or C₁-C₆ alkoxy.

5. The compound of formula (I), or the stereoisomer, the geometrical isomer, the tautomer, the solvate, the hydrate or the pharmaceutically acceptable salt thereof as said in claim 1, wherein W is cyclopentane, cyclohexane, phenyl, pyrrole, pyrazole, imidazole, thiazole, oxazole, isoxazole, oxadiazole, pyridine, pyrimidine, pyrazine, pyridazine, tetrahydrofuran, pyrrolidine, pyrazolidine, imidazolidine, tetrahydropyran, piperidine, piperazine, hexahydropyrimidine, morpholine, octahydropyrrolo[3,2-b]pyrrole, indole, benzopyran, benzimidazole, benzoxazole, benzotriazole, 4-azaindole, 5-azaindole, 6-azaindole, 7-azaindole, quinoline, isoquinoline, quinazoline, cinnoline, quinoxaline, naphthyl, indenyl, imidazolopyridine, 1*H*-pyrazolo[4,3-b]pyridine, 1*H*-pyrazolo[4,3-c]pyridine, 1*H*-pyrazolo[3,4-c]pyridine, 1*H*-pyrazolo[3,4-b]pyridine, 2*H*-pyrazolo[3,4-b]pyridine, 2,3-dihydrooxazolo[4,5-b]pyridine, isoindoline, indoline, dihydrobenzofuran, 2,3-dihydro-1*H*-benzo[d]imidazole, 2,3-dihydrobenzo[*d*]oxazole, 2,3-dihydrobenzo[*d*]thiazole, 2,3-dihydro-1*H*-indazole, 1,2-dihydroquinoline, tetrahydroquinoline, tetrahydroisoquinoline, tetrahydronaphthalene, dihydroindene, chroman, isochroman, dihydrobenzofuran, dihydroisobenzofuran, 1,2-dihydro-1,8-naphthyridine, oxazolo[4,5-*b*]pyridine, pyridin-2(1*H*)-one, 2-indolone, 2-benzoxazolone, quinolin-2(1*H*)-one, 1,4-dihydro-3(2*H*)-isoquinolone, 1,3-dihydrobenzimidazol-2-one, 2,3-dihydrochromen-4-one, 5,8-dihydro-6*H*-[1,6]naphthyridin-7-one, 7,8-dihydro-6*H*-[1,6]-naphthyridin-5-one, 1,8-naphthyridin-2(1*H*)-one, 3a,4,5,6,7,7a-hexahydro-1*H*-pyrazolo[4,3-c]pyridine, 3a,4,5,6,7,7a-hexahydro-1*H*-pyrazolo[3,4-*c*]pyridine, 2*H*-chromene, oxazolo[5,4-c]pyridin-2(1*H*)-one or oxazolo[4,5-b]pyridin-2(3*H*)-one, each of which is optionally substituted with one or more substituents independently selected from the group consisting of: -OH, F, Cl, CN, oxo, -NH₂, -NHCH₃, -NH(CH₂)₂OH, C₁-C₆ alkyl, C₁-C₆ alkoxy, haloC₁-C₆ alkyl, haloC₁-C₆ alkoxy, -NHC(O)CH₃, -NHC(O)OCH₃, -OC(O)NH-CH₃, -C(O)NH-CH₃, -C(O)NH₂, -O(CH₂)₂OH, -SO₂NH₂, -SO₂(CH₂)₂NH₂-, -NHSO₂CH₃, -N(CH₃)SO₂CH₃, -NHSO₂CH₂CH₃, -NHSO₂CH(CH₃)₂, -NHSO₂CH₂NH₂, -NHSO₂(CH₂)₂NH₂, -NHSO₂CF₃, -SO₂CH₃, -SO₂CH(CH₃)₂, -SO₂CH₂CH₃, cyclopentane, cyclohexane, pyridinyl, phenyl, morpholinyl, 1,3-oxazinylalkyl, tetrahydrofuranyl, hexahydropyrimidinyl, piperazinyl, pyrrole, imidazole, pyrazole, 4-methyl-piperazin-1-yl, piperidinyl and 4-hydroxy-piperidin-1-yl.

6. The compound of formula (I), or the stereoisomer, the geometrical isomer, the tautomer, the solvate, the hydrate or the pharmaceutically acceptable salt thereof as said in any claim of 1-5, wherein
n is 0 or 1;
X is N or CH;
Y is NR₄ or CHR₄, wherein R₄ is selected from H and C₁-C₆ alkyl;
L₀ is a bond, O, -NR₅-, -C(O)-NR₅- or -NR₅-C(O)-; R₅ is H or C₁-C₆ alkyl;
L₁ is a bond or O;
R₁, R₂ are each independently selected from H, C₁-C₆ alkyl, halogen, CN, -C₁-C₆ alkyl-NR₆R₇ and -C(O)-NR₆R₇, wherein R₆, R₇ are each independently selected from H and C₁-C₆ alkyl;
R₃ is C₁-C₆ alkyl or haloC₁-C₆ alkyl; or R₃ is 5- or 6-membered heterocycloalkyl optionally substituted with one or more substituents selected from halogen and hydroxyl, e.g. piperazinyl, 4-methyl-piperazin-1-yl, piperidinyl or 4-hydroxy-piperidin-1-yl.

7. The compound of formula (I), or the stereoisomer, the geometrical isomer, the tautomer, the solvate, the hydrate or the pharmaceutically acceptable salt thereof as said in claim 1, wherein the compound is selected from:

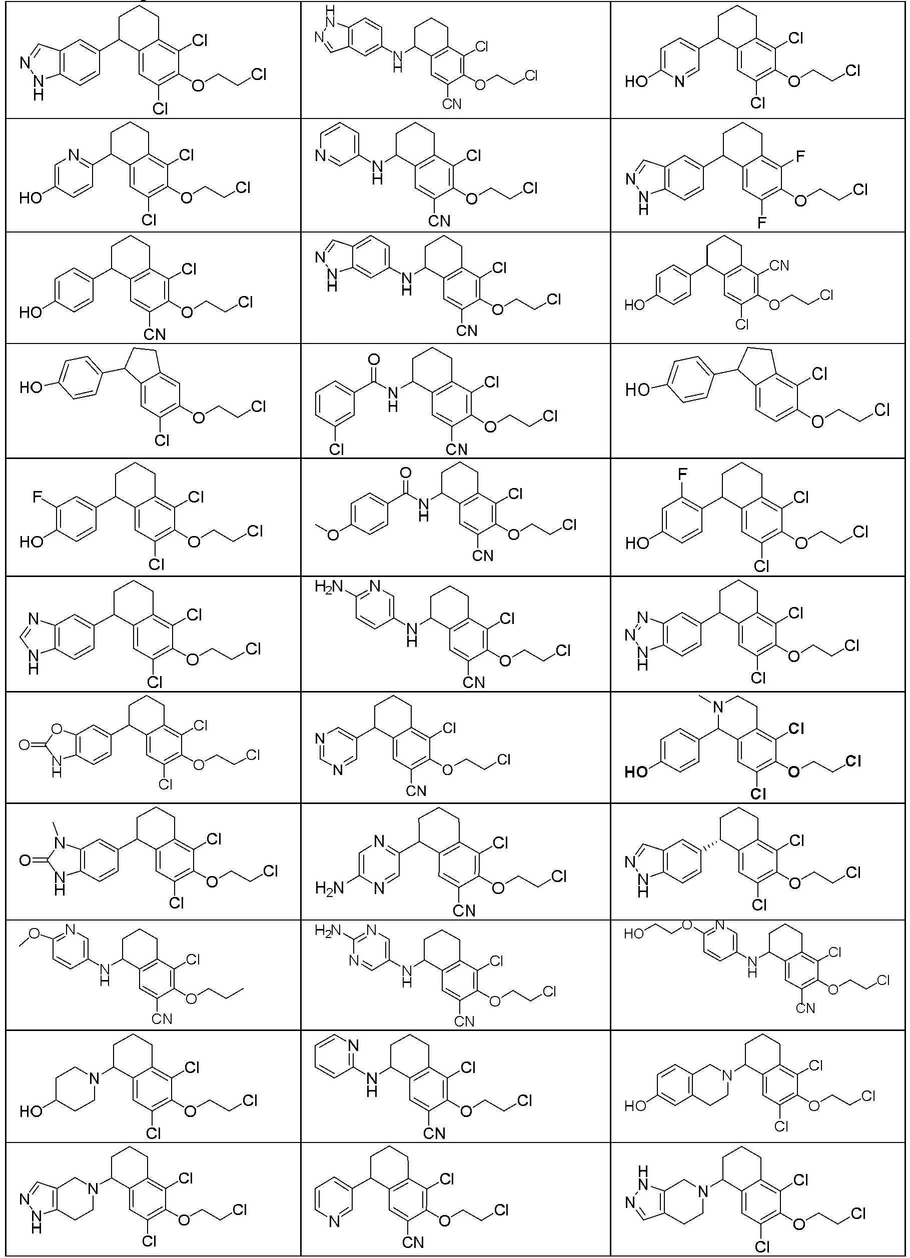

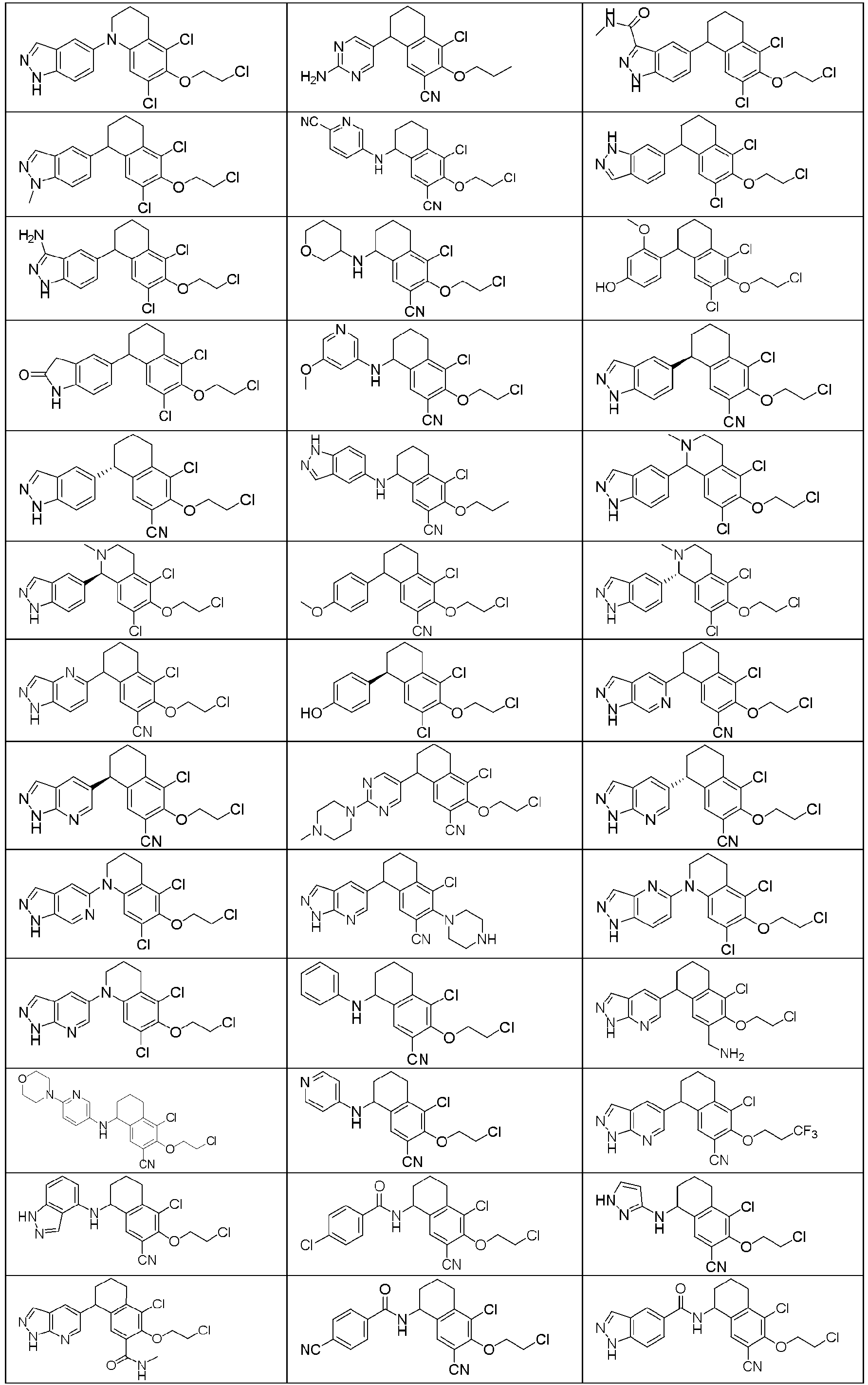

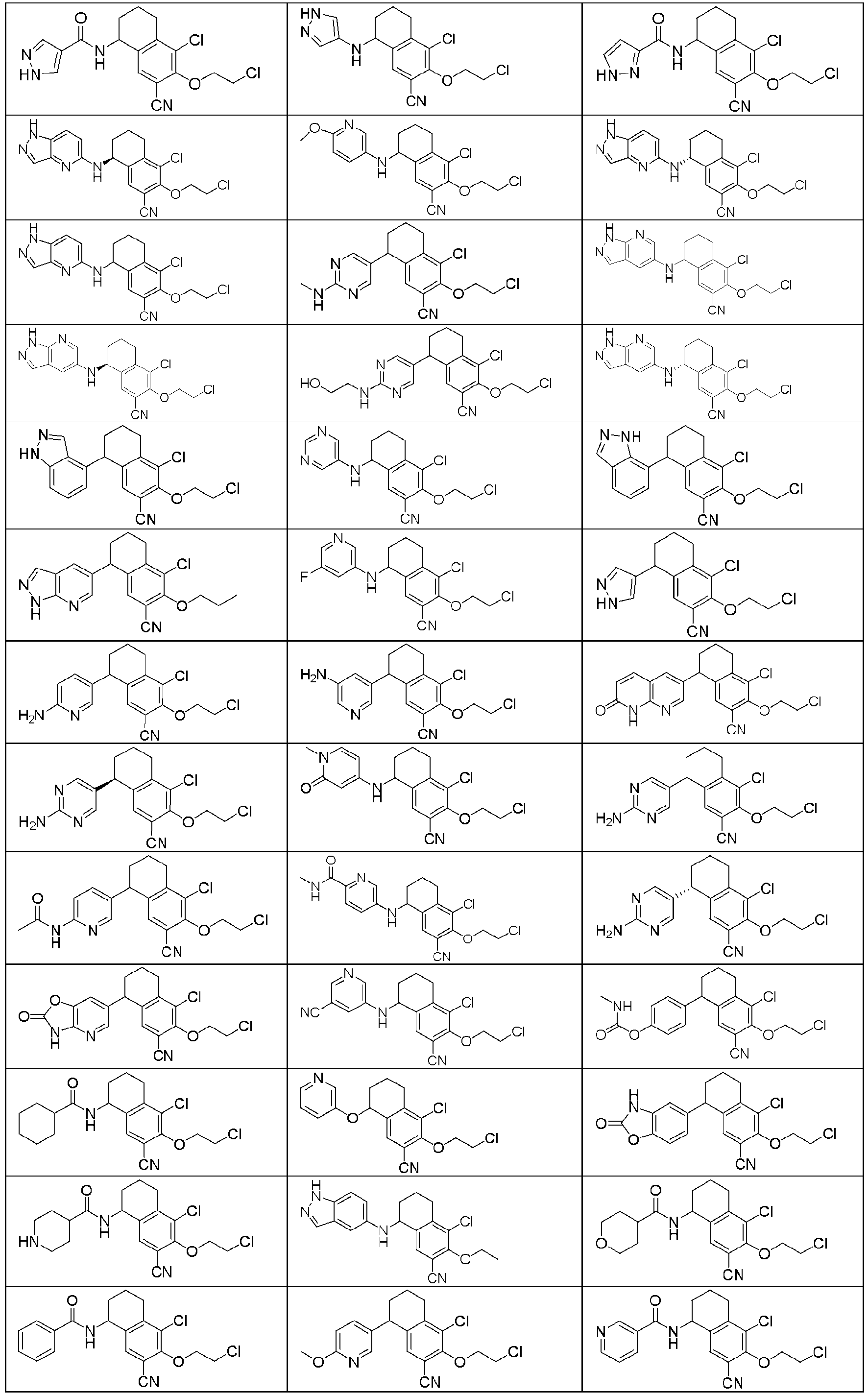

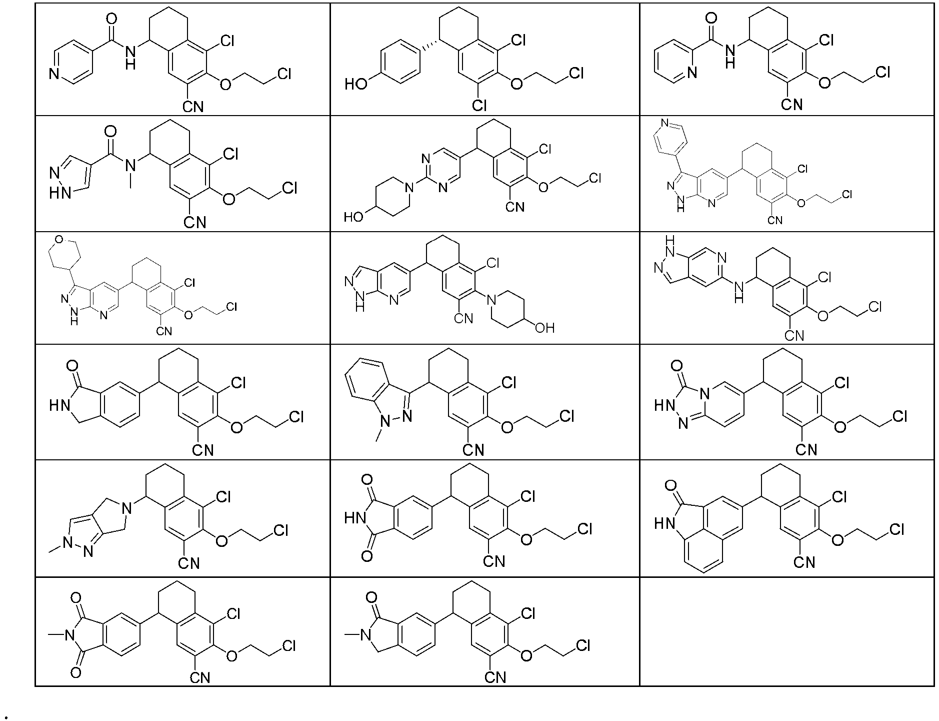

8. A pharmaceutical composition comprising a therapeutically effective amount of the compound as said in any claim of 1-7, or the stereoisomer, the geometrical isomer, the tautomer, the solvate, the hydrate, or the pharmaceutically acceptable salt thereof, and pharmaceutically acceptable excipient.

9. The compound as said in any claim of 1-7 or the stereoisomer, the geometrical isomer, the tautomer, the solvate, the hydrate, or the pharmaceutically acceptable salt thereof, which is used as a drug.

10. Use of the compound as said in any claim of 1-7, or the stereoisomer, the geometrical isomer, the tautomer, the solvate, the hydrate, or the pharmaceutically acceptable salt thereof, in the preparation of drugs for use in the prevention, treatment, or alleviation of disorders or diseases caused by abnormal androgen activity in patients.

11. The use as said in claim 10, the disorders or diseases selected from prostate cancer, other prostate disorders (prostatic hyperplasia, prostatitis, etc.), breast cancer, acne, hirsutism, hidradenitis suppurativa, androgenetic alopecia, cryptorchidism, androgen insensitivity and Kennedy's disease.

12. A method of treating or preventing androgen receptor-mediated diseases or disorders, comprising administering to patient in need of such treatment an effective amount of the compound as said in any claim of 1-7, or the stereoisomer, the geometrical isomer, the tautomer, the solvate, the hydrate, or the pharmaceutically acceptable salt thereof.

13. A method of treating or preventing androgen receptor-mediated diseases or disorders, comprising administering to patient in need of such treatment an effective amount of the compound as said in any claim of 1-7, or the stereoisomer, the geometrical isomer, the tautomer, the solvate, the hydrate, or the pharmaceutically acceptable salt thereof and one or more other active agents.

14. The method as said in claim 12 or 13, the disorders or diseases selected from prostate cancer, other prostate disorders (prostatic hyperplasia, prostatitis, etc.), breast cancer, acne, hirsutism, hidradenitis suppurativa, androgenetic alopecia, cryptorchidism, androgen insensitivity and Kennedy's disease.

15. Combination products or kits comprising the compound as said in any claim of 1-7, or the stereoisomer, the geometrical isomer, the tautomer, the solvate, the hydrate, or the pharmaceutically acceptable salt thereof, and one or more other active agents or pharmaceutical compositions comprising the active agents, which are use concurrently, separately, or sequentially, in therapies for treating or preventing androgen receptor-mediated diseases or disorders.
